# EUROPEAN PATENT SPECIFICATION

(11) **EP 4 077 732 B1**
(45) Date of publication and mention of the grant of the patent: **15.10.2025**
(21) Application number: 20841787.3
(22) Date of filing: 18.12.2020
(51) Int. Cl.: C12Q 1/6883

(54) **CIRCULATING MICRO-RNAS AS BIOMARKERS FOR THE DIAGNOSIS OF CELIAC DISEASE AND FOR MONITORING ADHERENCE TO A GLUTEN-FREE DIET**
ZIRKULIERENDE MIKRO-RNAS ALS BIOMARKER FÜR DIE DIAGNOSE VON ZÖLIAKIE UND ZUR ÜBERWACHUNG DER EINHALTUNG EINER GLUTENFREIEN ERNÄHRUNG
MICRO-ARN CIRCULANTS UTILISÉS EN TANT QUE BIOMARQUEURS POUR LE DIAGNOSTIC DE LA MALADIE COELIAQUE ET POUR SURVEILLER L'ADHÉSION À UN RÉGIME SANS GLUTEN

(30) Priority: 18.12.2019 IT 201900024550
(43) Date of publication of application: 26.10.2022
(73) Proprietor: Ospedale Pediatrico Bambino Gesù, 00165 Roma (IT)
(72) Inventor: MASOTTI, Andrea, 00165 Roma (IT); FELLI, Cristina, 00165 Roma (RM) (IT)
(74) Representative: Gitto, Serena
(86) International application number: PCT/IT2020/050313
(87) International publication number: WO 2021/124369

(56) References cited:
- WO-A1-2019/180271
- BUOLI COMANI GAIA ET AL: "miRNA-regulated gene expression differs in celiac disease patients according to the age of presentation", GENES & NUTRITION ; STUDYING THE RELATIONSHIP BETWEEN GENETICS AND NUTRITION IN THE IMPROVEMENT OF HUMAN HEALTH, BERLIN ; HEIDELBERG : SPRINGER, DE, vol. 10, no. 5, 2 August 2015 (2015-08-02), pages 1 - 12, XP035541082, ISSN: 1555-8932, [retrieved on 20150802], DOI: 10.1007/S12263-015-0482-2
- VAIRA VALENTINA ET AL: "MicroRNA profiles in coeliac patients distinguish different clinical phenotypes and are modulated by gliadin peptides in primary duodenal fibroblasts", vol. 126, no. 6, 1 March 2014 (2014-03-01), pages 417 - 423, XP009526528, ISSN: 0143-5221, Retrieved from the Internet <URL:http://portlandpress.com/clinsci/article-pdf/126/6/417/444417/cs1260417.pdf> [retrieved on 20131115], DOI: 10.1042/CS20130248
- CRISTINA FELLI ET AL: "Intestinal and Circulating MicroRNAs in Coeliac Disease", INTERNATIONAL JOURNAL OF MOLECULAR SCIENCES, vol. 18, no. 9, 6 September 2017 (2017-09-06), pages 1907, XP055716934, DOI: 10.3390/ijms18091907
- BASCUÑÁN KARLA A ET AL: "A miRNA-Based Blood and Mucosal Approach for Detecting and Monitoring Celiac Disease", DIGESTIVE DISEASES AND SCIENCES, SPRINGER NEW YORK LLC, US, vol. 65, no. 7, 28 November 2019 (2019-11-28), pages 1982 - 1991, XP037171808, ISSN: 0163-2116, [retrieved on 20191128], DOI: 10.1007/S10620-019-05966-Z
- K.S. AMR ET AL: "Circulating microRNAs as potential non-invasive biomarkers in pediatric patients with celiac disease", EUROPEAN ANNALS OF ALLERGY AND CLINICAL IMMUNOLOGY, vol. 51, no. 04, 1 April 2019 (2019-04-01), pages 159, XP055716928, DOI: 10.23822/EurAnnACI.1764-1489.90
- SARA A GEORGES ET AL: "Coordinated Regulation of Cell Cycle Transcripts by p53-Inducible microRNAs, miR-192 and miR-215", CANCER RESEARCH - PROCEEDINGS: AACR ANNUAL MEETING 2018; APRIL 14-18, 2018; CHICAGO, IL,, vol. 68, no. 24, 15 December 2008 (2008-12-15), pages 10105 - 10112, XP008138923, ISSN: 0008-5472, DOI: 10.1158/0008-5472.CAN-08-1846

## Description

The present invention relates to circulating microRNAs as biomarkers for the diagnosis of celiac disease and for monitoring adherence to a gluten-free diet. In particular, the invention relates to the use of circulating microRNAs as biomarkers for the non-invasive diagnosis of celiac disease and for monitoring adherence to a gluten-free diet and associated methods.

Celiac disease (CD) is an autoimmune enteropathy distributed throughout the world with a prevalence of about 1% [1] (Gujral N). Celiac disease is a multifactorial disease that occurs with a broad range of clinical, serological and histological manifestations. The environmental component responsible for its activation and maintenance is represented by gluten, a protein complex contained in wheat, barley and rye cereals. Genetic predisposition to the disease depends on the presence of particular haplotypes of HLA (human leukocyte antigen). HLA-DQ2 is present in about 90%-95% of patients, whereas the remaining 5%-10% express HLA-DQ8 [2] (Schuppan).

The immune response to gluten peptides triggers the production of highly specific autoantibodies. The main ones are represented by IgA anti-tissue transglutaminase (-tTG), IgG against deamidated gliadin peptides (DGP) (exams prescribed above all for children under two years of age) and IgA anti-endomysium (EMA) [3, 4] (Guandalini S, Amarri S). Due to the disease, one observes typical damage of the intestinal mucosa, characterised by a flattening of the villi, the presence of atypical squamous epithelial cells, an increase in the number of intraepithelial lymphocytes (IEL) and plasma cells in the lamina propria and crypt hypertrophy [5] (Kagnoff MF).

At present the gold standard for the diagnosis of CD consists in a combination of serological tests with duodenal biopsy. Since 2009 the guidelines defined by the European Society for Paediatric Gastroenterology Hepatology and Nutrition (ESPGHAN) have suggested the possibility of avoiding duodenal biopsies only in cases in which adolescents and children show the HLA DQ2/DQ8 haplotype, positivity to IgA anti-EMA antibodies and a value of IgA anti-tTG 10 times higher than the normal level (ULN) [6] (Husby S). Therefore, to date, no diagnostic test that totally excludes an invasive approach is known and the discovery of new biological markers represents a challenge for the prediction and early diagnosis of CD.

One class of molecules that has great potential in functioning as biomarkers is represented by microRNAs (miRNAs), small non-coding RNAs (18-22 nucleotides) which regulate gene expression at a post-transcriptional level and play important roles in various biological processes, including development, proliferation and apoptosis [7] (Li M). As they are highly stable and tissue specific, their levels of expression can represent the characteristic profiles of specific pathologies, or have a prognostic value [8] (Mitchell). MiRNAs can be transported outside cells in association with RNA-binding proteins, including high-density lipoproteins (HDL) and Argonaute-2 (Ago2), and enter the bloodstream [9] (Xi Chen).

Circulating miRNAs represent potential diagnostic biomarkers, since they are stable, resistant to digestion by RNase, extreme pHs, high temperatures, prolonged storage and multiple freezing-thawing cycles.

Differential miRNA expression levels in plasma samples of celiac patients have already been shown with respect to healthy controls and to patients on gluten-free diet [22, 23] (Buoli Comani, Felli). As regards therapy for CD, to date the exclusion of gluten from the diet (gluten-free diet, GFD) continues to be the only treatment capable of bringing about a symptomatic, serological and histological remission in the majority of the individuals with celiac disease, while reducing the risk of complications tied to the pathology [10] (Green PH). As for monitoring the response of individuals with celiac disease to a gluten-free diet, there has long been discussion about the sensitivity and accuracy of the serological tests that have been used up to now mainly for the diagnosis of CD, which, in the majority of cases, is confirmed by a histological evaluation of the intestinal mucosa. Though the results have demonstrated that an antibody assay is useful for monitoring adherence to the diet, the interpretation of assays can generate false positives. Furthermore, it is not possible to perform a duodenal biopsy in order to periodically monitor the effectiveness of the GFD and disease remission [11, 12] (Sugai, Fasano). To date, there are no available analytical and/or biochemical methods for monitoring the adherence to a gluten-free diet by patients with celiac disease.

In the light of the foregoing, it appears evident that there is a need to provide new methods and markers for the diagnosis of celiac disease and for monitoring the adherence to the GFD which overcome the disadvantages of the known methods.

The solution according to the present invention, which aims to provide new combinations of non-invasive biomarkers that can be used both for the diagnosis of celiac disease and for monitoring adherence to a gluten-free diet, fits into this context.

According to the present invention, specific combinations of circulating microRNAs have now been identified, whose usefulness as a new tool for the diagnosis of celiac disease and for follow-up during the GFD has been evaluated and confirmed.

In particular, as described below in example 1, a rather large cohort was recruited, made up of children with celiac disease (CD) (n = 40), children with celiac disease on a diet (GFD) (n = 40) and healthy donors (n = 40) of comparable age and sex as controls (CTRL). As a first step, the pattern of expression of circulating microRNAs in the serum of a group of children with active CD (n = 20), CD children on a GFD (n = 20) and healthy donors (n = 20) was evaluated by Next Generation Sequencing (NGS) [13] (Burgos). Then the differentially regulated circulating microRNAs were further validated by qPCR [14] (Chen C) in another 20 individuals per group.

Furthermore, in order to rule out that the regulation of these circulating microRNAs might be due to an inflammation of the gastrointestinal tract typical of other inflammatory intestinal diseases (for example IBD), a cohort of patients (n=14) affected by the two main forms of IBD [15] (Loddo I), i.e. ulcerative colitis or Crohn's disease, multifactorial diseases in which autoimmune and immune-mediated processes [16] are implicated (Pascual V), was recruited.

According to the present invention, several combinations of circulating microRNAs characteristic of celiac disease, deregulated in subjects with active celiac disease, were identified which can be advantageously used both in the diagnosis of CD and for monitoring adherence to the GFD by patients with celiac disease.

Therefore, a specific object of the present invention is a method for the *in vitro* diagnosis of celiac disease and/or for the *in vitro* monitoring of adherence to a gluten-free diet, wherein
said method comprises measuring the expression, in a biological sample, of both of the microRNAs hsa-miR-192-5p (MIMAT0000222), hsa-miR-215-5p (MIMAT0000272), wherein said microRNAs are over-expressed in celiac disease compared to their expression in healthy controls or are over-expressed in subjects with celiac disease who do not follow a gluten-free diet compared to their expression in subjects with celiac disease who follow the gluten-free diet, wherein the biological sample is a liquid biological sample consisting of serum or plasma.

According to the present invention, the expression of said microRNAs can be measured by means of a sequencing technique, such as, for example, RNA next generation sequencing, or by means of a Real-Time PCR technique.

According to the present invention, the above-mentioned method can further comprise measuring the expression of hsa-miR-125b-5p (MIMAT0000423) by Real-Time PCR, wherein said hsa-miR-125b-5p is over-expressed in celiac disease compared to its expression in healthy controls or is over-expressed in subjects with celiac disease who do not follow a gluten-free diet compared to its expression in subjects with celiac disease who follow the gluten-free diet. The combination of hsa-miR-192-5p, hsa-miR-215-5p and hsa-miR-125b-5p can thus be advantageously used for the in vitro diagnosis of celiac disease and/or for the in vitro monitoring of adherence to the gluten-free diet.

According to the present invention the above-described method can further comprise measuring the expression of one or more of the following microRNAs by Real-Time PCR: hsa-miR-150-5p (MIMAT0000451), hsa-miR-142-5p (MIMAT0000433), hsa-miR-223-3p (MIMAT0000280) and/or hsa-miR-338-3p (MIMAT0000763), wherein
hsa-miR-150-5p is over-expressed in celiac disease compared to its expression in healthy controls and is over-expressed in subjects with celiac disease who do not follow a gluten-free diet compared to its expression in subjects with celiac disease who follow the gluten-free diet; whereas
hsa-miR-142-5p, hsa-miR-223-3p and hsa-miR-338-3p are down-expressed in subjects with celiac disease who do not follow a gluten-free diet compared to their expression in subjects with celiac disease who follow the gluten-free diet. Therefore, the expression of hsa-miR-150-5p can be measured both for the in vitro diagnosis of celiac disease and for the in vitro monitoring of adherence to the gluten-free diet, whereas the expression of hsa-miR-142-5p, hsa-miR-223-3p and hsa-miR-338-3p can be measured for the in vitro monitoring of adherence to the gluten-free diet.

According to one embodiment of the present invention, the above-described method can comprise measuring the expression of all of the following microRNAs by Real-Time PCR: hsa-miR-192-5p, hsa-miR-215-5p, hsa-miR-125b-5p, hsa-miR-150-5p, hsa-miR-142-5p, hsa-miR-223-3p and hsa-miR-338-3p for the in vitro monitoring of adherence to the gluten-free diet.

According to the present invention, said method, in addition to measuring the expression of hsa-miR-192-5p, hsa-miR-215-5p and hsa-miR-125b-5p, can comprise measuring the expression of one or more of the following microRNAs by Real-Time PCR: hsa-miR-99a-5p (MIMAT0000097), hsa-miR-194-5p (MIMAT0000460), hsa-miR-142-3p (MIMAT0000434), and/or hsa-miR-885-5p (MIMAT0004947), wherein
hsa-miR-99a-5p, hsa-miR-194-5p and hsa-miR-885-5p are over-expressed in celiac disease compared to their expression in healthy controls and are over-expressed in subjects with celiac disease who do not follow a gluten-free diet compared to their expression in subjects with celiac disease who follow the gluten-free diet; whereas
hsa-miR-142-3p is down-expressed in celiac disease compared to its expression in healthy controls. Therefore, the expression of hsa-miR-99a-5p, hsa-miR-194-5p and hsa-miR-885-5p can be measured both for the in vitro diagnosis of celiac disease and for the in vitro monitoring of adherence to the gluten-free diet, whereas the expression of hsa-miR-142-3p can be measured for the in vitro diagnosis of celiac disease.

According to one embodiment of the present invention, the method comprises measuring the expression of all of the following microRNAs by Real-Time PCR: hsa-miR-192-5p, hsa-miR-215-5p, hsa-miR-125b-5p, hsa-miR-99a-5p, hsa-miR-194-5p, hsa-miR-885-5p and hsa-miR-142-3p.

According to the present invention, the method can comprise, in addition to measuring the expression of hsa-miR-192-5p and hsa-miR-215-5p, also measuring the expression of one or more of the following microRNAs by means of a sequencing technique, such as, for example, RNA next generation sequencing: (hsa-miR-141-3p (MIMAT0000432), hsa-miR-24-2-5p (MIMAT0004497), hsa-miR-30a-3p (MIMAT0000088) and/or hsa-miR-375-3p (MIMAT0000728), wherein
hsa-miR-141-3p, hsa-miR-30a-3p and hsa-miR-375-3p are over-expressed in celiac disease compared to their expression in healthy controls and are over-expressed in subjects with celiac disease who do not follow a gluten-free diet compared to their expression in subjects with celiac disease who follow the gluten-free diet;
whereas hsa-miR-24-2-5p is down-expressed in celiac disease compared to its expression in healthy controls and is down-expressed in subjects with celiac disease who do not follow a gluten-free diet compared to its expression in subjects with celiac disease who follow the gluten-free diet. Therefore, the expression of hsa-miR-141-3p, hsa-miR-30a-3p, hsa-miR-375-3p and hsa-miR-24-2-5p can be measured both for the in vitro diagnosis of celiac disease and for the in vitro monitoring of adherence to the gluten-free diet.

According to one embodiment of the present invention, said method can comprise measuring the expression of all of the following microRNAs by means of a sequencing technique:
hsa-miR-192-5p, hsa-miR-215-5p, hsa-miR-141-3p, hsa-miR-30a-3p, hsa-miR-375-3p and hsa-miR-24-2-5p. In addition to measuring the expression of one or more or all of the microRNAs hsa-miR-192-5p, hsa-miR-215-5p, hsa-miR-141-3p, hsa-miR-30a-3p, hsa-miR-375-3p and hsa-miR-24-2-5p, according to a further embodiment the method of the invention can further comprise measuring the expression of hsa-let-7c-5p (MIMAT0000064), wherein said microRNA is over-expressed in celiac disease compared to its expression in healthy controls. Therefore, the expression of hsa-let-7c-5p can be measured for the in vitro diagnosis of celiac disease. For example, the expression of all of the microRNAs hsa-miR-192-5p, hsa-miR-215-5p, hsa-miR-141-3p, hsa-miR-30a-3p, hsa-miR-375-3p, hsa-miR-24-2-5p and hsa-let-7c-5p can be measured for the in vitro diagnosis of celiac disease.

According to a further embodiment, in addition to measuring the expression of one or more or all of the microRNAs hsa-miR-192-5p, hsa-miR-215-5p, hsa-miR-141-3p, hsa-miR-30a-3p, hsa-miR-375-3p and hsa-miR-24-2-5p, the method of the invention can further comprise measuring the expression of one or more of the following microRNAs by means of a sequencing technique, such as, for example, RNA next generation sequencing: hsa-miR-328-3p (MIMAT0000752), hsa-miR-6847-5p (MIMAT0027594) and/or hsa-miR-424-3p (MIMAT0004749), wherein
hsa-miR-6847-5p is over-expressed in subjects with celiac disease who do not follow a gluten-free diet compared to its expression in subjects with celiac disease who follow the gluten-free diet;
whereas hsa-miR-328-3p and hsa-miR-424-3p are down-expressed in subjects with celiac disease who do not follow a gluten-free diet compared to their expression in subjects with celiac disease who follow the gluten-free diet. Therefore, the expression of hsa-miR-6847-5p, hsa-miR-328-3p and hsa-miR-424-3p can be measured for the in vitro monitoring of adherence to the gluten-free diet.

According to a further embodiment of the present invention, the method can comprise measuring the expression of all of the following microRNAs by means of a sequencing technique:
hsa-miR-192-5p, hsa-miR-215-5p, hsa-miR-141-3p, hsa-miR-30a-3p, hsa-miR-375-3p, hsa-miR-24-2-5p, hsa-miR-6847-5p, hsa-miR-328-3p and hsa-miR-424-3p.

Furthermore, the method according to the present invention can further comprise, for the in vitro **diagnosis of celiac disease,** measuring the expression, in a biological sample, of one or more of the following microRNAs: hsa-let-7b-3p (MIMAT0004482), hsa-let-7f-1-3p (MIMAT0004486), hsa-let-7f-5p (MIMAT0000067), hsa-miR-100-5p (MIMAT0000098), hsa-miR-11401 (MIMAT0044658), hsa-miR-1246 (MIMAT0005898), hsa-miR-125b-2-3p (MIMAT0004603), hsa-miR-127-5p (MIMAT0004604), hsa-miR-144-5p (MIMAT0004600), hsa-miR-145-5p (MIMAT0000437), hsa-miR-148b-5p (MIMAT0004699), hsa-miR-152-3p (MIMAT0000438), hsa-miR-181a-5p (MIMAT0000256), hsa-miR-1976 (MIMAT0009451), hsa-miR-200a-3p (MIMAT0000682), hsa-miR-205-5p (MIMAT0000266), hsa-miR-224-5p (MIMAT0000281), hsa-miR-28-3p (MIMAT0004502), hsa-miR-323b-3p (MIMAT0015050), hsa-miR-326 (MIMAT0000756), hsa-miR-335-3p (MIMAT0004703), hsa-miR-335-5p (MIMAT0000765), hsa-miR-339-5p (MIMAT0000764), hsa-miR-363-3p (MIMAT0000707), hsa-miR-370-3p (MIMAT0000722), hsa-miR-374b-3p (MIMAT0004956), hsa-miR-376c-3p (MIMAT0000720), hsa-miR-423-5p (MIMAT0004748), hsa-miR-431-5p (MIMAT0001625), hsa-miR-4745-5p (MIMAT0019878), hsa-miR-4775 (MIMAT0019931), hsa-miR-486-5p (MIMAT0002177), hsa-miR-5009-5p (MIMAT0021041), hsa-miR-511-5p (MIMAT0002808), hsa-miR-532-5p (MIMAT0002888), hsa-miR-5683 (MIMAT0022472), hsa-miR-577 (MIMAT0003242), hsa-miR-584-5p (MIMAT0003249), hsa-miR-628-3p (MIMAT0003297), hsa-miR-6787-3p (MIMAT0027475), hsa-miR-6843-3p (MIMAT0027588), hsa-miR-6894-5p (MIMAT0027688), hsa-miR-766-3p (MIMAT0003888), hsa-miR-7976 (MIMAT0031179), hsa-miR-874-3p (MIMAT0004911), hsa-miR-92b-3p (MIMAT0003218),
wherein the following microRNAs are over-expressed in celiac disease compared to their expression in healthy controls:
hsa-let-7b-3p (MIMAT0004482), hsa-let-7f-1-3p (MIMAT0004486), hsa-miR-100-5p (MIMAT0000098), hsa-miR-1246 (MIMAT0005898), hsa-miR-125b-2-3p (MIMAT0004603), hsa-miR-181a-5p (MIMAT0000256), hsa-miR-200a-3p (MIMAT0000682), hsa-miR-205-5p (MIMAT0000266), hsa-miR-224-5p (MIMAT0000281), hsa-miR-363-3p (MIMAT0000707), hsa-miR-374b-3p (MIMAT0004956), hsa-miR-423-5p (MIMAT0004748), hsa-miR-4745-5p (MIMAT0019878), hsa-miR-486-5p (MIMAT0002177), hsa-miR-5009-5p (MIMAT0021041), hsa-miR-511-5p (MIMAT0002808), hsa-miR-532-5p (MIMAT0002888), hsa-miR-5683 (MIMAT0022472), hsa-miR-577 (MIMAT0003242), hsa-miR-628-3p (MIMAT0003297), hsa-miR-6787-3p (MIMAT0027475), hsa-miR-6894-5p (MIMAT0027688), hsa-miR-7976 (MIMAT0031179), hsa-miR-874-3p (MIMAT0004911), hsa-miR-92b-3p (MIMAT0003218);
whereas the following microRNAs are down-expressed in celiac disease compared to their expression in healthy controls:
   hsa-let-7f-5p (MIMAT0000067), hsa-miR-11401 (MIMAT0044658), hsa-miR-127-5p (MIMAT0004604), hsa-miR-144-5p (MIMAT0004600), hsa-miR-145-5p (MIMAT0000437), hsa-miR-148b-5p (MIMAT0004699), hsa-miR-152-3p (MIMAT0000438), hsa-miR-1976 (MIMAT0009451), hsa-miR-28-3p (MIMAT0004502), hsa-miR-323b-3p (MIMAT0015050), hsa-miR-326 (MIMAT0000756), hsa-miR-335-3p (MIMAT0004703), hsa-miR-335-5p (MIMAT0000765), hsa-miR-339-5p (MIMAT0000764), hsa-miR-370-3p (MIMAT0000722), hsa-miR-376c-3p (MIMAT0000720), hsa-miR-431-5p (MIMAT0001625), hsa-miR-4775 (MIMAT0019931), hsa-miR-584-5p (MIMAT0003249), hsa-miR-6843-3p (MIMAT0027588) and hsa-miR-766-3p (MIMAT0003888); whereas,
   for the in vitro **monitoring of adherence to the gluten-free diet,** said method can further comprise measuring the expression, in a biological sample, of one or more of the following microRNAs:
      hsa-let-7b-5p (MIMAT0000063), hsa-miR-122-5p (MIMAT0000421), hsa-miR-125a-3p (MIMAT0004602), hsa-miR-125b-2-3p (MIMAT0004603), hsa-miR-134-5p (MIMAT0000447), hsa-miR-139-3p (MIMAT0004552), hsa-miR-140-5p (MIMAT0000431), hsa-miR-1468-5p (MIMAT0006789), hsa-miR-148a-5p (MIMAT0004549), hsa-miR-150-3p (MIMAT0004610), hsa-miR-151a-5p (MIMAT0004697), hsa-miR-185-5p (MIMAT0000455), hsa-miR-190a-5p (MIMAT0000458), hsa-miR-190b-5p (MIMAT0004929), hsa-miR-1976 (MIMAT0009451), hsa-miR-199a-5p (MIMAT0000231), hsa-miR-200a-3p (MIMAT0000682), hsa-miR-20a-5p (MIMAT0000075), hsa-miR-224-5p (MIMAT0000281), hsa-miR-301a-3p (MIMAT0000688), hsa-miR-30a-5p (MIMAT0000087), hsa-miR-30d-3p (MIMAT0004551), hsa-miR-3124-5p (MIMAT0014986), hsa-miR-3138 (MIMAT0015006), hsa-miR-326 (MIMAT0000756), hsa-miR-337-5p (MIMAT0004695), hsa-miR-339-5p (MIMAT0000764), hsa-miR-342-5p (MIMAT0004694), hsa-miR-362-5p (MIMAT0000705), hsa-miR-363-3p (MIMAT0000707), hsa-miR-3661 (MIMAT0018082), hsa-miR-370-3p (MIMAT0000722), hsa-miR-377-5p (MIMAT0004689), hsa-miR-423-3p (MIMAT0001340), hsa-miR-423-5p (MIMAT0004748), hsa-miR-432-5p (MIMAT0002814), hsa-miR-4745-5p (MIMAT0019878), hsa-miR-486-5p (MIMAT0002177), hsa-miR-487b-3p (MIMAT0003180), hsa-miR-5010-5p (MIMAT0021043), hsa-miR-539-3p (MIMAT0022705), hsa-miR-542-3p (MIMAT0003389), hsa-miR-5683 (MIMAT0022472), hsa-miR-6735-5p (MIMAT0027371), hsa-miR-7705 (MIMAT0030020), hsa-miR-873-5p (MIMAT0004953), hsa-miR-887-5p (MIMAT0026720);
      wherein the following microRNAs are over-expressed in subjects with celiac disease who do not follow a gluten-free diet compared to their expression in subjects with celiac disease who follow the gluten-free diet:
         hsa-let-7b-5p (MIMAT0000063), hsa-miR-122-5p (MIMAT0000421), hsa-miR-125b-2-3p (MIMAT0004603), hsa-miR-1468-5p (MIMAT0006789), hsa-miR-150-3p (MIMAT0004610), hsa-miR-200a-3p (MIMAT0000682), hsa-miR-224-5p (MIMAT0000281), , hsa-miR-30a-5p (MIMAT0000087), hsa-miR-3138 (MIMAT0015006), hsa-miR-342-5p (MIMAT0004694), hsa-miR-363-3p (MIMAT0000707), hsa-miR-3661 (MIMAT0018082), hsa-miR-423-5p (MIMAT0004748), hsa-miR-4745-5p (MIMAT0019878), hsa-miR-486-5p (MIMAT0002177), hsa-miR-5010-5p (MIMAT0021043), hsa-miR-5683 (MIMAT0022472), hsa-miR-6735-5p (MIMAT0027371);
         whereas the following microRNAs are down-expressed in subjects with celiac disease who do not follow a gluten-free diet compared to their expression in subjects with celiac disease who follow the gluten-free diet:
            hsa-miR-125a-3p (MIMAT0004602), hsa-miR-134-5p (MIMAT0000447), hsa-miR-139-3p (MIMAT0004552), hsa-miR-140-5p (MIMAT0000431), hsa-miR-148a-5p (MIMAT0004549), hsa-miR-151a-5p (MIMAT0004697), hsa-miR-185-5p (MIMAT0000455), hsa-miR-190a-5p (MIMAT0000458), hsa-miR-190b-5p (MIMAT0004929), hsa-miR-1976 (MIMAT0009451), hsa-miR-199a-5p (MIMAT0000231), hsa-miR-20a-5p (MIMAT0000075), hsa-miR-301a-3p (MIMAT0000688), hsa-miR-30d-3p (MIMAT0004551), hsa-miR-3124-5p (MIMAT0014986), hsa-miR-326 (MIMAT0000756), hsa-miR-337-5p (MIMAT0004695), hsa-miR-339-5p (MIMAT0000764), hsa-miR-362-5p (MIMAT0000705), hsa-miR-370-3p (MIMAT0000722), hsa-miR-377-5p (MIMAT0004689), hsa-miR-423-3p (MIMAT0001340), hsa-miR-432-5p (MIMAT0002814), hsa-miR-487b-3p (MIMAT0003180), hsa-miR-539-3p (MIMAT0022705), hsa-miR-542-3p (MIMAT0003389), hsa-miR-7705 (MIMAT0030020), hsa-miR-873-5p (MIMAT0004953) and hsa-miR-887-5p (MIMAT0026720).

According to a particular embodiment, said one or more microRNAs listed above of which to further measure the expression for the in vitro **diagnosis of celiac disease** can be selected from:
hsa-let-7b-3p (MIMAT0004482), hsa-let-7f-5p (MIMAT0000067), hsa-miR-200a-3p (MIMAT0000682), hsa-miR-28-3p (MIMAT0004502), hsa-miR-339-5p (MIMAT0000764), hsa-miR-4775 (MIMAT0019931), hsa-miR-577 (MIMAT0003242), hsa-miR-766-3p (MIMAT0003888) and hsa-miR-874-3p (MIMAT0004911);
alternatively, said one or more microRNAs can be selected from:
   hsa-miR-100-5p (MIMAT0000098), hsa-miR-125b-2-3p (MIMAT0004603), hsa-miR-323b-3p (MIMAT0015050), hsa-miR-335-5p (MIMAT0000765), hsa-miR-370-3p (MIMAT0000722), hsa-miR-423-5p (MIMAT0004748) and hsa-miR-92b-3p (MIMAT0003218); whereas
   for the in vitro **monitoring of adherence to the gluten-free diet** said one or more microRNAs of which to further measure the expression can be selected from:
      hsa-miR-150-3p (MIMAT0004610), hsa-miR-190a-5p (MIMAT0000458), hsa-miR-200a-3p (MIMAT0000682), hsa-miR-30a-3p (MIMAT0000088), hsa-miR-337-5p (MIMAT0004695), hsa-miR-377-5p (MIMAT0004689), hsa-miR-6735-5p (MIMAT0027371) and hsa-miR-887-5p (MIMAT0026720);
      alternatively, said one or more microRNAs can be selected from:
         hsa-miR-125b-2-3p (MIMAT0004603), hsa-miR-134-5p (MIMAT0000447), hsa-miR-185-5p (MIMAT0000455), hsa-miR-3661 (MIMAT0018082) and hsa-miR-423-5p (MIMAT0004748).

Therefore, the above-mentioned microRNAs together in combination can be used as biomarkers for the in vitro diagnosis of celiac disease or for the in vitro monitoring of adherence to the gluten-free diet.

According to an aspect that is not part of the present invention, a method that is valid both for the in vitro diagnosis of celiac disease and for the in vitro monitoring of adherence to the gluten-free diet is a method wherein
for the in vitro diagnosis of celiac disease said method comprises measuring the expression, in a biological sample, of at least two of the following fifty-nine (59) microRNAs: hsa-miR-192-5p (MIMAT0000222), hsa-miR-215-5p (MIMAT0000272), hsa-let-7b-3p (MIMAT0004482), hsa-let-7c-5p (MIMAT0000064), hsa-let-7f-1-3p (MIMAT0004486), hsa-let-7f-5p (MIMAT0000067), hsa-miR-100-5p (MIMAT0000098), hsa-miR-11401 (MIMAT0044658), hsa-miR-1246 (MIMAT0005898), hsa-miR-125b-2-3p (MIMAT0004603), hsa-miR-125b-5p (MIMAT0000423), hsa-miR-127-5p (MIMAT0004604), hsa-miR-141-3p (MIMAT0000432), hsa-miR-142-3p (MIMAT0000434), hsa-miR-144-5p (MIMAT0004600), hsa-miR-145-5p (MIMAT0000437), hsa-miR-148b-5p (MIMAT0004699), hsa-miR-150-5p (MIMAT0000451), hsa-miR-152-3p (MIMAT0000438), hsa-miR-181a-5p (MIMAT0000256), hsa-miR-194-5p (MIMAT0000460), hsa-miR-1976 (MIMAT0009451), hsa-miR-200a-3p (MIMAT0000682), hsa-miR-205-5p (MIMAT0000266), hsa-miR-224-5p (MIMAT0000281), hsa-miR-24-2-5p (MIMAT0004497), hsa-miR-28-3p (MIMAT0004502), hsa-miR-30a-3p (MIMAT0000088), hsa-miR-323b-3p (MIMAT0015050), hsa-miR-326 (MIMAT0000756), hsa-miR-335-3p (MIMAT0004703), hsa-miR-335-5p (MIMAT0000765), hsa-miR-339-5p (MIMAT0000764), hsa-miR-363-3p (MIMAT0000707), hsa-miR-370-3p (MIMAT0000722), hsa-miR-374b-3p (MIMAT0004956), hsa-miR-375-3p (MIMAT0000728), hsa-miR-376c-3p (MIMAT0000720), hsa-miR-423-5p (MIMAT0004748), hsa-miR-431-5p (MIMAT0001625), hsa-miR-4745-5p (MIMAT0019878), hsa-miR-4775 (MIMAT0019931), hsa-miR-486-5p (MIMAT0002177), hsa-miR-5009-5p (MIMAT0021041), hsa-miR-511-5p (MIMAT0002808), hsa-miR-532-5p (MIMAT0002888), hsa-miR-5683 (MIMAT0022472), hsa-miR-577 (MIMAT0003242), hsa-miR-584-5p (MIMAT0003249), hsa-miR-628-3p (MIMAT0003297), hsa-miR-6787-3p (MIMAT0027475), hsa-miR-6843-3p (MIMAT0027588), hsa-miR-6894-5p (MIMAT0027688), hsa-miR-766-3p (MIMAT0003888), hsa-miR-7976 (MIMAT0031179), hsa-miR-874-3p (MIMAT0004911), hsa-miR-885-5p (MIMAT0004947), hsa-miR-92b-3p (MIMAT0003218) and hsa-miR-99a-5p (MIMAT0000097);
wherein the following thirty-six (36) microRNAs are over-expressed in celiac disease compared to their expression in healthy controls:
   hsa-let-7b-3p (MIMAT0004482), hsa-let-7c-5p (MIMAT0000064), hsa-let-7f-1-3p (MIMAT0004486), hsa-miR-100-5p (MIMAT0000098), hsa-miR-1246 (MIMAT0005898), hsa-miR-125b-2-3p (MIMAT0004603), hsa-miR-125b-5p (MIMAT0000423), hsa-miR-141-3p (MIMAT0000432), hsa-miR-150-5p (MIMAT0000451), hsa-miR-181a-5p (MIMAT0000256), hsa-miR-192-5p (MIMAT0000222), hsa-miR-194-5p (MIMAT0000460), hsa-miR-200a-3p (MIMAT0000682), hsa-miR-205-5p (MIMAT0000266), hsa-miR-215-5p (MIMAT0000272), hsa-miR-224-5p (MIMAT0000281), hsa-miR-30a-3p (MIMAT0000088), hsa-miR-363-3p (MIMAT0000707), hsa-miR-374b-3p (MIMAT0004956), hsa-miR-375-3p (MIMAT0000728), hsa-miR-423-5p (MIMAT0004748), hsa-miR-4745-5p (MIMAT0019878), hsa-miR-486-5p (MIMAT0002177), hsa-miR-5009-5p (MIMAT0021041), hsa-miR-511-5p (MIMAT0002808), hsa-miR-532-5p (MIMAT0002888), hsa-miR-5683 (MIMAT0022472), hsa-miR-577 (MIMAT0003242), hsa-miR-628-3p (MIMAT0003297), hsa-miR-6787-3p (MIMAT0027475), hsa-miR-6894-5p (MIMAT0027688), hsa-miR-7976 (MIMAT0031179), hsa-miR-874-3p (MIMAT0004911), hsa-miR-885-5p (MIMAT0004947), hsa-miR-92b-3p (MIMAT0003218) and hsa-miR-99a-5p (MIMAT0000097),
   whereas the following twenty-three (23) microRNAs are down-expressed in celiac disease compared to their expression in healthy controls:
      hsa-let-7f-5p (MIMAT0000067), hsa-miR-11401 (MIMAT0044658), hsa-miR-127-5p (MIMAT0004604), hsa-miR-142-3p (MIMAT0000434), hsa-miR-144-5p (MIMAT0004600), hsa-miR-145-5p (MIMAT0000437), hsa-miR-148b-5p (MIMAT0004699), hsa-miR-152-3p (MIMAT0000438), hsa-miR-1976 (MIMAT0009451), hsa-miR-24-2-5p (MIMAT0004497), hsa-miR-28-3p (MIMAT0004502), hsa-miR-323b-3p (MIMAT0015050), hsa-miR-326 (MIMAT0000756), hsa-miR-335-3p (MIMAT0004703), hsa-miR-335-5p (MIMAT0000765), hsa-miR-339-5p (MIMAT0000764), hsa-miR-370-3p (MIMAT0000722), hsa-miR-376c-3p (MIMAT0000720), hsa-miR-431-5p (MIMAT0001625), hsa-miR-4775 (MIMAT0019931), hsa-miR-584-5p (MIMAT0003249), hsa-miR-6843-3p (MIMAT0027588) and hsa-miR-766-3p (MIMAT0003888);
      and wherein
      for the in vitro monitoring of adherence to the gluten-free diet, said method comprises measuring the expression, in a biological sample, of at least two of the following sixty-five (65) microRNAs:
         hsa-miR-192-5p (MIMAT0000222), hsa-miR-215-5p (MIMAT0000272), hsa-let-7b-5p (MIMAT0000063), hsa-let-7c-5p (MIMAT0000064), hsa-miR-122-5p (MIMAT0000421), hsa-miR-125a-3p (MIMAT0004602), hsa-miR-125b-2-3p (MIMAT0004603), hsa-miR-125b-5p (MIMAT0000423), hsa-miR-134-5p (MIMAT0000447), hsa-miR-139-3p (MIMAT0004552), hsa-miR-140-5p (MIMAT0000431), hsa-miR-141-3p (MIMAT0000432), hsa-miR-142-5p (MIMAT0000433), hsa-miR-1468-5p (MIMAT0006789), hsa-miR-148a-5p (MIMAT0004549), hsa-miR-150-3p (MIMAT0004610), hsa-miR-150-5p (MIMAT0000451), hsa-miR-151a-5p (MIMAT0004697), hsa-miR-185-5p (MIMAT0000455), hsa-miR-190a-5p (MIMAT0000458), hsa-miR-190b-5p (MIMAT0004929), hsa-miR-194-5p (MIMAT0000460), hsa-miR-1976 (MIMAT0009451), hsa-miR-199a-5p (MIMAT0000231), hsa-miR-200a-3p (MIMAT0000682), hsa-miR-20a-5p (MIMAT0000075), hsa-miR-223-3p (MIMAT0000280), hsa-miR-224-5p (MIMAT0000281), hsa-miR-24-2-5p (MIMAT0004497), hsa-miR-301a-3p (MIMAT0000688), hsa-miR-30a-3p (MIMAT0000088), hsa-miR-30a-5p (MIMAT0000087), hsa-miR-30d-3p (MIMAT0004551), hsa-miR-3124-5p (MIMAT0014986), hsa-miR-3138 (MIMAT0015006), hsa-miR-326 (MIMAT0000756), hsa-miR-328-3p (MIMAT0000752), hsa-miR-337-5p (MIMAT0004695), hsa-miR-338-3p (MIMAT0000763), hsa-miR-339-5p (MIMAT0000764), hsa-miR-342-5p (MIMAT0004694), hsa-miR-362-5p (MIMAT0000705), hsa-miR-363-3p (MIMAT0000707), hsa-miR-3661 (MIMAT0018082), hsa-miR-370-3p (MIMAT0000722), hsa-miR-375-3p (MIMAT0000728), hsa-miR-377-5p (MIMAT0004689), hsa-miR-423-3p (MIMAT0001340), hsa-miR-423-5p (MIMAT0004748), hsa-miR-424-3p (MIMAT0004749), hsa-miR-432-5p (MIMAT0002814), hsa-miR-4745-5p (MIMAT0019878), hsa-miR-486-5p (MIMAT0002177), hsa-miR-487b-3p (MIMAT0003180), hsa-miR-5010-5p (MIMAT0021043), hsa-miR-539-3p (MIMAT0022705), hsa-miR-542-3p (MIMAT0003389), hsa-miR-5683 (MIMAT0022472), hsa-miR-6735-5p (MIMAT0027371), hsa-miR-6847-5p (MIMAT0027594), hsa-miR-7705 (MIMAT0030020), hsa-miR-873-5p (MIMAT0004953), hsa-miR-885-5p (MIMAT0004947), hsa-miR-887-5p (MIMAT0026720) and hsa-miR-99a-5p (MIMAT0000097);
         wherein the following thirty (30) microRNAs are over-expressed in subjects with celiac disease who do not follow a gluten-free diet compared to their expression in subjects with celiac disease who follow the gluten-free diet:
            hsa-let-7b-5p (MIMAT0000063), hsa-let-7c-5p (MIMAT0000064), hsa-miR-122-5p (MIMAT0000421), hsa-miR-125b-2-3p (MIMAT0004603), hsa-miR-125b-5p (MIMAT0000423), hsa-miR-141-3p (MIMAT0000432), hsa-miR-1468-5p (MIMAT0006789), hsa-miR-150-3p (MIMAT0004610), hsa-miR-150-5p (MIMAT0000451), hsa-miR-192-5p (MIMAT0000222), hsa-miR-194-5p (MIMAT0000460), hsa-miR-200a-3p (MIMAT0000682), hsa-miR-215-5p (MIMAT0000272), hsa-miR-224-5p (MIMAT0000281), hsa-miR-30a-3p (MIMAT0000088), hsa-miR-30a-5p (MIMAT0000087), hsa-miR-3138 (MIMAT0015006), hsa-miR-342-5p (MIMAT0004694), hsa-miR-363-3p (MIMAT0000707), hsa-miR-3661 (MIMAT0018082), hsa-miR-375-3p (MIMAT0000728), hsa-miR-423-5p (MIMAT0004748), hsa-miR-4745-5p (MIMAT0019878), hsa-miR-486-5p (MIMAT0002177), hsa-miR-5010-5p (MIMAT0021043), hsa-miR-5683 (MIMAT0022472), hsa-miR-6735-5p (MIMAT0027371), hsa-miR-6847-5p (MIMAT0027594), hsa-miR-885-5p (MIMAT0004947) and hsa-miR-99a-5p (MIMAT0000097),
            whereas the following thirty-five (35) microRNAs are down-expressed in subjects with celiac disease who do not follow a gluten-free diet compared to their expression in subjects with celiac disease who follow the gluten-free diet:
               hsa-miR-125a-3p (MIMAT0004602), hsa-miR-134-5p (MIMAT0000447), hsa-miR-139-3p (MIMAT0004552), hsa-miR-140-5p (MIMAT0000431), hsa-miR-142-5p (MIMAT0000433), hsa-miR-148a-5p (MIMAT0004549), hsa-miR-151a-5p (MIMAT0004697), hsa-miR-185-5p (MIMAT0000455), hsa-miR-190a-5p (MIMAT0000458), hsa-miR-190b-5p (MIMAT0004929),
               hsa-miR-1976 (MIMAT0009451), hsa-miR-199a-5p (MIMAT0000231), hsa-miR-20a-5p (MIMAT0000075), hsa-miR-223-3p (MIMAT0000280), hsa-miR-24-2-5p (MIMAT0004497), hsa-miR-301a-3p (MIMAT0000688), hsa-miR-30d-3p (MIMAT0004551), hsa-miR-3124-5p (MIMAT0014986), hsa-miR-326 (MIMAT0000756), hsa-miR-328-3p (MIMAT0000752), hsa-miR-337-5p (MIMAT0004695), hsa-miR-338-3p (MIMAT0000763), hsa-miR-339-5p (MIMAT0000764), hsa-miR-362-5p (MIMAT0000705), hsa-miR-370-3p (MIMAT0000722), hsa-miR-377-5p (MIMAT0004689), hsa-miR-423-3p (MIMAT0001340), hsa-miR-424-3p (MIMAT0004749), hsa-miR-432-5p (MIMAT0002814), hsa-miR-487b-3p (MIMAT0003180), hsa-miR-539-3p (MIMAT0022705), hsa-miR-542-3p (MIMAT0003389), hsa-miR-7705 (MIMAT0030020), hsa-miR-873-5p (MIMAT0004953) and hsa-miR-887-5p (MIMAT0026720).

Therefore, the method described above comprises or consists in the steps of measuring the expression of at least two of the above-mentioned microRNAs and detecting celiac disease when the microRNAs mentioned above for this purpose are over-expressed or down-expressed compared to their expression in healthy controls. Furthermore, the method comprises measuring the expression of at least two of the above-mentioned microRNAs and detecting the non-adherence to the gluten-free diet when the microRNAs mentioned above for this purpose for this purpose are over-expressed or down-expressed compared to their expression in subjects with celiac disease who follow a gluten-free diet.

According to an aspect that is not part of the present invention, for the *in vitro* diagnosis of celiac disease said at least two microRNAs can be selected from:
hsa-let-7b-3p (MIMAT0004482), hsa-let-7c-5p (MIMAT0000064), hsa-let-7f-5p (MIMAT0000067), hsa-miR-125b-5p (MIMAT0000423), hsa-miR-141-3p (MIMAT0000432), hsa-miR-142-3p (MIMAT0000434), hsa-miR-192-5p (MIMAT0000222), hsa-miR-194-5p (MIMAT0000460), hsa-miR-200a-3p (MIMAT0000682), hsa-miR-215-5p (MIMAT0000272), hsa-miR-24-2-5p (MIMAT0004497), hsa-miR-28-3p (MIMAT0004502), hsa-miR-30a-3p (MIMAT0000088), hsa-miR-339-5p (MIMAT0000764), hsa-miR-375-3p (MIMAT0000728), hsa-miR-4775 (MIMAT0019931), hsa-miR-577 (MIMAT0003242), hsa-miR-766-3p (MIMAT0003888), hsa-miR-874-3p (MIMAT0004911), hsa-miR-885-5p (MIMAT0004947) and hsa-miR-99a-5p (MIMAT0000097), which have shown to be statistically significant (p-value<0.05) and to have a good diagnostic power (AUC>0.7). Among them, hsa-miR-192-5p (MIMAT0000222), hsa-miR-215-5p (MIMAT0000272), hsa-let-7c-5p (MIMAT0000064), hsa-miR-874-3p (MIMAT0004911) or hsa-miR-99a-5p (MIMAT0000097) have been detected with both the sequencing and qPCR techniques.

According to an alternative aspect, which is not part of the present invention, said at least two microRNAs can be selected from:
hsa-let-7c-5p (MIMAT0000064), hsa-miR-100-5p (MIMAT0000098), hsa-miR-125b-2-3p (MIMAT0004603), hsa-miR-125b-5p (MIMAT0000423), hsa-miR-141-3p (MIMAT0000432), hsa-miR-142-3p (MIMAT0000434), hsa-miR-150-5p (MIMAT0000451), hsa-miR-192-5p (MIMAT0000222), hsa-miR-194-5p (MIMAT0000460), hsa-miR-215-5p (MIMAT0000272), hsa-miR-24-2-5p (MIMAT0004497), hsa-miR-30a-3p (MIMAT0000088), hsa-miR-323b-3p (MIMAT0015050), hsa-miR-335-5p (MIMAT0000765), hsa-miR-370-3p (MIMAT0000722), hsa-miR-375-3p (MIMAT0000728), hsa-miR-423-5p (MIMAT0004748), hsa-miR-885-5p (MIMAT0004947), hsa-miR-92b-3p (MIMAT0003218) and hsa-miR-99a-5p (MIMAT0000097), which have a high statistical significance (FDR <0.1).

Preferably, said at least two microRNAs can be selected from: hsa-let-7c-5p (MIMAT0000064), hsa-miR-125b-5p (MIMAT0000423), hsa-miR-141-3p (MIMAT0000432), hsa-miR-142-3p (MIMAT0000434), hsa-miR-192-5p (MIMAT0000222), hsa-miR-194-5p (MIMAT0000460), hsa-miR-215-5p (MIMAT0000272), hsa-miR-24-2-5p (MIMAT0004497), hsa-miR-30a-3p (MIMAT0000088), hsa-miR-375-3p (MIMAT0000728), hsa-miR-885-5p (MIMAT0004947) and hsa-miR-99a-5p (MIMAT0000097), which have shown to have a good diagnostic power (AUC>0.7) and a high statistical significance (FDR <0.1). Among them, hsa-miR-192-5p (MIMAT0000222) and hsa-miR-215-5p (MIMAT0000272) have been detected with both the sequencing and qPCR techniques and are thus identifiable irrespective of the technique used.

According to an aspect that is not part of the present invention,
for the in vitro monitoring of adherence to the gluten-free diet said at least two microRNAs can be selected from:
hsa-miR-125b-5p (MIMAT0000423), hsa-miR-141-3p (MIMAT0000432), hsa-miR-142-5p (MIMAT0000433), hsa-miR-150-3p (MIMAT0004610), hsa-miR-150-5p (MIMAT0000451), hsa-miR-190a-5p (MIMAT0000458), hsa-miR-192-5p (MIMAT0000222), hsa-miR-194-5p (MIMAT0000460), hsa-miR-200a-3p (MIMAT0000682), hsa-miR-215-5p (MIMAT0000272), hsa-miR-223-3p (MIMAT0000280), hsa-miR-24-2-5p (MIMAT0004497), hsa-miR-30a-3p (MIMAT0000088), hsa-miR-328-3p (MIMAT0000752), hsa-miR-337-5p (MIMAT0004695), hsa-miR-338-3p (MIMAT0000763), hsa-miR-375-3p (MIMAT0000728), hsa-miR-377-5p (MIMAT0004689), hsa-miR-424-3p (MIMAT0004749), hsa-miR-6735-5p (MIMAT0027371), hsa-miR-6847-5p (MIMAT0027594), hsa-miR-885-5p (MIMAT0004947) and hsa-miR-887-5p (MIMAT0026720), which have shown to be statistically significant (p-value<0.05) and have a good diagnostic power (AUC>0.7).

Said at least two microRNAs can be selected from:
hsa-miR-125b-2-3p (MIMAT0004603), hsa-miR-125b-5p (MIMAT0000423), hsa-miR-134-5p (MIMAT0000447), hsa-miR-141-3p (MIMAT0000432), hsa-miR-142-5p (MIMAT0000433), hsa-miR-150-5p (MIMAT0000451), hsa-miR-185-5p (MIMAT0000455), hsa-miR-192-5p (MIMAT0000222), hsa-miR-215-5p (MIMAT0000272), hsa-miR-223-3p (MIMAT0000280), hsa-miR-24-2-5p (MIMAT0004497), hsa-miR-30a-3p (MIMAT0000088), hsa-miR-328-3p (MIMAT0000752), hsa-miR-338-3p (MIMAT0000763), hsa-miR-3661 (MIMAT0018082), hsa-miR-375-3p (MIMAT0000728), hsa-miR-423-5p (MIMAT0004748), hsa-miR-424-3p (MIMAT0004749) and hsa-miR-6847-5p (MIMAT0027594), which have a high statistical significance (FDR <0.1).

Preferably, said at least two microRNAs can be selected from the following fourteen (14) microRNAs: hsa-miR-125b-5p (MIMAT0000423), hsa-miR-141-3p (MIMAT0000432), hsa-miR-142-5p (MIMAT0000433), hsa-miR-150-5p (MIMAT0000451), hsa-miR-192-5p (MIMAT0000222), hsa-miR-215-5p (MIMAT0000272), hsa-miR-223-3p (MIMAT0000280), hsa-miR-24-2-5p (MIMAT0004497), hsa-miR-30a-3p (MIMAT0000088), hsa-miR-328-3p (MIMAT0000752), hsa-miR-338-3p (MIMAT0000763), hsa-miR-375-3p (MIMAT0000728), hsa-miR-424-3p (MIMAT0004749) and hsa-miR-6847-5p (MIMAT0027594), which have shown to have a good diagnostic power (AUC>0.7) and a high statistical significance (FDR <0.1) and wherein hsa-miR-192-5p (MIMAT0000222) and hsa-miR-215-5p (MIMAT0000272) have been detected with both the sequencing and qPCR techniques and are thus identifiable irrespective of the technique used.

According to a further embodiment of the present invention, said method can comprise measuring the expression of at least the following two microRNAs:
hsa-miR-192-5p (MIMAT0000222) and hsa-miR-215-5p (MIMAT0000272), possibly in combination with one or more of the remaining microRNAs listed above,
for example in combination with hsa-miR-141-3p (MIMAT0000432), hsa-miR-375-3p (MIMAT0000728), hsa-miR-24-2-5p (MIMAT0004497), hsa-let-7c-5p (MIMAT0000064) and hsa-miR-30a-3p (MIMAT0000088);
or else in combination with hsa-miR-99a-5p (MIMAT0000097), hsa-miR-125b-5p (MIMAT0000423), hsa-miR-194-5p (MIMAT0000460), hsa-miR-142-3p (MIMAT0000434) and hsa-miR-885-5p (MIMAT0004947);
or else in combination with hsa-miR-141-3p (MIMAT0000432), hsa-miR-30a-3p (MIMAT0000088), hsa-miR-24-2-5p (MIMAT0004497), hsa-miR-328-3p (MIMAT0000752), hsa-miR-375-3p (MIMAT0000728), hsa-miR-6847-5p (MIMAT0027594) and hsa-miR-424-3p (MIMAT0004749);
or else in combination with hsa-miR-125b-5p (MIMAT0000423), hsa-miR-150-5p (MIMAT0000451), hsa-miR-142-5p (MIMAT0000433), hsa-miR-223-3p (MIMAT0000280) and hsa-miR-338-3p (MIMAT0000763).

According to the present invention, as mentioned above, the biological sample is a liquid biological sample (so-called "liquid biopsy"), that is serum or plasma, preferably serum.

The method according to the present invention can be carried out by Real-Time PCR (qPCR), sequencing, such as, for example, RNA next generation sequencing, Droplet Digital PCR, Microarrays, Flow cytometry, or RNA hybridization methods such Northern Blot or Dot Blot.

In particular, the sequencing technique can be used for the following miRNAs:
hsa-let-7c-5p (MIMAT0000064), hsa-let-7f-1-3p (MIMAT0004486), hsa-miR-100-5p (MIMAT0000098), hsa-miR-11401 (MIMAT0044658), hsa-miR-1246 (MIMAT0005898), hsa-miR-125a-3p (MIMAT0004602), hsa-miR-125b-2-3p (MIMAT0004603), hsa-miR-127-5p (MIMAT0004604), hsa-miR-134-5p (MIMAT0000447), hsa-miR-139-3p (MIMAT0004552), hsa-miR-141-3p (MIMAT0000432), hsa-miR-144-5p (MIMAT0004600), hsa-miR-145-5p (MIMAT0000437), hsa-miR-1468-5p (MIMAT0006789), hsa-miR-148a-5p (MIMAT0004549), hsa-miR-148b-5p (MIMAT0004699), hsa-miR-150-3p (MIMAT0004610), hsa-miR-150-5p (MIMAT0000451), hsa-miR-152-3p (MIMAT0000438), hsa-miR-181a-5p (MIMAT0000256), hsa-miR-185-5p (MIMAT0000455), hsa-miR-190a-5p (MIMAT0000458), hsa-miR-190b-5p (MIMAT0004929), hsa-miR-192-5p (MIMAT0000222), hsa-miR-1976 (MIMAT0009451),
   hsa-miR-200a-3p (MIMAT0000682), hsa-miR-205-5p (MIMAT0000266), hsa-miR-20a-5p (MIMAT0000075), hsa-miR-215-5p (MIMAT0000272), hsa-miR-224-5p (MIMAT0000281), hsa-miR-24-2-5p (MIMAT0004497), hsa-miR-30a-3p (MIMAT0000088), hsa-miR-30d-3p (MIMAT0004551), hsa-miR-3124-5p (MIMAT0014986), hsa-miR-3138 (MIMAT0015006), hsa-miR-323b-3p (MIMAT0015050), hsa-miR-328-3p (MIMAT0000752), hsa-miR-335-3p (MIMAT0004703), hsa-miR-335-5p (MIMAT0000765), hsa-miR-337-5p (MIMAT0004695), hsa-miR-342-5p (MIMAT0004694), hsa-miR-362-5p (MIMAT0000705), hsa-miR-3661 (MIMAT0018082), hsa-miR-370-3p (MIMAT0000722), hsa-miR-374b-3p (MIMAT0004956), hsa-miR-375-3p (MIMAT0000728), hsa-miR-376c-3p (MIMAT0000720), hsa-miR-377-5p (MIMAT0004689), hsa-miR-423-5p (MIMAT0004748), hsa-miR-424-3p (MIMAT0004749), hsa-miR-431-5p (MIMAT0001625), hsa-miR-432-5p (MIMAT0002814), hsa-miR-4745-5p (MIMAT0019878), hsa-miR-4775 (MIMAT0019931), hsa-miR-487b-3p (MIMAT0003180), hsa-miR-5009-5p (MIMAT0021041), hsa-miR-5010-5p (MIMAT0021043), hsa-miR-511-5p (MIMAT0002808), hsa-miR-539-3p (MIMAT0022705), hsa-miR-542-3p (MIMAT0003389), hsa-miR-5683 (MIMAT0022472), hsa-miR-577 (MIMAT0003242), hsa-miR-628-3p (MIMAT0003297), hsa-miR-6735-5p (MIMAT0027371), hsa-miR-6787-3p (MIMAT0027475), hsa-miR-6843-3p (MIMAT0027588), hsa-miR-6847-5p (MIMAT0027594), hsa-miR-6894-5p (MIMAT0027688), hsa-miR-766-3p (MIMAT0003888), hsa-miR-7705 (MIMAT0030020), hsa-miR-7976 (MIMAT0031179), hsa-miR-873-5p (MIMAT0004953), hsa-miR-874-3p (MIMAT0004911), hsa-miR-887-5p (MIMAT0026720), hsa-miR-92b-3p (MIMAT0003218) and hsa-miR-99a-5p (MIMAT0000097);
whereas the qPCR technique can be used for the following miRNAs:
   hsa-let-7b-3p (MIMAT0004482), hsa-let-7b-5p (MIMAT0000063), hsa-let-7c-5p (MIMAT0000064), hsa-let-7f-5p (MIMAT0000067), hsa-miR-122-5p (MIMAT0000421), hsa-miR-125b-5p (MIMAT0000423), hsa-miR-140-5p (MIMAT0000431), hsa-miR-142-3p (MIMAT0000434), hsa-miR-142-5p (MIMAT0000433), hsa-miR-150-5p (MIMAT0000451), hsa-miR-151a-5p (MIMAT0004697), hsa-miR-192-5p (MIMAT0000222), hsa-miR-194-5p (MIMAT0000460), hsa-miR-199a-5p (MIMAT0000231), hsa-miR-200a-3p (MIMAT0000682), hsa-miR-215-5p (MIMAT0000272), hsa-miR-223-3p (MIMAT0000280), hsa-miR-28-3p (MIMAT0004502), hsa-miR-301a-3p (MIMAT0000688), hsa-miR-30a-5p (MIMAT0000087), hsa-miR-326 (MIMAT0000756), hsa-miR-338-3p (MIMAT0000763), hsa-miR-339-5p (MIMAT0000764), hsa-miR-363-3p (MIMAT0000707), hsa-miR-375-3p (MIMAT0000728), hsa-miR-423-3p (MIMAT0001340), hsa-miR-486-5p (MIMAT0002177), hsa-miR-532-5p (MIMAT0002888), hsa-miR-584-5p (MIMAT0003249), hsa-miR-874-3p (MIMAT0004911), hsa-miR-885-5p (MIMAT0004947) and hsa-miR-99a-5p (MIMAT0000097).

Furthermore, an aspect that is not part of the present invention relates to the use of at least two microRNAs as biomarkers for the in vitro diagnosis of celiac disease or for the in vitro monitoring of adherence to a gluten-free diet, wherein
for the in vitro diagnosis of celiac disease said at least two microRNAs are selected from:
hsa-miR-192-5p (MIMAT0000222), hsa-miR-215-5p (MIMAT0000272), hsa-let-7b-3p (MIMAT0004482), hsa-let-7c-5p (MIMAT0000064), hsa-let-7f-1-3p (MIMAT0004486), hsa-let-7f-5p (MIMAT0000067), hsa-miR-100-5p (MIMAT0000098), hsa-miR-11401 (MIMAT0044658), hsa-miR-1246 (MIMAT0005898), hsa-miR-125b-2-3p (MIMAT0004603), hsa-miR-125b-5p (MIMAT0000423), hsa-miR-127-5p (MIMAT0004604), hsa-miR-141-3p (MIMAT0000432), hsa-miR-142-3p (MIMAT0000434), hsa-miR-144-5p (MIMAT0004600), hsa-miR-145-5p (MIMAT0000437), hsa-miR-148b-5p (MIMAT0004699), hsa-miR-150-5p (MIMAT0000451), hsa-miR-152-3p (MIMAT0000438), hsa-miR-181a-5p (MIMAT0000256), hsa-miR-194-5p (MIMAT0000460), hsa-miR-1976 (MIMAT0009451), hsa-miR-200a-3p (MIMAT0000682), hsa-miR-205-5p (MIMAT0000266), hsa-miR-224-5p (MIMAT0000281), hsa-miR-24-2-5p (MIMAT0004497), hsa-miR-28-3p (MIMAT0004502), hsa-miR-30a-3p (MIMAT0000088), hsa-miR-323b-3p (MIMAT0015050), hsa-miR-326 (MIMAT0000756), hsa-miR-335-3p (MIMAT0004703), hsa-miR-335-5p (MIMAT0000765), hsa-miR-339-5p (MIMAT0000764), hsa-miR-363-3p (MIMAT0000707), hsa-miR-370-3p (MIMAT0000722), hsa-miR-374b-3p (MIMAT0004956), hsa-miR-375-3p (MIMAT0000728), hsa-miR-376c-3p (MIMAT0000720), hsa-miR-423-5p (MIMAT0004748), hsa-miR-431-5p (MIMAT0001625), hsa-miR-4745-5p (MIMAT0019878), hsa-miR-4775 (MIMAT0019931), hsa-miR-486-5p (MIMAT0002177), hsa-miR-5009-5p (MIMAT0021041), hsa-miR-511-5p (MIMAT0002808), hsa-miR-532-5p (MIMAT0002888), hsa-miR-5683 (MIMAT0022472), hsa-miR-577 (MIMAT0003242), hsa-miR-584-5p (MIMAT0003249), hsa-miR-628-3p (MIMAT0003297), hsa-miR-6787-3p (MIMAT0027475), hsa-miR-6843-3p (MIMAT0027588), hsa-miR-6894-5p (MIMAT0027688), hsa-miR-766-3p (MIMAT0003888), hsa-miR-7976 (MIMAT0031179), hsa-miR-874-3p (MIMAT0004911), hsa-miR-885-5p (MIMAT0004947), hsa-miR-92b-3p (MIMAT0003218) and hsa-miR-99a-5p (MIMAT0000097);
wherein the following thirty-six (36) microRNAs are over-expressed in celiac disease compared to their expression in healthy controls:
   hsa-let-7b-3p (MIMAT0004482), hsa-let-7c-5p (MIMAT0000064), hsa-let-7f-1-3p (MIMAT0004486), hsa-miR-100-5p (MIMAT0000098), hsa-miR-1246 (MIMAT0005898), hsa-miR-125b-2-3p (MIMAT0004603), hsa-miR-125b-5p (MIMAT0000423), hsa-miR-141-3p (MIMAT0000432), hsa-miR-150-5p (MIMAT0000451), hsa-miR-181a-5p (MIMAT0000256), hsa-miR-192-5p (MIMAT0000222), hsa-miR-194-5p (MIMAT0000460), hsa-miR-200a-3p (MIMAT0000682), hsa-miR-205-5p (MIMAT0000266), hsa-miR-215-5p (MIMAT0000272), hsa-miR-224-5p (MIMAT0000281), hsa-miR-30a-3p (MIMAT0000088), hsa-miR-363-3p (MIMAT0000707), hsa-miR-374b-3p (MIMAT0004956), hsa-miR-375-3p (MIMAT0000728), hsa-miR-423-5p (MIMAT0004748), hsa-miR-4745-5p (MIMAT0019878), hsa-miR-486-5p (MIMAT0002177), hsa-miR-5009-5p (MIMAT0021041), hsa-miR-511-5p (MIMAT0002808), hsa-miR-532-5p (MIMAT0002888), hsa-miR-5683 (MIMAT0022472), hsa-miR-577 (MIMAT0003242), hsa-miR-628-3p (MIMAT0003297), hsa-miR-6787-3p (MIMAT0027475), hsa-miR-6894-5p (MIMAT0027688), hsa-miR-7976 (MIMAT0031179), hsa-miR-874-3p (MIMAT0004911), hsa-miR-885-5p (MIMAT0004947), hsa-miR-92b-3p (MIMAT0003218) and hsa-miR-99a-5p (MIMAT0000097);
   whereas the following twenty-three (23) microRNAs are down-expressed in celiac disease compared to their expression in healthy controls:
      hsa-let-7f-5p (MIMAT0000067), hsa-miR-11401 (MIMAT0044658), hsa-miR-127-5p (MIMAT0004604), hsa-miR-142-3p (MIMAT0000434), hsa-miR-144-5p (MIMAT0004600), hsa-miR-145-5p (MIMAT0000437), hsa-miR-148b-5p (MIMAT0004699), hsa-miR-152-3p (MIMAT0000438), hsa-miR-1976 (MIMAT0009451), hsa-miR-24-2-5p (MIMAT0004497), hsa-miR-28-3p (MIMAT0004502), hsa-miR-323b-3p (MIMAT0015050), hsa-miR-326 (MIMAT0000756), hsa-miR-335-3p (MIMAT0004703), hsa-miR-335-5p (MIMAT0000765), hsa-miR-339-5p (MIMAT0000764), hsa-miR-370-3p (MIMAT0000722), hsa-miR-376c-3p (MIMAT0000720), hsa-miR-431-5p (MIMAT0001625), hsa-miR-4775 (MIMAT0019931), hsa-miR-584-5p (MIMAT0003249), hsa-miR-6843-3p (MIMAT0027588) and hsa-miR-766-3p (MIMAT0003888);
      and wherein
      for the in vitro monitoring of adherence to a gluten-free diet, said at least two microRNAs are selected from:
         hsa-miR-192-5p (MIMAT0000222), hsa-miR-215-5p (MIMAT0000272), hsa-let-7b-5p (MIMAT0000063), hsa-let-7c-5p (MIMAT0000064), hsa-miR-122-5p (MIMAT0000421), hsa-miR-125a-3p (MIMAT0004602), hsa-miR-125b-2-3p (MIMAT0004603), hsa-miR-125b-5p (MIMAT0000423), hsa-miR-134-5p (MIMAT0000447), hsa-miR-139-3p (MIMAT0004552), hsa-miR-140-5p (MIMAT0000431), hsa-miR-141-3p (MIMAT0000432), hsa-miR-142-5p (MIMAT0000433), hsa-miR-1468-5p (MIMAT0006789), hsa-miR-148a-5p (MIMAT0004549), hsa-miR-150-3p (MIMAT0004610), hsa-miR-150-5p (MIMAT0000451), hsa-miR-151a-5p (MIMAT0004697), hsa-miR-185-5p (MIMAT0000455), hsa-miR-190a-5p (MIMAT0000458), hsa-miR-190b-5p (MIMAT0004929), hsa-miR-194-5p (MIMAT0000460), hsa-miR-1976 (MIMAT0009451), hsa-miR-199a-5p (MIMAT0000231), hsa-miR-200a-3p (MIMAT0000682), hsa-miR-20a-5p (MIMAT0000075), hsa-miR-223-3p (MIMAT0000280), hsa-miR-224-5p (MIMAT0000281), hsa-miR-24-2-5p (MIMAT0004497), hsa-miR-301a-3p (MIMAT0000688), hsa-miR-30a-3p (MIMAT0000088), hsa-miR-30a-5p (MIMAT0000087), hsa-miR-30d-3p (MIMAT0004551), hsa-miR-3124-5p (MIMAT0014986), hsa-miR-3138 (MIMAT0015006), hsa-miR-326 (MIMAT0000756), hsa-miR-328-3p (MIMAT0000752), hsa-miR-337-5p (MIMAT0004695), hsa-miR-338-3p (MIMAT0000763), hsa-miR-339-5p (MIMAT0000764), hsa-miR-342-5p (MIMAT0004694), hsa-miR-362-5p (MIMAT0000705), hsa-miR-363-3p (MIMAT0000707), hsa-miR-3661 (MIMAT0018082), hsa-miR-370-3p (MIMAT0000722), hsa-miR-375-3p (MIMAT0000728), hsa-miR-377-5p (MIMAT0004689), hsa-miR-423-3p (MIMAT0001340), hsa-miR-423-5p (MIMAT0004748), hsa-miR-424-3p (MIMAT0004749), hsa-miR-432-5p (MIMAT0002814), hsa-miR-4745-5p (MIMAT0019878), hsa-miR-486-5p (MIMAT0002177), hsa-miR-487b-3p (MIMAT0003180), hsa-miR-5010-5p (MIMAT0021043), hsa-miR-539-3p (MIMAT0022705), hsa-miR-542-3p (MIMAT0003389), hsa-miR-5683 (MIMAT0022472), hsa-miR-6735-5p (MIMAT0027371), hsa-miR-6847-5p (MIMAT0027594), hsa-miR-7705 (MIMAT0030020), hsa-miR-873-5p (MIMAT0004953), hsa-miR-885-5p (MIMAT0004947), hsa-miR-887-5p (MIMAT0026720) and hsa-miR-99a-5p (MIMAT0000097);
         wherein the following thirty (30) microRNAs are over-expressed in subjects with celiac disease who do not follow a gluten-free diet compared to their expression in subjects with celiac disease who follow the gluten-free diet:
            hsa-let-7b-5p (MIMAT0000063), hsa-let-7c-5p (MIMAT0000064), hsa-miR-122-5p (MIMAT0000421), hsa-miR-125b-2-3p (MIMAT0004603), hsa-miR-125b-5p (MIMAT0000423), hsa-miR-141-3p (MIMAT0000432), hsa-miR-1468-5p (MIMAT0006789), hsa-miR-150-3p (MIMAT0004610), hsa-miR-150-5p (MIMAT0000451), hsa-miR-192-5p (MIMAT0000222), hsa-miR-194-5p (MIMAT0000460), hsa-miR-200a-3p (MIMAT0000682), hsa-miR-215-5p (MIMAT0000272), hsa-miR-224-5p (MIMAT0000281), hsa-miR-30a-3p (MIMAT0000088), hsa-miR-30a-5p (MIMAT0000087), hsa-miR-3138 (MIMAT0015006), hsa-miR-342-5p (MIMAT0004694), hsa-miR-363-3p (MIMAT0000707), hsa-miR-3661 (MIMAT0018082), hsa-miR-375-3p (MIMAT0000728), hsa-miR-423-5p (MIMAT0004748), hsa-miR-4745-5p (MIMAT0019878), hsa-miR-486-5p (MIMAT0002177), hsa-miR-5010-5p (MIMAT0021043), hsa-miR-5683 (MIMAT0022472), hsa-miR-6735-5p (MIMAT0027371), hsa-miR-6847-5p (MIMAT0027594), hsa-miR-885-5p (MIMAT0004947) and hsa-miR-99a-5p (MIMAT0000097);
            whereas the following thirty-five (35) microRNAs are down-expressed in subjects with celiac disease who do not follow a gluten-free diet compared to their expression in subjects with celiac disease who follow the gluten-free diet:
               hsa-miR-125a-3p (MIMAT0004602), hsa-miR-134-5p (MIMAT0000447), hsa-miR-139-3p (MIMAT0004552), hsa-miR-140-5p (MIMAT0000431), hsa-miR-142-5p (MIMAT0000433), hsa-miR-148a-5p (MIMAT0004549), hsa-miR-151a-5p (MIMAT0004697), hsa-miR-185-5p (MIMAT0000455), hsa-miR-190a-5p (MIMAT0000458), hsa-miR-190b-5p (MIMAT0004929),
               hsa-miR-1976 (MIMAT0009451), hsa-miR-199a-5p (MIMAT0000231), hsa-miR-20a-5p (MIMAT0000075), hsa-miR-223-3p (MIMAT0000280), hsa-miR-24-2-5p (MIMAT0004497), hsa-miR-301a-3p (MIMAT0000688), hsa-miR-30d-3p (MIMAT0004551), hsa-miR-3124-5p (MIMAT0014986), hsa-miR-326 (MIMAT0000756), hsa-miR-328-3p (MIMAT0000752), hsa-miR-337-5p (MIMAT0004695), hsa-miR-338-3p (MIMAT0000763), hsa-miR-339-5p (MIMAT0000764), hsa-miR-362-5p (MIMAT0000705), hsa-miR-370-3p (MIMAT0000722), hsa-miR-377-5p (MIMAT0004689), hsa-miR-423-3p (MIMAT0001340), hsa-miR-424-3p (MIMAT0004749), hsa-miR-432-5p (MIMAT0002814), hsa-miR-487b-3p (MIMAT0003180), hsa-miR-539-3p (MIMAT0022705), hsa-miR-542-3p (MIMAT0003389), hsa-miR-7705 (MIMAT0030020), hsa-miR-873-5p (MIMAT0004953) and hsa-miR-887-5p (MIMAT0026720).

According to an aspect that is not part of the present invention, the use of the biomarkers for the diagnosis of celiac disease comprises using at least two microRNAs which can be selected from:
hsa-let-7b-3p (MIMAT0004482), hsa-let-7c-5p (MIMAT0000064), hsa-let-7f-5p (MIMAT0000067), hsa-miR-125b-5p (MIMAT0000423), hsa-miR-141-3p (MIMAT0000432), hsa-miR-142-3p (MIMAT0000434), hsa-miR-192-5p (MIMAT0000222), hsa-miR-194-5p (MIMAT0000460), hsa-miR-200a-3p (MIMAT0000682), hsa-miR-215-5p (MIMAT0000272), hsa-miR-24-2-5p (MIMAT0004497), hsa-miR-28-3p (MIMAT0004502), hsa-miR-30a-3p (MIMAT0000088), hsa-miR-339-5p (MIMAT0000764), hsa-miR-375-3p (MIMAT0000728), hsa-miR-4775 (MIMAT0019931), hsa-miR-577 (MIMAT0003242), hsa-miR-766-3p (MIMAT0003888), hsa-miR-874-3p (MIMAT0004911), hsa-miR-885-5p (MIMAT0004947) and hsa-miR-99a-5p (MIMAT0000097), which have shown to be statistically significant (p-value<0.05) and have a good diagnostic power (AUC>0.7). Among them, hsa-miR-192-5p (MIMAT0000222), hsa-miR-215-5p (MIMAT0000272), hsa-let-7c-5p (MIMAT0000064), hsa-miR-874-3p (MIMAT0004911) or hsa-miR-99a-5p (MIMAT0000097) have been detected with both the sequencing and qPCR techniques.

A further aspect that is not part of the present invention is the use of the biomarkers for the diagnosis of celiac disease comprising the use of at least two microRNAs which can be selected from:
hsa-let-7c-5p (MIMAT0000064), hsa-miR-100-5p (MIMAT0000098), hsa-miR-125b-2-3p (MIMAT0004603), hsa-miR-125b-5p (MIMAT0000423), hsa-miR-141-3p (MIMAT0000432), hsa-miR-142-3p (MIMAT0000434), hsa-miR-150-5p (MIMAT0000451), hsa-miR-192-5p (MIMAT0000222), hsa-miR-194-5p (MIMAT0000460), hsa-miR-215-5p (MIMAT0000272), hsa-miR-24-2-5p (MIMAT0004497), hsa-miR-30a-3p (MIMAT0000088), hsa-miR-323b-3p (MIMAT0015050), hsa-miR-335-5p (MIMAT0000765), hsa-miR-370-3p (MIMAT0000722), hsa-miR-375-3p (MIMAT0000728), hsa-miR-423-5p (MIMAT0004748), hsa-miR-885-5p (MIMAT0004947), hsa-miR-92b-3p (MIMAT0003218) and hsa-miR-99a-5p (MIMAT0000097), which have a high statistical significance (FDR <0.1).

The use of the biomarkers for the diagnosis of celiac disease comprises the use of at least two microRNAs which can be selected from: hsa-let-7c-5p (MIMAT0000064), hsa-miR-125b-5p (MIMAT0000423), hsa-miR-141-3p (MIMAT0000432), hsa-miR-142-3p (MIMAT0000434), hsa-miR-192-5p (MIMAT0000222), hsa-miR-194-5p (MIMAT0000460), hsa-miR-215-5p (MIMAT0000272), hsa-miR-24-2-5p (MIMAT0004497), hsa-miR-30a-3p (MIMAT0000088), hsa-miR-375-3p (MIMAT0000728), hsa-miR-885-5p (MIMAT0004947) and hsa-miR-99a-5p (MIMAT0000097), which have shown to have a good diagnostic power (AUC>0.7) and a high statistical significance (FDR <0.1). Among them, hsa-miR-192-5p (MIMAT0000222) and hsa-miR-215-5p (MIMAT0000272) have been detected with both the sequencing and qPCR techniques and are thus identifiable irrespective of the technique used.

According to a further aspect that is not part of the present invention, the use of the biomarkers for monitoring adherence to the gluten-free diet comprises the use of at least two microRNAs which can be selected from:
hsa-miR-125b-5p (MIMAT0000423), hsa-miR-141-3p (MIMAT0000432), hsa-miR-142-5p (MIMAT0000433), hsa-miR-150-3p (MIMAT0004610), hsa-miR-150-5p (MIMAT0000451), hsa-miR-190a-5p (MIMAT0000458), hsa-miR-192-5p (MIMAT0000222), hsa-miR-194-5p (MIMAT0000460), hsa-miR-200a-3p (MIMAT0000682), hsa-miR-215-5p (MIMAT0000272), hsa-miR-223-3p (MIMAT0000280), hsa-miR-24-2-5p (MIMAT0004497), hsa-miR-30a-3p (MIMAT0000088), hsa-miR-328-3p (MIMAT0000752), hsa-miR-337-5p (MIMAT0004695), hsa-miR-338-3p (MIMAT0000763), hsa-miR-375-3p (MIMAT0000728), hsa-miR-377-5p (MIMAT0004689), hsa-miR-424-3p (MIMAT0004749), hsa-miR-6735-5p (MIMAT0027371), hsa-miR-6847-5p (MIMAT0027594), hsa-miR-885-5p (MIMAT0004947) and hsa-miR-887-5p (MIMAT0026720), which have shown to be statistically significant (p-value<0.05) and have a good diagnostic power (AUC>0.7).

According to an alternative aspect that is not part of the present invention, the use of the biomarkers for monitoring adherence to the gluten-free diet comprises the use of at least two microRNAs which can be selected from:
hsa-miR-125b-2-3p (MIMAT0004603), hsa-miR-125b-5p (MIMAT0000423), hsa-miR-134-5p (MIMAT0000447), hsa-miR-141-3p (MIMAT0000432), hsa-miR-142-5p (MIMAT0000433), hsa-miR-150-5p (MIMAT0000451), hsa-miR-185-5p (MIMAT0000455), hsa-miR-192-5p (MIMAT0000222), hsa-miR-215-5p (MIMAT0000272), hsa-miR-223-3p (MIMAT0000280), hsa-miR-24-2-5p (MIMAT0004497), hsa-miR-30a-3p (MIMAT0000088), hsa-miR-328-3p (MIMAT0000752), hsa-miR-338-3p (MIMAT0000763), hsa-miR-3661 (MIMAT0018082), hsa-miR-375-3p (MIMAT0000728), hsa-miR-423-5p (MIMAT0004748), hsa-miR-424-3p (MIMAT0004749) and hsa-miR-6847-5p (MIMAT0027594), which have a high statistical significance (FDR <0.1).

Preferably, the use of the biomarkers for monitoring adherence to the gluten-free diet comprises the use of at least two microRNAs which can be selected from the following fourteen (14) microRNAs: hsa-miR-125b-5p (MIMAT0000423), hsa-miR-141-3p (MIMAT0000432), hsa-miR-142-5p (MIMAT0000433), hsa-miR-150-5p (MIMAT0000451), hsa-miR-192-5p (MIMAT0000222), hsa-miR-215-5p (MIMAT0000272), hsa-miR-223-3p (MIMAT0000280), hsa-miR-24-2-5p (MIMAT0004497), hsa-miR-30a-3p (MIMAT0000088), hsa-miR-328-3p (MIMAT0000752), hsa-miR-338-3p (MIMAT0000763), hsa-miR-375-3p (MIMAT0000728), hsa-miR-424-3p (MIMAT0004749) and hsa-miR-6847-5p (MIMAT0027594), which have shown to have a good diagnostic power (AUC>0.7) and a high statistical significance (FDR <0.1) and wherein hsa-miR-192-5p (MIMAT0000222) and hsa-miR-215-5p (MIMAT0000272) have been detected with both the sequencing and qPCR techniques and are thus identifiable irrespective of the technique used.

According to a further embodiment of the present invention, the use of the biomarkers can comprise the use of at least the following two microRNAs:
hsa-miR-192-5p (MIMAT0000222) and hsa-miR-215-5p (MIMAT0000272), possibly in combination with one or more of the remaining microRNAs listed above,
for example in combination with hsa-miR-141-3p (MIMAT0000432), hsa-miR-375-3p (MIMAT0000728), hsa-miR-24-2-5p (MIMAT0004497), hsa-let-7c-5p (MIMAT0000064) and hsa-miR-30a-3p (MIMAT0000088),
or else in combination with hsa-miR-99a-5p (MIMAT0000097), hsa-miR-125b-5p (MIMAT0000423), hsa-miR-194-5p (MIMAT0000460), hsa-miR-142-3p (MIMAT0000434) and hsa-miR-885-5p (MIMAT0004947),
or else in combination with hsa-miR-141-3p (MIMAT0000432), hsa-miR-30a-3p (MIMAT0000088), hsa-miR-24-2-5p (MIMAT0004497), hsa-miR-328-3p (MIMAT0000752), hsa-miR-375-3p (MIMAT0000728), hsa-miR-6847-5p (MIMAT0027594) and hsa-miR-424-3p (MIMAT0004749),
or else in combination with hsa-miR-125b-5p (MIMAT0000423), hsa-miR-150-5p (MIMAT0000451), hsa-miR-142-5p (MIMAT0000433), hsa-miR-223-3p (MIMAT0000280) and hsa-miR-338-3p (MIMAT0000763).

According to the use of the present invention said biomarkers can be detected by Real-Time PCR, Sequencing, such as, for example, RNA next generation sequencing, Droplet Digital PCR, Microarrays, Flow cytometry, or RNA hybridization methods such as Northern Blot or Dot Blot.

In particular, the sequencing technique can be used for the following miRNAs:
hsa-let-7c-5p (MIMAT0000064), hsa-let-7f-1-3p (MIMAT0004486), hsa-miR-100-5p (MIMAT0000098), hsa-miR-11401 (MIMAT0044658), hsa-miR-1246 (MIMAT0005898), hsa-miR-125a-3p (MIMAT0004602), hsa-miR-125b-2-3p (MIMAT0004603), hsa-miR-127-5p (MIMAT0004604), hsa-miR-134-5p (MIMAT0000447), hsa-miR-139-3p (MIMAT0004552), hsa-miR-141-3p (MIMAT0000432), hsa-miR-144-5p (MIMAT0004600), hsa-miR-145-5p (MIMAT0000437), hsa-miR-1468-5p (MIMAT0006789), hsa-miR-148a-5p (MIMAT0004549), hsa-miR-148b-5p (MIMAT0004699), hsa-miR-150-3p (MIMAT0004610), hsa-miR-150-5p (MIMAT0000451), hsa-miR-152-3p (MIMAT0000438), hsa-miR-181a-5p (MIMAT0000256), hsa-miR-185-5p (MIMAT0000455), hsa-miR-190a-5p (MIMAT0000458), hsa-miR-190b-5p (MIMAT0004929), hsa-miR-192-5p (MIMAT0000222), hsa-miR-1976 (MIMAT0009451),
   hsa-miR-200a-3p (MIMAT0000682), hsa-miR-205-5p (MIMAT0000266), hsa-miR-20a-5p (MIMAT0000075), hsa-miR-215-5p (MIMAT0000272), hsa-miR-224-5p (MIMAT0000281), hsa-miR-24-2-5p (MIMAT0004497), hsa-miR-30a-3p (MIMAT0000088), hsa-miR-30d-3p (MIMAT0004551), hsa-miR-3124-5p (MIMAT0014986), hsa-miR-3138 (MIMAT0015006), hsa-miR-323b-3p (MIMAT0015050), hsa-miR-328-3p (MIMAT0000752), hsa-miR-335-3p (MIMAT0004703), hsa-miR-335-5p (MIMAT0000765), hsa-miR-337-5p (MIMAT0004695), hsa-miR-342-5p (MIMAT0004694), hsa-miR-362-5p (MIMAT0000705), hsa-miR-3661 (MIMAT0018082), hsa-miR-370-3p (MIMAT0000722), hsa-miR-374b-3p (MIMAT0004956), hsa-miR-375-3p (MIMAT0000728), hsa-miR-376c-3p (MIMAT0000720), hsa-miR-377-5p (MIMAT0004689), hsa-miR-423-5p (MIMAT0004748), hsa-miR-424-3p (MIMAT0004749), hsa-miR-431-5p (MIMAT0001625), hsa-miR-432-5p (MIMAT0002814), hsa-miR-4745-5p (MIMAT0019878), hsa-miR-4775 (MIMAT0019931), hsa-miR-487b-3p (MIMAT0003180), hsa-miR-5009-5p (MIMAT0021041), hsa-miR-5010-5p (MIMAT0021043), hsa-miR-511-5p (MIMAT0002808), hsa-miR-539-3p (MIMAT0022705), hsa-miR-542-3p (MIMAT0003389), hsa-miR-5683 (MIMAT0022472), hsa-miR-577 (MIMAT0003242), hsa-miR-628-3p (MIMAT0003297), hsa-miR-6735-5p (MIMAT0027371), hsa-miR-6787-3p (MIMAT0027475), hsa-miR-6843-3p (MIMAT0027588), hsa-miR-6847-5p (MIMAT0027594), hsa-miR-6894-5p (MIMAT0027688), hsa-miR-766-3p (MIMAT0003888), hsa-miR-7705 (MIMAT0030020), hsa-miR-7976 (MIMAT0031179), hsa-miR-873-5p (MIMAT0004953), hsa-miR-874-3p (MIMAT0004911), hsa-miR-887-5p (MIMAT0026720), hsa-miR-92b-3p (MIMAT0003218) and hsa-miR-99a-5p (MIMAT0000097);
whereas the qPCR technique can be used for the following miRNAs:
   hsa-let-7b-3p (MIMAT0004482), hsa-let-7b-5p (MIMAT0000063), hsa-let-7c-5p (MIMAT0000064), hsa-let-7f-5p (MIMAT0000067), hsa-miR-122-5p (MIMAT0000421), hsa-miR-125b-5p (MIMAT0000423), hsa-miR-140-5p (MIMAT0000431), hsa-miR-142-3p (MIMAT0000434), hsa-miR-142-5p (MIMAT0000433), hsa-miR-150-5p (MIMAT0000451), hsa-miR-151a-5p (MIMAT0004697), hsa-miR-192-5p (MIMAT0000222), hsa-miR-194-5p (MIMAT0000460), hsa-miR-199a-5p (MIMAT0000231), hsa-miR-200a-3p (MIMAT0000682), hsa-miR-215-5p (MIMAT0000272), hsa-miR-223-3p (MIMAT0000280), hsa-miR-28-3p (MIMAT0004502), hsa-miR-301a-3p (MIMAT0000688), hsa-miR-30a-5p (MIMAT0000087), hsa-miR-326 (MIMAT0000756), hsa-miR-338-3p (MIMAT0000763), hsa-miR-339-5p (MIMAT0000764), hsa-miR-363-3p (MIMAT0000707), hsa-miR-375-3p (MIMAT0000728), hsa-miR-423-3p (MIMAT0001340), hsa-miR-486-5p (MIMAT0002177), hsa-miR-532-5p (MIMAT0002888), hsa-miR-584-5p (MIMAT0003249), hsa-miR-874-3p (MIMAT0004911), hsa-miR-885-5p (MIMAT0004947) and hsa-miR-99a-5p (MIMAT0000097).

The present invention will now be described by way of illustration and not by way of limitation, according to a preferred embodiment thereof, with particular reference to examples and to the figures in the appended drawings, wherein:

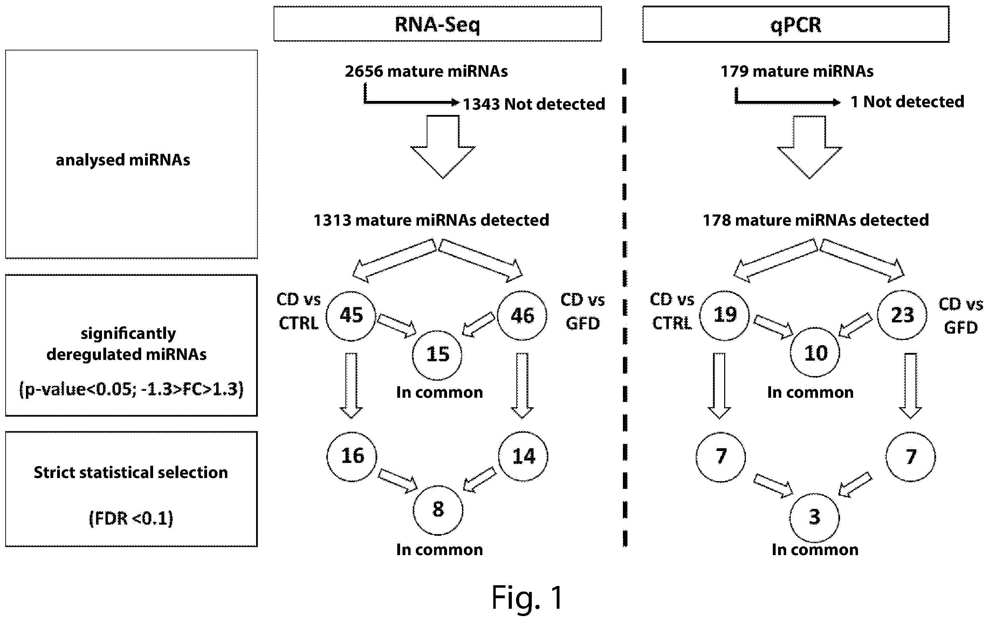

- **Figure 1** shows the workflow that led to the selection of the miRNAs which are the object of the present patent application. In particular, with the sequencing of circulating RNA, 1313 mature microRNAs were identified out of a total number of 2656 human microRNAs (miRBase rel.22.1), which were thus taken into consideration for the next steps, as they were expressed in all of the samples analysed (CTRL, GFD and CD). The analysis then focused on characterising which miRNAs were statistically significant (p-value<0.05) and deregulated in the CD samples compared to the CTRL or GFD samples (Fold Change (FC) greater than 1.3 or less than -1.3) until arriving at an even stricter selection (False Discovery Rate, FDR less than 0.1). In parallel to the sequencing, starting from a total number of 179 miRNAs, 178 miRNAs were identified by quantitative real-time PCR (qPCR) and the same selection criteria as used for the analysis of the miRNAs obtained by sequencing (p-value, FC and FDR) were followed.

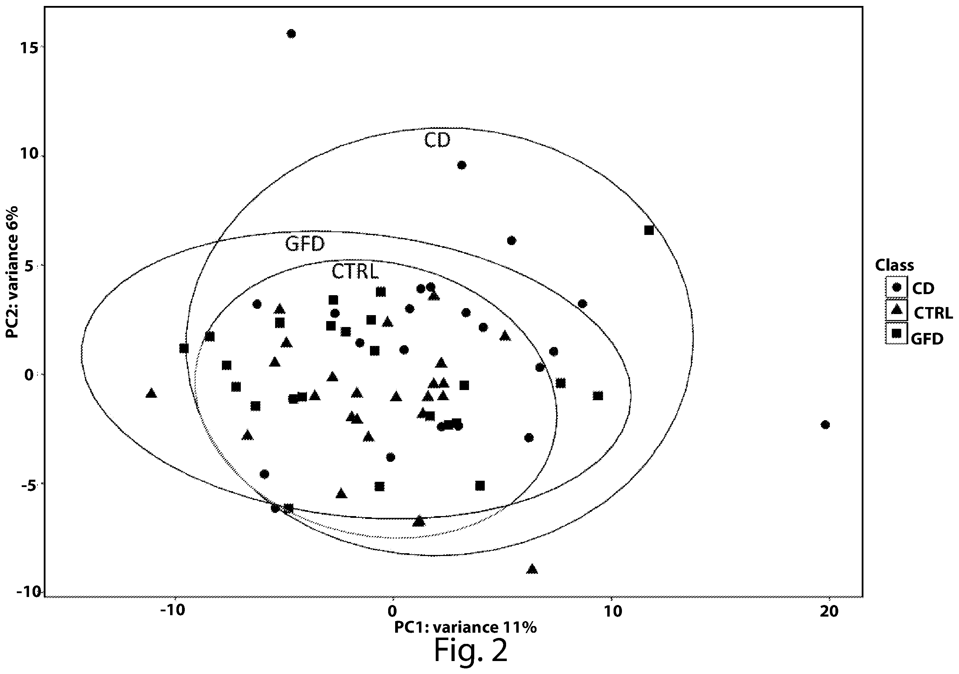

- **Figure 2** shows the expression of the circulating microRNAs analysed by principal component analysis (PCA) of the three groups of sequenced samples. As predicted, the distribution of the controls (CTRL) and of the subjects on a diet (GFD) is practically identical. In contrast, the distribution of subjects with celiac disease (CDs) is wider and deviates from that of the other two groups. The PCA analysis suggests that the diet can restore the expression of circulating microRNAs to levels comparable to those of healthy subjects.

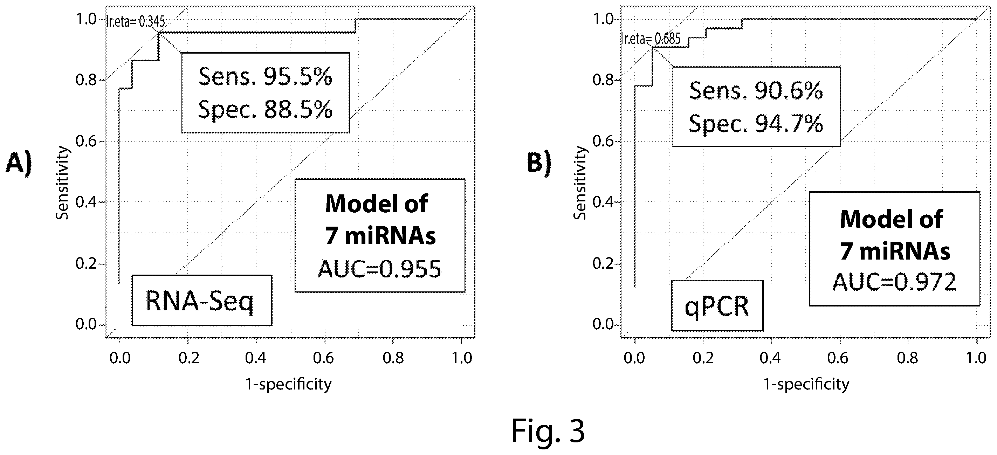

- **Figure 3** shows the ROC curve of the predictive model obtained starting from the 7 miRNAs identified with the RNA-Seq technique (hsa-miR-215-5p (MIMAT0000272), hsa-miR-141-3p (MIMAT0000432), hsa-miR-375-3p (MIMAT0000728), hsa-miR-192-5p (MIMAT0000222), hsa-miR-24-2-5p (MIMAT0004497), hsa-let-7c-5p (MIMAT0000064) and hsa-miR-30a-3p (MIMAT0000088)), which are highly significant (FDR<0.1) and deregulated (-1.3>FC>1.3) in CD compared to CTRL **(****Figure 3A****)** and the ROC curve of the predictive model obtained starting from the 7 miRNAs identified with the qPCR technique (hsa-miR-192-5p (MIMAT0000222), hsa-miR-215-5p (MIMAT0000272), hsa-miR-99a-5p (MIMAT0000097), hsa-miR-125b-5p (MIMAT0000423), hsa-miR-194-5p (MIMAT0000460), hsa-miR-142-3p (MIMAT0000434) and hsa-miR-885-5p (MIMAT0004947)), which are highly significant (FDR<0.1) and deregulated (-1.3>FC>1.3) in CD compared to CTRL.

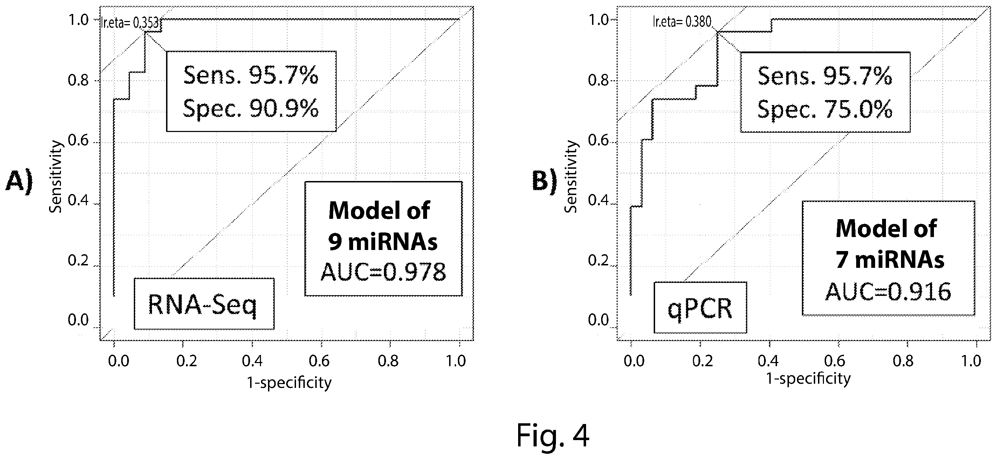

- **Figure 4** shows the ROC curve of the predictive model obtained starting from the 9 miRNAs identified with the RNA-Seq technique (hsa-miR-215-5p (MIMAT0000272), hsa-miR-141-3p (MIMAT0000432), hsa-miR-30a-3p (MIMAT0000088), hsa-miR-24-2-5p (MIMAT0004497), hsa-miR-328-3p (MIMAT0000752), hsa-miR-375-3p (MIMAT0000728), hsa-miR-6847-5p (MIMAT0027594), hsa-miR-192-5p (MIMAT0000222) and hsa-miR-424-3p (MIMAT0004749)), which are highly significant (FDR<0.1) and deregulated (-1.3>FC>1.3) in CD compared to GFD **(****Figure 4A****)** and the ROC curve of the predictive model obtained starting from the 7 miRNAs identified with the qPCR technique (hsa-miR-192-5p (MIMAT0000222), hsa-miR-125b-5p (MIMAT0000423), hsa-miR-215-5p (MIMAT0000272), hsa-miR-150-5p (MIMAT0000451), hsa-miR-142-5p (MIMAT0000433), hsa-miR-223-3p (MIMAT0000280) and hsa-miR-338-3p (MIMAT0000763)), which are highly significant (FDR<0.1) and deregulated (-1.3>FC>1.3) in CD compared to GFD **(****Figure 4B****).**

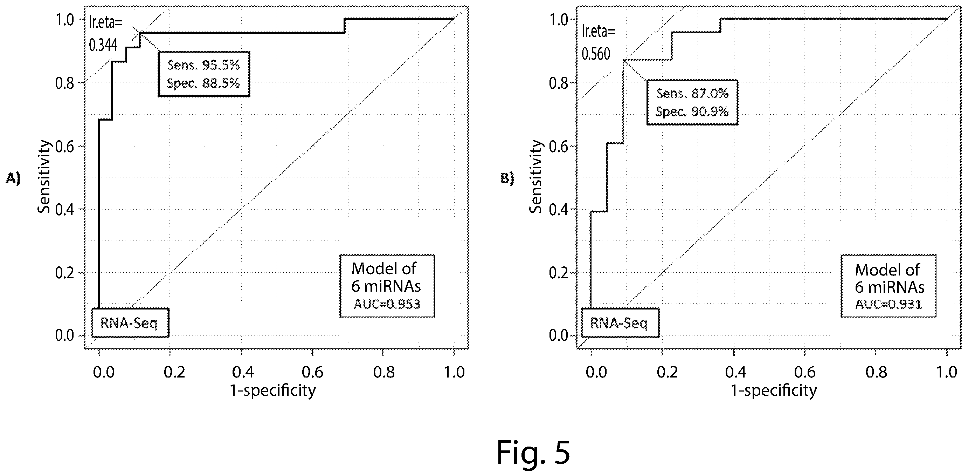

- **Figure 5** shows the ROC curve of the predictive model obtained starting from the 6 miRNAs identified with the RNA-Seq technique (hsa-miR-141-3p (MIMAT0000432), hsa-miR-192-5p (MIMAT0000222), hsa-miR-215-5p (MIMAT0000272), hsa-miR-24-2-5p (MIMAT0004497), hsa-miR-30a-3p (MIMAT0000088) and hsa-miR-375-3p (MIMAT0000728)), which are highly significant (FDR<0.1) and deregulated (-1.3>FC>1.3) in CD compared to CTRL **(****Figure 5A****)** and the ROC curve of the predictive model obtained starting from the same 6 miRNAs identified with the RNA-Seq technique, which are highly significant (FDR<0.1) and deregulated (-1.3>FC>1.3) in CD compared to GFD **(****Figure 5B****).** These miRNAs are expressed and identifiable both in the CD vs CTRL comparison and in the CD vs GFD comparison with the RNA-Seq technique.

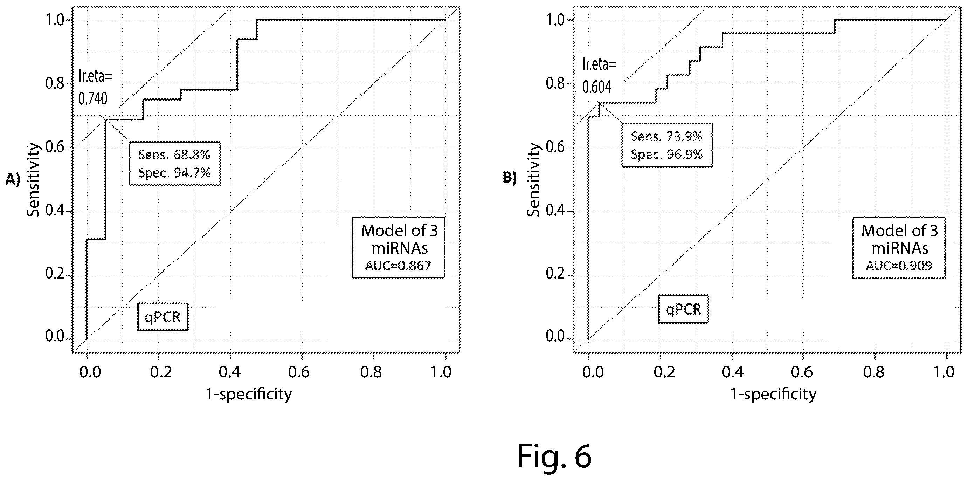

- **Figure 6** shows the ROC curve of the predictive model obtained starting from the 3 miRNAs identified with the qPCR technique (hsa-miR-125b-5p (MIMAT0000423), hsa-miR-192-5p (MIMAT0000222) and hsa-miR-215-5p (MIMAT0000272)), which are highly significant (FDR<0.1) and deregulated (-1.3>FC>1.3) in CD compared to CTRL **(****Figure 6A****)** and the ROC curve of the predictive model obtained starting from the same 3 miRNAs identified with the qPCR technique, which are highly significant (FDR<0.1) and deregulated (-1.3>FC>1.3) in CD compared to GFD **(****Figure 6B****).** These miRNAs are expressed and identifiable both in the CD vs CTRL comparison and in the CD vs GFD comparison with the qPCR technique.

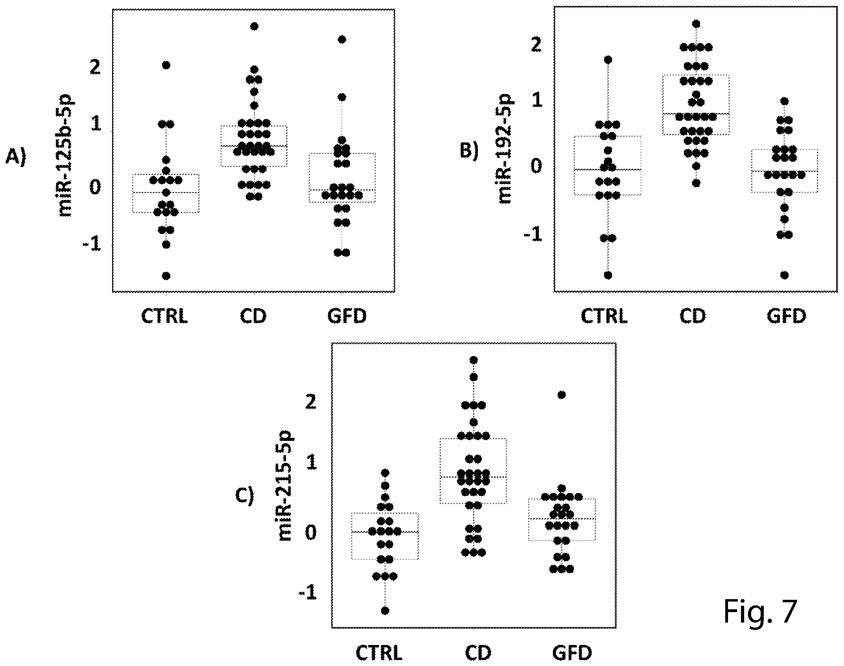

- **Figure 7** shows a dot plot which schematically illustrates the pattern of hsa-miR-125b-5p (MIMAT0000423) in the three groups of patients and controls considered (CTRL, CD and GFD) **(****Figure 7A). Figure 7B** shows a dot plot which schematically illustrates the pattern of hsa-miR-192-5p (MIMAT0000222) in the three groups of patients and controls considered (CTRL, CD and GFD). **Figure 7C** shows a dot plot which schematically illustrates the pattern of hsa-miR-215-5p (MIMAT0000272) in the three groups of patients and controls considered (CTRL, CD and GFD).

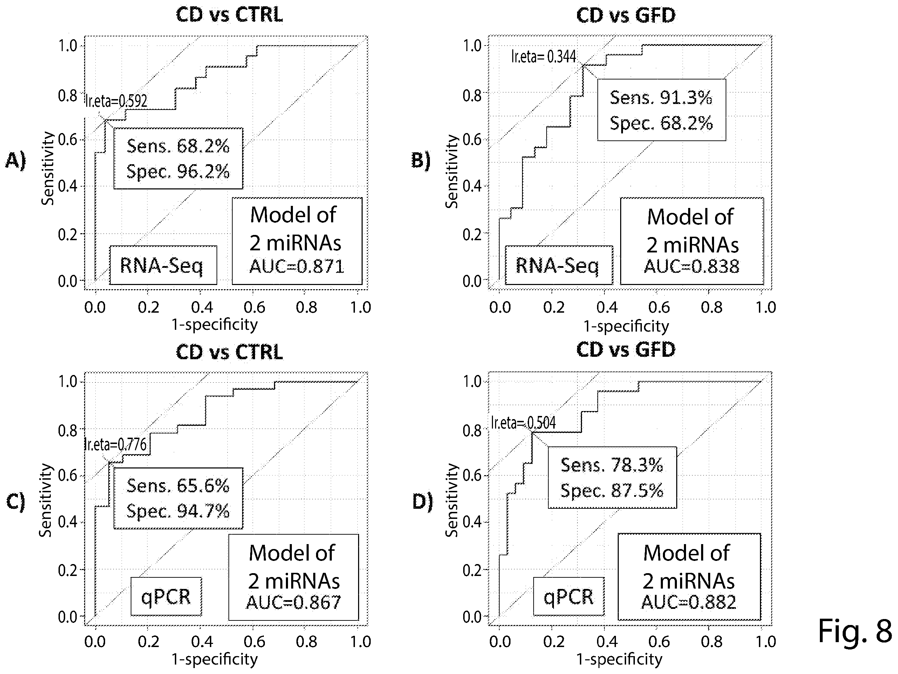

- **Figure 8** shows the ROC curves of the predictive model of the 2 miRNAs identified with the RNA-Seq technique (hsa-miR-192-5p (MIMAT0000222) and hsa-miR-215-5p (MIMAT0000272)), which are highly significant (FDR<0.1) and deregulated (-1.3>FC>1.3) in CD compared to CTRL **(****Figure 8A****)** and in CD compared to GFD **(****Figure 8B****),** which were also identified with the qPCR technique in CD compared to CTRL **(****Figure 8C****)** and in CD compared to GFD **(****Figure 8D****).**

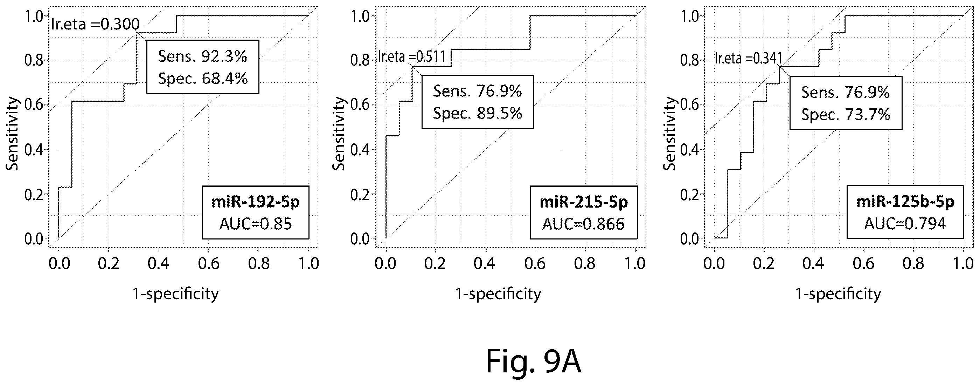

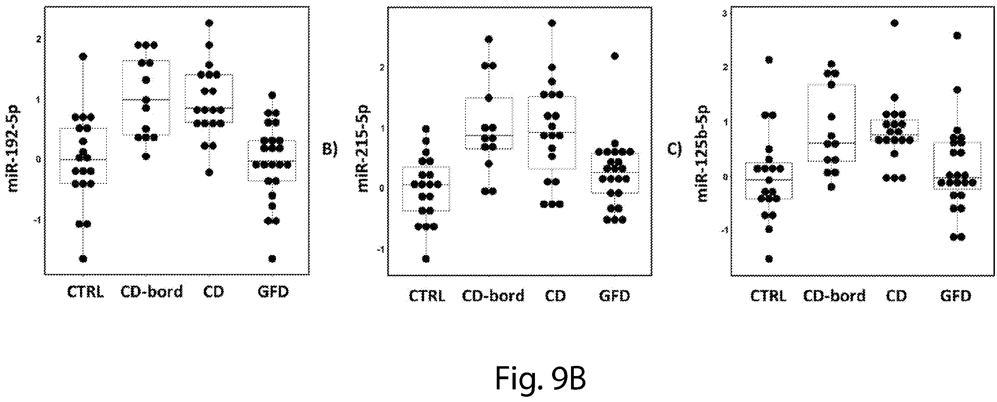

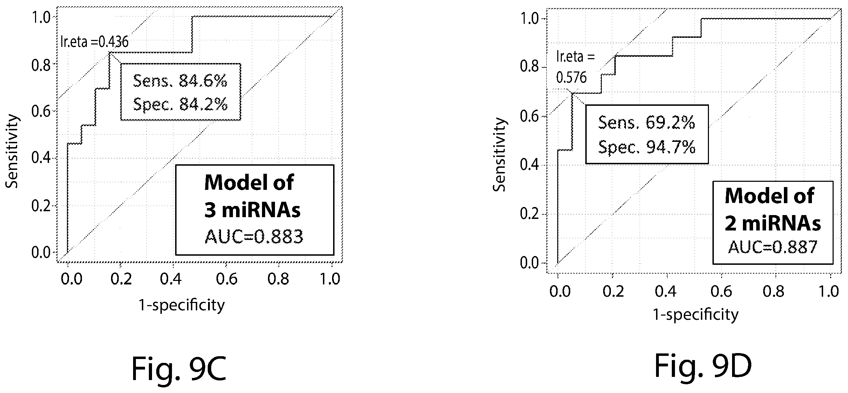

- **Figure 9** shows the ROC curves of the 3 miRNAs identified with the qPCR technique (hsa-miR-125b-5p (MIMAT0000423), hsa-miR-192-5p (MIMAT0000222) and hsa-miR-215-5p (MIMAT0000272)), which are highly significant (FDR<0.1) and deregulated (-1.3>FC>1.3) in the 'borderline' CD group compared to CTRL **(****Figure 9A****).** **Figure 9B** shows the dot plots of the 3 miRNAs identified with the qPCR technique (hsa-miR-125b-5p (MIMAT0000423), hsa-miR-192-5p (MIMAT0000222) and hsa-miR-215-5p (MIMAT0000272)) in the four groups of patients and controls considered (CTRL, CD, 'borderline' CD and GFD). **Figure 9C** shows the ROC curve of the predictive model of the 3 miRNAs identified with the qPCR technique (hsa-miR-125b-5p (MIMAT0000423), hsa-miR-192-5p (MIMAT0000222) and hsa-miR-215-5p (MIMAT0000272)) for the diagnosis of celiac disease in the 'borderline' CD patients. **Figure 9D** shows the ROC curve of the predictive model of the 2 miRNAs identified with the qPCR technique (hsa-miR-192-5p (MIMAT0000222) and hsa-miR-215-5p (MIMAT0000272)) for the diagnosis of celiac disease in the 'borderline' CD patients.

### EXAMPLE 1. Identification of circulating microRNAs deregulated in patients affected by celiac disease.

### Material and Methods

**Participants in the study.** A total number of 120 subjects divided into three groups (n = 40 for CD, n = 40 for GFD and n = 40 for CTRL) were recruited at the Bambino Gesù Children's Hospital in Rome in the Department of Hepatology, Gastroenterology and Nutrition. Each set was made up of 40 children with active CD, 40 CD subjects on a gluten-free diet (GFD) for over six months and 40 healthy subjects (controls, CTRL). A group of n = 14 children affected by IBD (n = 8 children with ulcerative colitis and n = 6 children with Crohn's disease) were recruited at the Paediatrics Department of the Marche Polytechnic University in Ancona (Italy) and considered as further controls. After a check on the haemolysis of the serum sample we excluded one sample of the CD group, seven samples of the GFD group and three samples of the CTRL group.

The demographic and clinical characteristics of the patients are shown in Table 1. All variables are expressed as means ± standard deviation (SD), whereas the qualitative data are expressed as percentages.

**Table 1**

| **Demographic characteristics** | **CTRL (n=37)** | **CD (n=39)** | **GFD (n=33)** | **p-value** |
|---|---|---|---|---|
| Sex | M=19, F=18 | M=15, F=24 | M=13, F=20 | 0.4598 (Chi-square) |
| Age (years) | 9.27(4.32) | 7.57(3.59) | 7.71(3.71) | 0.118 |
| BMI | 19.63(5.3 5) | 17.44(4.01) | 17.66(3.71) | 0.069 |
| Total serum IgA (mg/dL) | 138.8(53. 1) | 116.2(58.1) | 116.9(73.6) | 0.246 |
| Total serum tTGA at diagnosis (U/mL) | --- | 2036(3585) | --- | --- |
| Total serum tTGA after gluten-free diet (U/mL) | --- | --- | 3.7(10.4) | --- |
| EMA positivity (n, %) (at diagnosis) | --- | n=38 (97.4%) | n=26 (78.8%) | --- |

| **Histology** | **CTRL (n=37)** | **CD (n=39)** | **GFD (n=33)** | |
|---|---|---|---|---|
| Confirmed with biopsy (at diagnosis) | --- | n=13 | n=25 | |
| Confirmed according to ESPGHAN guidelines (at diagnosis) | --- | n=26 | n=2 | |
| not available/not performed | n=34 | n=26 | n=8 | |

| **Marsh type** | | | | |
|---|---|---|---|---|
| 0 | --- | --- | --- | |
| 1 | --- | --- | --- | |
| 2 | --- | --- | --- | |
| 3° | --- | n=3 | n=2 | |
| 3b | --- | n=8 | n=6 | |
| 3c | --- | n=2 | n=17 | |

| **Genetics** | | | | |
|---|---|---|---|---|
| DQ2/DQ8 | n=2 | n=32 | n=9 | |
| Negatives | n=1 | n=1 | --- | |
| not available | n=34 | n=6 | n=24 | |

A comparison of means among the groups was carried out using ANOVA or the Chi-Square test (contingency tables). A p value of less than 0.05 (p<0.05) was considered statistically significant. No patients with severe clinical or psychiatric conditions that could influence the evaluation of the protocol, with diabetes, with complications of any type or with other associated autoimmune diseases or subjects treated with drugs were enrolled.

*Written informed consent was obtained from all of the enrolled individuals and the Ethics Committee of the Bambino Gesù Children's Hospital approved the study (669_OPBG_2013; 10*/*07*/*2013).*

**Collection of serum samples.** Blood was collected in BD Vacutainer ^{®} SST ^{™} II Advance test tubes. The samples were processed within 2 hours of being drawn. The serum was obtained after centrifugation of the test tubes at 1800 g for 15 minutes at room temperature. In order to rule out haemolysis, the absorbance of the serum samples was measured at λ = 414 nm (determination of Nanodrop 2000) and at additional peaks of λ = 541 nm and λ = 576 nm. Samples with an absorbance at 414 nm greater than 0.2 (A414> 0.2) were considered haemolyzed and thus discarded. The collected serum was stored at -80°C until the analysis.

**RNA extraction.** The total RNA was isolated using a kit for circulating RNA and exosome purification (Norgen) starting from 250 µl or 500 µl of serum depending on the analysis to be carried out (250 µl for qPCR and 500 for per RNA-Seq), according to the manufacturer's instructions. The RNA was stored at -80° C until the time of use.

**Investigation of circulating microRNAs by RNA sequencing.** The circulating microRNAs were sequenced using the small RNA sequencing (RNA-Seq) protocol. The RNA samples were concentrated to about 5 µl using a SpeedVac instrument and the whole volume was used to construct small RNA libraries using the TruSeq SmallRNA Sample Prep kit (Illumina, San Diego, CA), following the manufacturer's instructions. Both the RNA samples and the final libraries were quantified using the Qubit 2.0 Fluorometer (Invitrogen, Carlsbad, CA) and the quality was evaluated with the Agilent 2100 Bioanalyzer RNA Nano assay (Agilent Technologies, Santa Clara, CA). The libraries were then processed with IIIBase cBot for the generation of clusters on the flow cell, following the manufacturer's instructions, and sequenced in the single-end mode at the multiplexing level required on a HiSeq 2000 (Illumina, San Diego, CA). CASAVA version 1.8.2 of the Illumina pipeline was used to process the raw data for format conversion and de-multiplexing.

**Bioinformatic analysis of RNA-Seq.** Quality controls were applied to the raw data using the FastQC package integrated into the Galaxy online data analysis platform (https://usegalaxy.org/). The adaptors were removed and bases of low quality were cut by means of the TrimGalore script. The reads were mapped on the human reference genome hg19 with Bowtie 2 (Galaxy Version 2.3.2.2) and the number of alignments was counted by means of the HTSeq-count script [18]. The differentially expressed miRNAs were obtained using the DESeq2 package [19] after applying corrections for the batch effect using the COMBAT correction method.

**Investigation of circulating microRNAs by qPCR.** The circulating microRNAs were also analysed by qPCR. The analyses were carried out using the Serum/Plasma PCR Panel I + II (Exiqon), which evaluated a total of 179 miRNAs. The RNA was reverse-transcribed in 25 µl using miRCURY LNA ^{™} Universal RT cDNA Synthesis (Exiqon), following the manufacturer's instructions. An RNA spike-in control (UniSp6) was used to monitor the efficiency of the reverse-transcription reaction. Then the cDNA was diluted (1:40) in nuclease-free water and the qPCR assay was performed in 10 µl using the ExiLENT SYBR^{®} Green Master Mix (Exiqon), according to the protocol. The qPCR reactions were run in a QuantStudio^{™} 12K Flex Real-Time PCR system using a PCR program which consists in a denaturation step at 95°C for 10 min followed by 40 cycles (95°C for 10 sec and 60°C for 1 min). A dissociation protocol was acquired after the last qPCR cycle to confirm the absence of nonspecific amplification products.

**Data analysis.** The raw data of the quantification cycle (Cq) were analysed using GenEx 6 software (MultiD Analyzes AB, Sweden) following the manufacturer's instructions. The qPCR values were normalized with the global average expression of all the genes [20] and analysis of variance (ANOVA) with correction for multiple comparisons. A value of p<0.05 was considered statistically significant. The diagnostic utility of the differentially expressed miRNAs was evaluated by calculating the receiver operating characteristic (ROC) curve with R/Bioconductor (https://www.bioconductor.org). The area beneath the curve (AUC) was determined in order to establish sensitivity and specificity [21].

### Results

**Characteristics of the cohorts.** The starting cohort consisted in 120 patients divided into three groups: CD (n=40), GFD (n=40) and CTRL (n=40). After a check on haemolysis was performed, one sample was excluded from CD, seven samples from GFD and three samples from CTRL. The clinical characteristics of the cohort considered are shown in Table 1. Positivity for IgA anti-EMA (at diagnosis) for the CD and GFD groups was evaluated in 88.8% of the patients.

Celiac disease was confirmed by intestinal biopsy in 52.7% of CD and GFD children, whereas in 38.8% of cases it was diagnosed in accordance with ESPGHAN guidelines. The biopsies were all classified as Marsh type 3 (n=5 grade 3a, n=14 grade 3b and n=19 grade 3c). It should be noted that 41 patients with celiac disease (CD and GFD) tested positive for DQ2/DQ8, one negative (but positive for DQA1*05, DQB1*0301/04, DRB1*11, DRB1*12) and 30 were not evaluated. The majority of the controls tested negative or were not evaluated (n=35), whereas two, despite having tested positive for DQ2, were asymptomatic. No genetic tests were performed in 30 CD and GFD patients and 31 controls. Biopsies were not performed on the CTRL group or on 47.2% of the CD or GFD patients (patients with celiac disease according to ESPGHAN guidelines). All of the children in the GFD group had been on a diet for at least six months.

**Sequencing of circulating microRNAs.** The sequencing of the RNA obtained from serum samples of the CD, GFD and CTRL patients produced a large set of reads (more than 10M of reads per sample), which were cut and mapped on the human genome in the genome positions of mature miRNAs (genome hg19), and the number of reads mapped on each position was calculated. For every sample the count of these positions made it possible to examine the coverage of every mature miRNA (a total number of 2656 miRNA) (Figure 1). Of the 2656 mature miRNAs, 1343 were not detected in all of the samples and the subsequent analyses were performed considering the remaining 1313 miRNAs. Subsequently, the principal component analysis (PCA) was used to represent the distribution of the miRNAs expressed among the three groups (CD, GFD and CTRL) (Figure 2). As expected, the distribution of samples in the CTRL and GFD groups is almost identical, whereas the CD group is wider and clearly distinct from the other two. This fact highlights that a gluten-free diet can restore (at least in part, if not completely) the profile of circulating microRNAs and that this change can be correlated to the restoration of a normal intestinal phenotype. It is interesting to note that, when comparing the GFD and CTRL profiles, no statistically significant miRNAs were found, once again confirming that the two groups are not significantly different, at least from a molecular viewpoint.

A list of the statistically deregulated miRNAs in the CD compared to the CTRL and GFD groups was subsequently extracted in order to identify the up- and down-regulated miRNAs in the pathological condition.

Thirteen microRNAs that were statistically significant (p-value<0.05) but had a fold change (compared to CTRL or compared to GFD) less than 1.3 or greater than -1.3 (-1.3<FC<1.3) were excluded from the subsequent analyses. Said microRNAs are the following: hsa-miR-10b-5p (MIMAT0000254), hsa-miR-125a-5p (MIMAT0000443), hsa-miR-143-3p (MIMAT0000435), hsa-miR-155-5p (MIMAT0000646), hsa-miR-181c-3p (MIMAT0004559), hsa-miR-27a-3p (MIMAT0000084), hsa-miR-30a-5p (MIMAT0000087), hsa-miR-30d-5p (MIMAT0000245), hsa-miR-30e-5p (MIMAT0000692), hsa-miR-361-5p (MIMAT0000703), hsa-miR-425-5p (MIMAT0003393), hsa-miR-769-5p (MIMAT0003886) and hsa-miR-98-5p (MIMAT0000096).

Table 2 lists the miRNAs that were significantly (p-value <0.05) deregulated (-1.3>FC>1.3) found in CD compared to CTRL (45 miRNA) and compared to GFD (46 miRNA).

**Table 2**

| | **CD vs CTRL** | | | | | **CD vs GFD** | | | | |
|---|---|---|---|---|---|---|---|---|---|---|
| **miRNA name** | **FoldChange** | **FDR** | **Sens.** | **Spec.** | **AUC** | **FoldChange** | **FDR** | **Sens.** | **Spec.** | **AUC** |
| hsa-let-7c-5p (MIMAT0000064) | 1.51 | **2.63E-02** | 63.6 | 80.8 | 0.719 | 1.37 | 1.65E-01 | 43.5 | 90.9 | 0.682 |
| hsa-let-7f-1-3p (MIMAT0004486) | 1.60 | 2.82E-01 | 45.5 | 88.5 | 0.619 | | | | | |
| hsa-miR-100-5p (MIMAT0000098) | 1.52 | **6.82E-02** | 95.5 | 26.9 | 0.533 | | | | | |
| hsa-miR-11401 (MIMAT0044658) | -1.50 | 3.13E-01 | 86.4 | 50 | 0.657 | | | | | |
| hsa-miR-1246 (MIMAT0005898) | 1.60 | 2.82E-01 | 86.4 | 57.7 | 0.698 | | | | | |
| hsa-miR-125a-3p (MIMAT0004602) | | | | | | -1.55 | 3.35E-01 | 39.1 | 90.9 | 0.632 |
| hsa-miR-125b-2-3p (MIMAT0004603) | 1.42 | **6.82E-02** | 54.5 | 84.6 | 0.673 | 1.42 | **9.33E-02** | 91.3 | 54.5 | 0.672 |
| hsa-miR-127-5p (MIMAT0004604) | -1.61 | 2.82E-01 | 54.5 | 73.1 | 0.622 | | | | | |
| hsa-miR-134-5p (MIMAT0000447) | | | | | | -1.62 | **7.09E-02** | 69.6 | 63.6 | 0.688 |
| hsa-miR-139-3p (MIMAT0004552) | | | | | | -1.69 | 2.01E-01 | 82.6 | 50 | 0.63 |
| hsa-miR-141-3p (MIMAT0000432) | 2.08 | **2.98E-05** | 77.3 | 84.6 | 0.834 | 1.91 | **1.03E-03** | 73.9 | 77.3 | 0.787 |
| hsa-miR-144-5p (MIMAT0004600) | -1.36 | 2.82E-01 | 27.3 | 92.3 | 0.572 | | | | | |
| hsa-miR-145-5p (MIMAT0000437) | -1.66 | 1.43E-01 | 45.5 | 80.8 | 0.621 | | | | | |
| hsa-miR-1468-5p (MIMAT0006789) | | | | | | 1.39 | 2.17E-01 | 45.5 | 86.4 | 0.66 |
| hsa-miR-148a-5p (MIMAT0004549) | | | | | | -1.30 | 2.48E-01 | 87 | 40.9 | 0.623 |
| hsa-miR-148b-5p (MIMAT0004699) | -1.36 | 1.43E-01 | 100 | 26.9 | 0.572 | | | | | |
| hsa-miR-150-3p (MIMAT0004610) | | | | | | 1.55 | 2.92E-01 | 65.2 | 77.3 | 0.745 |
| hsa-miR-150-5p (MIMAT0000451) | 1.57 | **6.10E-02** | 54.5 | 73.1 | 0.635 | 1.50 | 1.37E-01 | 100 | 22.7 | 0.573 |
| hsa-miR-152-3p (MIMAT0000438) | -1.37 | 2.64E-01 | 27.3 | 88.5 | 0.523 | | | | | |
| hsa-miR-181a-5p (MIMAT0000256) | 1.41 | 3.48E-01 | 54.5 | 84.6 | 0.643 | | | | | |
| hsa-miR-185-5p (MIMAT0000455) | | | | | | -1.56 | **7.09E-02** | 52.2 | 77.3 | 0.642 |
| hsa-miR-190a-5p (MIMA T0000458) | | | | | | -1.68 | 2.09E-01 | 65.2 | 77.3 | 0.719 |
| hsa-miR-190b-5p (MIMAT0004929) | | | | | | -1.72 | 1.84E-01 | 52.2 | 95.5 | 0.646 |
| hsa-miR-192-5p (MIMAT0000222) | 1.68 | **9.59E-05** | 54.5 | 96.2 | 0.766 | 1.55 | **4.56E-03** | 91.3 | 54.5 | 0.709 |
| hsa-miR-1976 (MIMAT0009451) | -1.55 | 3.48E-01 | 72.7 | 66.4 | 0.696 | -1.72 | 1.81E-01 | 52.2 | 81.8 | 0.654 |
| hsa-miR-200a-3p (MIMAT0000682) | | | | | | 1.74 | 1.91E-01 | 56.5 | 81.8 | 0.7 |
| hsa-miR-205-5p (MIMAT0000266) | 1.57 | 3.13E-01 | 63.6 | 76.9 | 0.675 | | | | | |
| hsa-miR-20a-5p (MIMAT0000075) | | | | | | -1.33 | 3.35E-01 | 56.5 | 86.4 | 0.67 |
| hsa-miR-215-5p (MIMAT0000272) | 3.55 | **1.95E-10** | 72.7 | 96.2 | 0.867 | 2.58 | **2.55E-05** | 87 | 72.7 | 0.832 |
| hsa-miR-224-5p (MIMAT0000281) | 1.53 | 1.59E-01 | 86.4 | 42.3 | 0.619 | 1.51 | 2.09E-01 | 52.2 | 86.4 | 0.67 |
| hsa-miR-24-2-5p (MIMAT0004497) | -1.51 | **5.36E-02** | 68.2 | 73.1 | 0.724 | -1.51 | **6.63E-02** | 56.5 | 90.9 | 0.747 |
| hsa-miR-30a-3p (MIMAT0000088) | 1.47 | **6.82E-02** | 63.6 | 80.8 | 0.708 | 1.48 | **9.33E-02** | 95.7 | 63.6 | 0.783 |
| hsa-miR-30d-3p (MIMAT0004551) | | | | | | -1.37 | 3.33E-01 | 69.6 | 59.1 | 0.64 |
| hsa-miR-3124-5p (MIMAT0014986) | | | | | | -1.50 | 3.15E-01 | 47.8 | 90.9 | 0.632 |
| hsa-miR-3138 (MIMAT0015006) | | | | | | 1.62 | 2.64E-01 | 30.4 | 95.5 | 0.609 |
| hsa-miR-323b-3p (MIMAT0015050) | -1.73 | **6.82E-02** | 86.4 | 34.6 | 0.596 | | | | | |
| hsa-miR-328-3p (MIMAT0000752) | | | | | | -1.49 | **6.35E-02** | 52.2 | 95.5 | 0.735 |
| hsa-miR-335-3p (MIMAT0004703) | -1.42 | 3.48E-01 | 36.4 | 88.5 | 0.615 | | | | | |
| hsa-miR-335-5p (MIMAT0000765) | -1.52 | **6.82E-02** | 31.8 | 100 | 0.654 | | | | | |
| hsa-miR-337-5p (MIMAT0004695) | | | | | | -1.63 | 2.17E-01 | 56.5 | 90.9 | 0.709 |
| hsa-miR-342-5p (MIMAT0004694) | | | | | | 1.43 | 2.92E-01 | 60.9 | 72.7 | 0.642 |
| hsa-miR-362-5p (MIMAT0000705) | | | | | | -1.56 | 3.15E-01 | 60.9 | 68.2 | 0.626 |
| hsa-miR-3661 (MIMAT0018082) | | | | | | 1.87 | **9.33E-02** | 95.7 | 45.5 | 0.652 |
| hsa-miR-370-3p (MIMAT0000722) | -1.78 | **2.18E-02** | 63.6 | 73.1 | 0.652 | -1.54 | 1.59E-01 | 82.6 | 63.6 | 0.686 |
| hsa-miR-374b-3p (MIMAT0004956) | 1.49 | 3.71E-01 | 68.2 | 73.1 | 0.657 | | | | | |
| hsa-miR-375-3p (MIMAT0000728) | 2.26 | **2.59E-05** | 68.2 | 92.3 | 0.809 | 1.81 | **1.16E-02** | 91.3 | 50 | 0.729 |
| hsa-miR-376c-3p (MIMAT0000720) | -1.58 | 3.13E-01 | 45.5 | 84.6 | 0.657 | | | | | |
| hsa-miR-377-5p (MIMAT0004689) | | | | | | -1.63 | 2.64E-01 | 43.5 | 95.5 | 0.708 |
| hsa-miR-423-5p (MIMAT0004748) | 1.67 | **6.82E-02** | 40.9 | 88.5 | 0.629 | 1.84 | **3.66E-02** | 52.2 | 86.4 | 0.694 |
| hsa-miR-424-3p (MIMAT0004749) | | | | | | -1.77 | **7.09E-02** | 87 | 54.5 | 0.709 |
| hsa-miR-431-5p (MIMAT0001625) | -1.51 | 3.71E-01 | 40.9 | 92.3 | 0.624 | | | | | |
| hsa-miR-432-5p (MIMAT0002814) | | | | | | -1.36 | 3.25E-01 | 95.7 | 40.9 | 0.674 |
| hsa-miR-4745-5p (MIMAT0019878) | 1.56 | 3.13E-01 | 22.7 | 96.2 | 0.577 | 1.53 | 3.35E-01 | 43.5 | 77.3 | 0.573 |
| hsa-miR-4775 (MIMAT0019931) | -1.59 | 3.13E-01 | 54.5 | 88.5 | 0.705 | | | | | |
| hsa-miR-487b-3p (MIMAT0003180) | | | | | | -1.52 | 2.17E-01 | 56.5 | 81.8 | 0.698 |
| hsa-miR-5009-5p (MIMAT0021041) | 1.43 | 3.60E-01 | 68.2 | 65.4 | 0.624 | | | | | |
| hsa-miR-5010-5p (MIMAT0021043) | | | | | | 1.54 | 3.44E-01 | 60.9 | 68.2 | 0.65 |
| hsa-miR-511-5p (MIMAT0002808) | 1.55 | 3.48E-01 | 45.5 | 84.6 | 0.675 | | | | | |
| hsa-miR-539-3p (MIMAT0022705) | | | | | | -1.57 | 3.25E-01 | 56.5 | 77.3 | 0.613 |
| hsa-miR-542-3p (MIMAT0003389) | | | | | | -1.46 | 3.35E-01 | 30.4 | 100 | 0.53 |
| hsa-miR-5683 (MIMAT0022472) | 1.55 | 3.60E-01 | 27.3 | 100 | 0.601 | 1.56 | 3.35E-01 | 82.6 | 45.5 | 0.599 |
| hsa-miR-577 (MIMAT0003242) | 1.74 | 1.43E-01 | 72.7 | 69.2 | 0.703 | | | | | |
| hsa-miR-628-3p (MIMAT0003297) | 1.35 | 1.27E-01 | 95.5 | 23.1 | 0.5 | | | | | |
| hsa-miR-6735-5p (MIMAT0027371) | | | | | | 1.81 | 1.02E-01 | 100 | 36.4 | 0.733 |
| hsa-miR-6787-3p (MIMAT0027475) | 1.46 | 3.60E-01 | 62.8 | 53.8 | 0.523 | | | | | |
| hsa-miR-6843-3p | -1.56 | 3.48E-01 | 77.3 | 61.5 | 0.67 | | | | | |
| (MIMAT0027588) | | | | | | | | | | |
| hsa-miR-6847-5p (MIMAT00027594) | | | | | | 1.88 | **9.33E-02** | 78.3 | 63.6 | 0.713 |
| hsa-miR-6894-5p (MIMAT0027688) | 1.53 | 2.99E-01 | 31.8 | 96.2 | 0.517 | | | | | |
| hsa-miR-766-3p (MIMAT0003888) | -1.43 | 3.48E-01 | 90.9 | 53.8 | 0.717 | | | | | |
| hsa-miR-7705 (MIMAT0030020) | | | | | | -1.55 | 3.35E-01 | 56.5 | 77.3 | 0.68 |
| hsa-miR-7976 (MIMAT0031179) | 1.53 | 3.48E-01 | 31.8 | 92.3 | 0.575 | | | | | |
| hsa-miR-873-5p (MIMAT0004953) | | | | | | -1.52 | 3.15E-01 | 52.2 | 81.8 | 0.626 |
| hsa-miR-874-3p (MIMAT0004911) | 1.52 | 2.10E-01 | 68.2 | 76.9 | 0.664 | | | | | |
| hsa-miR-987-5p (MIMAT0026720) | | | | | | -1.51 | 3.54E-01 | 82.6 | 59.1 | 0.729 |
| usa-miR⁻92b-3p (MIMAT0003218) | 1.45 | **9.59E-02** | 36.4 | 96.2 | 0.642 | | | | | |
| hsa-miR-99a-5p (MIMAT0000097) | 1.58 | **1.47E-02** | 77.3 | 46.2 | 0.577 | | | | | |

From these two lists it is possible to infer that 15 miRNAs are in common, i.e. are useful both as biomarkers of celiac disease and as biomarkers of adherence to the diet: (hsa-let-7c-5p (MIMAT0000064), hsa-miR-125b-2-3p (MIMAT0004603), hsa-miR-141-3p (MIMAT0000432), hsa-miR-150-5p (MIMAT0000451), hsa-miR-192-5p (MIMAT0000222), hsa-miR-1976 (MIMAT0009451), hsa-miR-215-5p (MIMAT0000272), hsa-miR-224-5p (MIMAT0000281), hsa-miR-24-2-5p (MIMAT0004497), hsa-miR-30a-3p (MIMAT0000088), hsa-miR-370-3p (MIMAT0000722), hsa-miR-375-3p (MIMAT0000728), hsa-miR-423-5p (MIMAT0004748), hsa-miR-4745-5p (MIMAT0019878) and hsa-miR-5683 (MIMAT0022472)). Applying the multiple correction (FDR <0.1) it was found that 16 miRNAs are deregulated in CD compared to CTRL. In particular, 12 miRNAs are over-expressed (hsa-let-7c-5p (MIMAT0000064), hsa-miR-100-5p (MIMAT0000098), hsa-miR-125b-2-3p (MIMAT0004603), hsa-miR-141-3p (MIMAT0000432), hsa-miR-150-5p (MIMAT0000451), hsa-miR-192-5p (MIMAT0000222), hsa-miR-215-5p (MIMAT0000272), hsa-miR-30a-3p (MIMAT0000088), hsa-miR-375-3p (MIMAT0000728), hsa-miR-423-5p (MIMAT0004748), hsa-miR-92b-3p (MIMAT0003218) and hsa-miR-99a-5p (MIMAT0000097)) and 4 miRNAs are down-expressed (hsa-miR-24-2-5p (MIMAT0004497), hsa-miR-323b-3p (MIMAT0015050), hsa-miR-335-5p (MIMAT0000765) and hsa-miR-370-3p (MIMAT0000722)).

Furthermore, 14 miRNAs are deregulated in CD compared to GFD. In particular, 9 miRNAs are over-expressed (hsa-miR-125b-2-3p (MIMAT0004603), hsa-miR-141-3p (MIMAT0000432), hsa-miR-192-5p (MIMAT0000222), hsa-miR-215-5p (MIMAT0000272), hsa-miR-30a-3p (MIMAT0000088), hsa-miR-3661 (MIMAT0018082), hsa-miR-375-3p (MIMAT0000728), hsa-miR-423-5p (MIMAT0004748) and hsa-miR-6847-5p (MIMAT0027594)) and 5 miRNAs are down-expressed (hsa-miR-134-5p (MIMAT0000447), hsa-miR-185-5p (MIMAT0000455), hsa-miR-24-2-5p (MIMAT0004497), hsa-miR-328-3p (MIMAT0000752) and hsa-miR-424-3p (MIMAT0004749)).

Only 8 miRNAs were present in both lists with a comparable fold change (FC), i.e. are useful both as biomarkers of celiac disease and as biomarkers of adherence to the diet: hsa-miR-125b-2-3p (MIMAT0004603), hsa-miR-141-3p (MIMAT0000432), hsa-miR-192-5p (MIMAT0000222), hsa-miR-215-5p (MIMAT0000272), hsa-miR-24-2-5p (MIMAT0004497), hsa-miR-30a-3p (MIMAT0000088), hsa-miR-375-3p (MIMAT0000728) and hsa-miR-423-5p (MIMAT0004748). This highlights that the expression of these miRNAs is specifically altered in CD, and that the GFD is sufficient to restore the expression thereof to levels comparable to those of healthy subjects. Therefore, these miRNAs were further validated as biomarkers suitable for diagnosing celiac disease and for monitoring adherence to the GFD.

**qPCR evaluation of circulating microRNAs.** Independently of the sequencing, the different expression of circulating miRNAs was also evaluated by quantitative PCR (qPCR). In particular, use was made of a specifically designed technology optimized in order to detect up to 179 miRNAs generally circulating in the serum/plasma (Figure 1).

Identification of the circulating microRNAs by means of this technique takes place using PCR primers having specific sequences. In particular, at least one of the two PCR primers generally has the same sequence as the mature microRNA to be analysed. For this reason, genome sequences that are homologous but belong to other animal or plant species could also be used for the analysis of human circulating microRNAs.

Sixteen microRNAs that were statistically significant (p-value<0.05), but had a fold change (compared to CTRL or compared to GFD) less than 1.3 or greater than -1.3 (-1.3<FC<1.3) were excluded from the subsequent analyses. Said microRNAs are the following: hsa-let-7a-5p (MIMAT0000062), hsa-let-7d-3p (MIMAT0004484), hsa-let-7d-5p (MIMAT0000065), hsa-miR-103a-3p (MIMAT0000101), hsa-miR-107 (MIMAT0000104), hsa-miR-146a-5p (MIMAT0000449), hsa-miR-151a-5p (MIMAT0004697), hsa-miR-210-3p (MIMAT0000267), hsa-miR-21-5p (MIMAT0000076), hsa-miR-222-3p (MIMAT0000279), hsa-miR-26a-5p (MIMAT0000082), hsa-miR-27b-3p (MIMAT0000419), hsa-miR-30c-5p (MIMAT0000244), hsa-miR-320b (MIMAT0005792), hsa-miR-331-3p (MIMAT0000760) and hsa-miR-92a-3p (MIMAT0000092).

Based on the qPCR results, two lists of circulating microRNAs that are significantly (p value<0.05) deregulated (-1.3>FC>1.3) in CD patients compared to the CTRL (19 miRNA) or in CD compared to GFD patients (23 miRNA) (Table 3) were obtained.

**Table 3**

| | **CD vs CTRL** | | | | | **CD vs GFD** | | | | |
|---|---|---|---|---|---|---|---|---|---|---|
| **miRNA name** | **Fold Change** | **FDR** | **Sens.** | **Spec.** | **AUC** | **Fold Change** | **FDR** | **Sens.** | **Spec.** | **Fold Change** |
| hsa-let-7b-3p (MIMAT0004482) | 1.48 | 2.46E-01 | 68.8 | 73.7 | 0.701 | | | | | |
| hsa-let-7b-5p (MIMAT0000063) | | | | | | 1.34 | 1.56E-01 | 69.6 | 68.8 | 0..693 |
| hsa-let-7c-5p (MIMAT0000064) | -1.36 | 2.70E-01 | 84.4 | 52.6 | 0.689 | | | | | |
| hsa-let-7f-5p (MIMAT0000067) | -1.44 | 1.45E-01 | 78.1 | 63.2 | 0.73 | | | | | |
| hsa-miR-122-5p (MIMAT0000421) | | | | | | 1.96 | 1.99E-01 | 73.9 | 59.4 | 0.693 |
| hsa-miR-125b-5p (MIMAT0000423) | 1.80 | **1.22E-02** | 78.1 | 78.9 | 0.803 | 1.60 | **5.89E-02** | 56.5 | 93.8 | 0..785 |
| hsa-miR-140-5p (MIMAT0000431) | | | | | | -1.31 | 1.92E-01 | 73.9 | 56.2 | 0.662 |
| hsa-miR-142-3p (MIMAT0000434) | **-**1.44 | **8.51E-02** | 59.4 | 89.5 | 0.748 | | | | | |
| hsa-miR-142-5p (MiMAT0000433) | | | | | | -1.48 | **6.24E-02** | 87 | 53.1 | 0.739 |
| hsa-miR-150-5p (MIMAT0000451) | | | | | | 1.65 | **1.93E-02** | 52.2 | 90.6 | 0..757 |
| hsa-miR-151a-5p (MIMAT0004697) | | | | | | -1.30 | 1.64E-01 | 65.2 | 81.2 | 0.695 |
| hsa-miR-192-5p (MIMAT0000222) | 2..00 | **9.35E-04** | 62.5 | 94.7 | 0.854 | 2.04 | **4.36E-05** | 78.3 | 84.4 | 0.879 |
| hsa-miR-1914-5p (MIMAT0000460) | 1.66 | **2.06E-02** | 71.9 | 78.9 | 0.789 | 1.42 | 1.57E-01 | 82.6 | 68.8 | 0..732 |
| hsa-miR-199a-5p (MIMAT0000231) | | | | | | -1.42 | 2.75E-01 | 95.7 | 34.4 | 0.625 |
| hsa-miR-200a-3p (MIMAT0000682) | 1.81 | 2.75E-01 | 75 | 84.2 | 0.765 | | | | | |
| hsa-miR-215-5p (MIMAT0000272) | 1.97 | **1.73E-03** | 71.9 | 89.5 | 0.842 | 1.63 | **3.78E-02** | 87 | 71.9 | 0.783 |
| hsa-miR-223-3p (MIMAT0000280) | | | | | | -1.57 | **6.17E-02** | 60.9 | 81.2 | 0.73 |
| hsa-miR-28-3p (MIMAT0004502) | -1.32 | 1.47E-01 | 68.8 | 84.2 | 0.725 | | | | | |
| hsa-MiR-301a-3p (MIMAT0000688) | | | | | | -1.36 | 1.99E-01 | 73.9 | 68.8 | 0.692 |
| hsa-miR-30a-5p (MIMAT0000087) | | | | | | 1.78 | 1.50E-01 | 69.6 | 62.5 | 0.698 |
| hsa-miR-326 (MIMAT0000756) | -1.83 | 2.84E-01 | 75 | 57.9 | 0.656 | -1.89 | 1.84E-01 | 73.9 | 59.4 | 0.678 |
| hsa-MiR-338-3p (M IMAT0000763) | | | | | | -1.77 | **6.15E-02** | 56.5 | 84.4 | 0.73 |
| hsa-miR-339-5p (MiMAT0000764) | -1.67 | 1.55E-01 | 56.2 | 89.5 | 0.735 | -1.51 | 2.08E-01 | 87 | 56.2 | 0.681 |
| hsa-miR-363-3p (MIMAT0000707) | 1.34 | 2.31 E-01 | 96.9 | 42.1 | 0.653 | 1.36 | 1.49E-01 | 73.9 | 62.5 | 0.685 |
| hsa-miR-375-3p (MIMAT0000728) | | | | | | 1.89 | 1.52E-01 | 82.6 | 68.8 | 0..742 |
| hsa-miR-423-3p (MIMAT0001340) | | | | | | -1.32 | 1.81E-01 | 78.3 | 59.4 | 0.694 |
| hsa-miR-486-5p (MIMAT0002177) | 1.38 | 2.78E-01 | 75 | 68.4 | 0.699 | 1.43 | 1.91E-01 | 69.6 | 75 | 0.688 |
| hsa-miR-532-5p (MIMAT0002888) | 1.46 | 2.66E-01 | 81.2 | 57.9 | 0.697 | | | | | |
| hsa-miR-584-5p (MIMAT0003249) | -1.52 | 2.74E-01 | 84.4 | 42.1 | 0.63 | | | | | |
| hsa-miR-874-3p (MIMAT0004911) | 1.92 | 2.04E-01 | 78.1 | 68.4 | 0.725 | | | | | |
| hsa-miR-885-5p (MIMAT0004947) | 1.92 | **8.19.E-02** | 71.9 | 73.7 | 0.737 | 1.79 | 1.66E-01 | 82.6 | 53.1 | 0.702 |
| hsa-miR-99a-5p (MIMAT0000097) | 1.71 | **8.09E-03** | 84.4 | 73.7 | 0.808 | 1.39 | 2.42E-01 | 87 | 50 | 0.694 |

An analysis of these lists showed that 10 miRNAs were significantly deregulated (8 over-expressed and 2 down-expressed) in CD patients compared both to the CTRL and GFD patients: hsa-miR-125b-5p (MIMAT0000423), hsa-miR-192-5p (MIMAT0000222), hsa-miR-194-5p (MIMAT0000460), hsa-miR-215-5p (MIMAT0000272), hsa-miR-326 (MIMAT0000756), hsa-miR-339-5p (MIMAT0000764), hsa-miR-363-3p (MIMAT0000707), hsa-miR-486-5p (MIMAT0002177), hsa-miR-885-5p (MIMAT0004947) and hsa-miR-99a-5p (MIMAT0000097).

When the most significant (FDR <0.1) miRNAs were selected from Table 3 it was found that 7 miRNAs (hsa-miR-125b-5p (MIMAT0000423), hsa-miR-142-3p (MIMAT0000434), hsa-miR-192-5p (MIMAT0000222), hsa-miR-194-5p (MIMAT0000460), hsa-miR-215-5p (MIMAT0000272), hsa-miR-885-5p (MIMAT0004947) and hsa-miR-99a-5p (MIMAT0000097)) were differentially expressed in CD compared to CTRL: 6 miRNAs are over-expressed (hsa-miR-125b-5p (MIMAT0000423), hsa-miR-192-5p (MIMAT0000222), hsa-miR-194-5p (MIMAT0000460), hsa-miR-215-5p (MIMAT0000272), hsa-miR-885-5p (MIMAT0004947) and hsa-miR-99a-5p (MIMAT0000097)) and 1 miRNA is down-expressed (hsa-miR-142-3p (MIMAT0000434)). When the CD was compared with GFD, 7 deregulated miRNAs (FDR <0.1) were obtained (hsa-miR-125b-5p (MIMAT0000423), hsa-miR-142-5p (MIMAT0000433), hsa-miR-150-5p (MIMAT0000451), hsa-miR-192-5p (MIMAT0000222), hsa-miR-215-5p (MIMAT0000272), hsa-miR-223-3p (MIMAT0000280) and hsa-miR-338-3p (MIMAT0000763)), of which 4 miRNAs are over-expressed (hsa-miR-125b-5p (MIMAT0000423), hsa-miR-150-5p (MIMAT0000451), hsa-miR-192-5p (MIMAT0000222) and hsa-miR-215-5p (MIMAT0000272)) and 3 miRNAs are down-expressed (hsa-miR-142-5p (MIMAT0000433), hsa-miR-223-3p (MIMAT0000280) and hsa-miR-338-3p (MIMAT0000763)). Only 3 miRNAs were present in both lists (hsa-miR-125b-5p (MIMAT0000423), hsa-miR-192-5p (MIMAT0000222) and hsa-miR-215-5p (MIMAT0000272)).

With this technique (qPCR) as well, it was observed that when the multiple correction was applied (FDR<0.1), no miRNA showed to be statistically significant, confirming once again that the differences between the GFD and CTRL groups are negligible, at least as regards circulating miRNAs.

By qPCR analysis it was confirmed that the deregulation of circulating microRNAs observed with sequencing was also observable with other techniques. All the statistically deregulated miRNAs (FDR <0.1) obtained by small RNA-Seq were also present in the panel of miRNAs evaluated with qPCR. It is interesting to note that among the most significant (FDR <0.1) miRNAs obtained by qPCR, two of them (namely hsa-miR-192-5p (MIMAT0000222) and hsa-miR-215-5p (MIMAT0000272)) are in common with those found with the sequencing technique in both comparisons: CD versus CTRL and CD versus GFD.

**Comparison with miRNA expression of other intestinal diseases.** In order to assess whether the statistically modulated miRNAs described above are characteristic of and specific to CD and not to other intestinal diseases (e.g. ulcerative colitis or Crohn's disease), the expression of circulating microRNAs was analysed by qPCR in a group of patients (n = 14) with IBD. The results (not shown) revealed that patients with IBD had numerous deregulated miRNAs compared to the CTRL, but surprisingly none of the 72 microRNAs characteristic of celiac disease, listed below, were expressed in patients with IBD: hsa-let-7b-5p (MIMAT0000063), hsa-let-7c-5p (MIMAT0000064), hsa-let-7f-1-3p (MIMAT0004486), hsa-miR-11401 (MIMAT0044658), hsa-miR-1246 (MIMAT0005898), hsa-miR-125a-3p (MIMAT0004602), hsa-miR-125b-2-3p (MIMAT0004603), hsa-miR-125b-5p (MIMAT0000423), hsa-miR-127-5p (MIMAT0004604), hsa-miR-134-5p (MIMAT0000447), hsa-miR-139-3p (MIMAT0004552), hsa-miR-141-3p (MIMAT0000432), hsa-miR-142-3p (MIMAT0000434), hsa-miR-144-5p (MIMAT0004600), hsa-miR-1468-5p (MIMAT0006789), hsa-miR-148a-5p (MIMAT0004549), hsa-miR-148b-5p (MIMAT0004699), hsa-miR-150-3p (MIMAT0004610), hsa-miR-150-5p (MIMAT0000451), hsa-miR-151a-5p (MIMAT0004697), hsa-miR-152-3p (MIMAT0000438), hsa-miR-181a-5p (MIMAT0000256), hsa-miR-185-5p (MIMAT0000455), hsa-miR-190a-5p (MIMAT0000458), hsa-miR-190b-5p (MIMAT0004929), hsa-miR-192-5p (MIMAT0000222), hsa-miR-1976 (MIMAT0009451), hsa-miR-200a-3p (MIMAT0000682), hsa-miR-215-5p (MIMAT0000272), hsa-miR-224-5p (MIMAT0000281), hsa-miR-24-2-5p (MIMAT0004497), hsa-miR-30a-3p (MIMAT0000088), hsa-miR-30a-5p (MIMAT0000087), hsa-miR-30d-3p (MIMAT0004551), hsa-miR-3124-5p (MIMAT0014986), hsa-miR-3138 (MIMAT0015006), hsa-miR-323b-3p (MIMAT0015050), hsa-miR-328-3p (MIMAT0000752), hsa-miR-335-5p (MIMAT0000765), hsa-miR-337-5p (MIMAT0004695), hsa-miR-342-5p (MIMAT0004694), hsa-miR-362-5p (MIMAT0000705), hsa-miR-3661 (MIMAT0018082), hsa-miR-370-3p (MIMAT0000722), hsa-miR-374b-3p (MIMAT0004956), hsa-miR-375-3p (MIMAT0000728), hsa-miR-376c-3p (MIMAT0000720), hsa-miR-377-5p (MIMAT0004689), hsa-miR-424-3p (MIMAT0004749), hsa-miR-431-5p (MIMAT0001625), hsa-miR-432-5p (MIMAT0002814), hsa-miR-4745-5p (MIMAT0019878), hsa-miR-4775 (MIMAT0019931), hsa-miR-487b-3p (MIMAT0003180), hsa-miR-5009-5p (MIMAT0021041), hsa-miR-5010-5p (MIMAT0021043), hsa-miR-511-5p (MIMAT0002808), hsa-miR-539-3p (MIMAT0022705), hsa-miR-542-3p (MIMAT0003389), hsa-miR-5683 (MIMAT0022472), hsa-miR-577 (MIMAT0003242), hsa-miR-628-3p (MIMAT0003297), hsa-miR-6735-5p (MIMAT0027371), hsa-miR-6787-3p (MIMAT0027475), hsa-miR-6843-3p (MIMAT0027588), hsa-miR-6847-5p (MIMAT0027594), hsa-miR-6894-5p (MIMAT0027688), hsa-miR-766-3p (MIMAT0003888), hsa-miR-7705 (MIMAT0030020), hsa-miR-7976 (MIMAT0031179), hsa-miR-873-5p (MIMAT0004953) and hsa-miR-887-5p (MIMAT0026720).

**Models of circulating microRNAs as biomarkers of CD.** In order to assess the diagnostic capacity of the microRNAs that were found to be characteristic of CD, an analysis of the ROC (Receiver Operating Characteristic) curves was performed. As shown in tables 2 and 3, all of the microRNAs identified in this study and predictive of CD achieved very good predictive parameters (sensitivity, specificity and AUC). In order to devise a predictive model for the diagnosis of celiac disease based on the use of such microRNAs, statistically significant (FDR<0.1) microRNAs having AUC values greater than 0.7 were considered. These microRNAs were entered into mathematical linear regression models for the calculation of the ROC curves (Figure 3). In particular, in a first model the microRNAs hsa-miR-215-5p (MIMAT0000272), hsa-miR-141-3p (MIMAT0000432), hsa-miR-375-3p (MIMAT0000728), hsa-miR-192-5p (MIMAT0000222), hsa-miR-24-2-5p (MIMAT0004497), hsa-let-7c-5p (MIMAT0000064) and hsa-miR-30a-3p (MIMAT0000088) were used for the diagnosis of celiac disease by means of a sequencing technique, whereas hsa-miR-215-5p (MIMAT0000272), hsa-miR-192-5p (MIMAT0000222), hsa-miR-99a-5p (MIMAT0000097), hsa-miR-125b-5p (MIMAT0000423), hsa-miR-194-5p (MIMAT0000460), hsa-miR-142-3p (MIMAT0000434) and hsa-miR-885-5p (MIMAT0004947) were used for the diagnosis of celiac disease by means of the quantitative real-time PCR (qPCR) technique. Using a sequencing technique, the mathematical model (Figure 3A) is able to diagnose celiac disease with a specificity of 88.5% and a sensitivity of 95.5% (AUC=0.955), whereas using the qPCR technique the mathematical model (Figure 3B) is able to diagnose celiac disease with a specificity of 94.7% and a sensitivity of 90.6% (AUC=0.972).

**Models of circulating microRNAs as biomarkers for monitoring adherence to the GFD by patients with celiac disease.**

In order to calculate a diagnostic model that can be used for monitoring adherence to the gluten-free diet by patients with celiac disease, the microRNAs hsa-miR-215-5p (MIMAT0000272), hsa-miR-141-3p (MIMAT0000432), hsa-miR-30a-3p (MIMAT0000088), hsa-miR-24-2-5p (MIMAT0004497), hsa-miR-328-3p (MIMAT0000752), hsa-miR-375-3p (MIMAT0000728), hsa-miR-6847-5p (MIMAT0027594), hsa-miR-192-5p (MIMAT0000222) and hsa-miR-424-3p (MIMAT0004749) were considered for the model generated from sequencing data, whereas the microRNAs hsa-miR-215-5p (MIMAT0000272), hsa-miR-192-5p (MIMAT0000222), hsa-miR-125b-5p (MIMAT0000423), hsa-miR-150-5p (MIMAT0000451), hsa-miR-142-5p (MIMAT0000433), hsa-miR-223-3p (MIMAT0000280) and hsa-miR-338-3p (MIMAT0000763) were considered for the model generated from qPCR data. Using a sequencing technique, the mathematical model (Figure 4A) is able to diagnose adherence to the GFD with a specificity of 90.9% and a sensitivity of 95.7% (AUC=0.978), whereas using the qPCR technique the mathematical model (Figure 4B) is able to diagnose adherence to the GFD with a specificity of 95.7% and a sensitivity of 75.0% (AUC=0.916).

**Models of circulating microRNAs as biomarkers for the simultaneous monitoring of celiac disease and adherence to a gluten-free diet.**

In order to calculate a diagnostic model that can be used for the diagnosis of celiac disease and for simultaneously monitoring adherence to the gluten-free diet by patients with celiac disease, the microRNAs hsa-miR-141-3p (MIMAT0000432), hsa-miR-192-5p (MIMAT0000222), hsa-miR-215-5p (MIMAT0000272), hsa-miR-24-2-5p (MIMAT0004497), hsa-miR-30a-3p (MIMAT0000088) and
hsa-miR-375-3p (MIMAT0000728) were considered by means of a sequencing technique (**Figure 5**), whereas the microRNAs hsa-miR-125b-5p (MIMAT0000423), hsa-miR-192-5p (MIMAT0000222) and
hsa-miR-215-5p (MIMAT0000272) were considered for the model generated from qPCR data (**Figure 6**).

Using a sequencing technique, the mathematical model (**Figure 5**) is able to diagnose celiac disease with a specificity of 88.5% and a sensitivity of 95.5% (AUC=0.953) and adherence to the GFD with a specificity of 90.9% and a sensitivity of 87.0% (AUC=0.931), whereas using the qPCR technique the mathematical model (**Figure 6**) is able to diagnose celiac disease with a specificity of 94.7% and a sensitivity of 68.8% (AUC=0.867) and adherence to the GFD with a specificity of 96.9% and a sensitivity of 73.9% (AUC=0.909). **Figures 7A, 7B** and **7C** show dot plots illustrating the differences in expression of these three miRNAs in the three groups of patients (CTRL, CD and GFD).

Therefore, following the workflow illustrated in Figure 1, it was possible to identify combinations of microRNAs that are specifically dysregulated in subjects with active celiac disease, and therefore usable as molecular markers for the diagnosis of celiac disease, and equally dysregulated compared to patients with celiac disease on a gluten-free diet and, therefore, usable as molecular markers for monitoring the adherence of subjects with celiac disease to the GFD.

**The model comprising the circulating microRNAs hsa-miR-215-5p (MIMAT0000272) and hsa-miR-192-5p (MIMAT0000222) for the diagnosis of CD and for the simultaneous monitoring of adherence to a GFD with both the sequencing and qPCR techniques.**

From the data obtained it was observed that the microRNAs hsa-miR-215-5p (MIMAT0000272) and hsa-miR-192-5p (MIMAT0000222) can be detected in patients with celiac disease both with sequencing techniques and with qPCR techniques and that these microRNAs are over-expressed in CD compared both with the controls and with the patients on a GFD.

Using a sequencing technique, the microRNA hsa-miR-215-5p (MIMAT0000272) is able to diagnose celiac disease with a specificity of 96.2% and a sensitivity of 72.7% (AUC=0.867), whereas using the qPCR technique it is able to diagnose celiac disease with a specificity of 89.5% and a sensitivity of 71.9% (AUC=0.842). Using a sequencing technique, the microRNA hsa-miR-192-5p (MIMAT0000222) is able to diagnose celiac disease with a specificity of 96.2% and a sensitivity of 54.5% (AUC=0.766), whereas using the qPCR technique it is able to diagnose celiac disease with a specificity of 94.7% and a sensitivity of 62.5% (AUC=0.854).

As for monitoring the adherence of patients with celiac disease to the GFD, using a sequencing technique the microRNA hsa-miR-215-5p (MIMAT0000272) is able to diagnose celiac disease with a specificity of 72.7% and a sensitivity of 87.0% (AUC=0.832), whereas using the qPCR technique it is able to diagnose celiac disease with a specificity of 71.9% and a sensitivity of 87.0% (AUC=0.783). Using a sequencing technique, the microRNA hsa-miR-192-5p (MIMAT0000222) is able to diagnose celiac disease with a specificity of 54.5% and a sensitivity of 91.3% (AUC=0.709), whereas using the qPCR technique it is able to diagnose celiac disease with a specificity of 84.4% and a sensitivity of 78.3% (AUC=0.879).

Finally, with the aim of devising a complete diagnostic model that is more sensitive and effective compared to the individual microRNAs, that simultaneously includes the two miRNAs and that potentially has a high predictive power both for celiac disease and for monitoring adherence to a gluten-free diet, the ROC curve was generated using a linear model of the two microRNAs. Figure 8 shows the ROC curves of the models of the two microRNAs (hsa-miR-215-5p and hsa-miR-192-5p) calculated for the diagnosis of celiac disease with sequencing techniques (Figure 8A), which reach a specificity of 96.2% and sensitivity of 68.2% (AUC=0.971), and qPCR (Figure 8C), which reaches a specificity of 94.7% and sensitivity of 65.6% (AUC=0.867), and for monitoring the GFD in CD patients by means of a sequencing technique (Figure 8B), which reaches a specificity of 68.2% and sensitivity of 91.3% (AUC=0.838), and qPCR (Figure 8D), which reaches a specificity of 87.5% and sensitivity of 78.3% (AUC=0.882). Figures 7B and 7C show dot plots illustrating the differences in the expression of these two miRNAs in the three groups of patients (CTRL, CD and GFD).

These results suggest that the combination of the two microRNAs identified in CD can also be successfully used to discriminate, with good reliability, the patients with CD subjected to a strict gluten-free diet irrespective of the technique used (sequencing or qPCR). This result is relevant, since at present no reliable analytic tests are available for monitoring adherence to a GFD.

**The model comprising the circulating microRNAs hsa-miR-125b-5p (MIMAT0000423), hsa-miR-192-5p (MIMAT0000222) and hsa-miR-215-5p (MIMAT0000272) for the diagnosis of CD in "borderline" patients using the qPCR technique.**

As a further investigation, it was assessed whether the model comprising the three microRNAs hsa-miR-125b-5p (MIMAT0000423), hsa-miR-192-5p (MIMAT0000222) and hsa-miR-215-5p (MIMAT0000272) were useful for predicting CD also in doubtful cases (i.e. patients with moderately high levels of IgA anti-tTG). For this purpose, only the patients (n = 13) with a value of IgA anti-tTG less than ten times the upper normal limit (according to ESPHGAN guidelines) were drawn from the cohort of CD patients recruited, and the expression of the three circulating microRNAs was examined. These patients were indicated as "borderline" patients (CD-bord).

As shown in Figure 9, the preferred microRNAs (i.e. miR-125b-5p, miR-192-5p and miR-215-5p) are also able to discriminate these groups of patients rather well. In particular, the microRNA hsa-miR-125b-5p (MIMAT0000423) reached a sensitivity of 76.9% and a specificity of 73.7% (AUC = 0.794), the microRNA hsa-miR-192-5p (MIMAT0000222) reached a sensitivity of 92.3% and a specificity of 68.4% (AUC = 0.85), whereas the microRNA hsa-miR-215-5p (MIMAT0000272) reached a sensitivity of 76.9% and a specificity of 89.5% (AUC = 0.866) (Figure 9A). The dot plot in Figure 9B illustrates the differences in the expression of these three miRNAs in the four groups of patients (CTRL, CD-bord, CD and GFD). Finally, with the aim of devising a complete diagnostic model that simultaneously includes all three of the miRNAs and potentially has the maximum predictive power, an analysis of the ROC curve was done using the function of the linear model. The results are shown in Figure 9C. The ROC curve of the model obtained using the three microRNAs reached a specificity of 84.2% and a sensitivity of 84.6% (AUC=0.883) for the diagnosis of "borderline" CD patients compared to the controls and monitored using the qPCR technique.

**The model comprising the circulating microRNAs hsa-miR-215-5p (MIMAT0000272) and hsa-miR-192-5p (MIMAT0000222) for the diagnosis of CD in "borderline" patients irrespective of the technique used.**

With the aim of devising a diagnostic model that is sensitive and effective compared to the individual microRNAs and has a high predictive power both for celiac disease and for monitoring adherence to a gluten-free diet also in "borderline" patients irrespective of the technique used (sequencing or qPCR), the predictive capacity of the linear model comprising the two circulating microRNAs hsa-miR-192-5p (MIMAT0000222) and hsa-miR-215-5p (MIMAT0000272) was assessed using an analysis of the ROC curve.

The results are shown in Figure 9D. The ROC curve of the model obtained using the two microRNAs reached a specificity of 94.7% and a sensitivity of 69.2% (AUC=0.887) for the diagnosis of "borderline" CD patients compared to the controls.

### References

[1] Gujral N, Freeman HJ, Thomson AB. Celiac disease: Prevalence, diagnosis, pathogenesis and treatment. World Journal of Gastroenterology : WJG. 2012; 18(42):6036-6059.
[2] Schuppan D, Junker Y, Barisani D. Celiac disease: from pathogenesis to novel therapies. Gastroenterology. 2009 Dec; 137(6):1912-33. PMID: 19766641
[3] Guandalini S, Assiri A. Celiac disease: a review. JAMA Pediatr. 2014 Mar;168(3):272-8. PMID: 24395055
[4] Amarri S, Alvisi P, De Giorgio R, Gelli CD, Cicola R, Tovoli F, Sassatelli R, Caio G, Volta U. Antibodies to deamidated gliadin peptides: an accurate predictor of coeliac disease in infancy. J Clin Immunol. 2013 Jul; 33(5):1027-30. PMID: 23558824
[5] Kagnoff MF. Celiac disease: pathogenesis of a model immunogenetic disease. J Clin Invest. 2007 Jan; 117(1):41-9. PMID: 17200705
[6] Husby S, Koletzko S, Korponay-Szabó IR, Mearin ML, Phillips A, Shamir R, Troncone R, Giersiepen K, Branski D, Catassi C, Lelgeman M, Mäki M, Ribes-Koninckx C, Ventura A, Zimmer KP; ESPGHAN Working Group on Coeliac Disease Diagnosis; ESPGHAN Gastroenterology Committee; European Society for Pediatric Gastroenterology, Hepatology, and Nutrition.; European Society for Pediatric Gastroenterology, Hepatology, and Nutrition guidelines for the diagnosis of coeliac disease J Pediatr Gastroenterol Nutr. 2012; 54(1):136-60. PMID: 22197856
[7] Li M, Marin-Muller C, Bharadwaj U, Chow K-H, Yao Q, Chen C. MicroRNAs: Control and Loss of Control in Human Physiology and Disease. World journal of surgery. 2009; 33(4):667-684. PCD2933043
[8] Mitchell PS, Parkin RK, Kroh EM, Fritz BR, Wyman SK, Pogosova-Agadjanyan EL, Peterson A, Noteboom J, O'Briant KC, Allen A, Lin DW, Urban N, Drescher CW, Knudsen BS, Stirewalt DL, Gentleman R, Vessella RL, Nelson PS, Martin DB, Tewari M. Circulating microRNAs as stable blood-based markers for cancer detection. Proc Natl Acad Sci U S A. 2008 Jul 29; 105(30):10513-8. PCDID: PCD2492472
[9] Chen X, Liang H, Zhang J, Zen K, Zhang CY. Secreted microRNAs: a new form of intercellular communication. Trends Cell Biol. 2012 Mar; 22(3):125-32. PMID: 22260888
[10] J Green PH, Lebwohl B, Greywoode R. Celiac disease. Allergy Clin Immunol. 2015 May;135(5):1099-106; PMID: 25956012
[11] Sugai E, Nachman F, Váquez H, González A, Andrenacci P, Czech A, Niveloni S, Mazure R, Smecuol E, Cabanne A, Mauriño E, Bai JC. Dynamics of celiac disease-specific serology after initiation of a gluten-free diet and use in the assessment of compliance with treatment. Dig Liver Dis. 2010 May;42(5):352-8. PMID: 19679520
[12] Fasano A, Catassi C. Current approaches to diagnosis and treatment of celiac disease: an evolving spectrum. Gastroenterology. 2001;120:636-651. PMID: 11179241
[13] Burgos KL, Javaherian A, Bomprezzi R, et al. Identification of extracellular miRNA in human cerebrospinal fluid by next-generation sequencing. RNA. 2013;19(5):712-722. PCDID: PCD3677285
[14] Chen C, Ridzon DA, Broomer AJ, Zhou Z, Lee DH, Nguyen JT, Barbisin M, Xu NL, Mahuvakar VR, Andersen MR, Lao KQ, Livak KJ, Guegler KJ. Real-time quantification of microRNAs by stem-loop RT-PCR. Nucleic Acids Res. 2005 Nov 27;33(20):e179. PMID: 16314309[12] Green PH, Cellier C. Celiac disease. N Engl J Med. 2007 Oct 25;357(17):1731-43. PMID: 17960014
[15] Loddo I, Romano C. Inflammatory Bowel Disease: Genetics, Epigenetics, and Pathogenesis. Front Immunol. 2015; 6: 551. PMID: 26579126
[16] Pascual V, Dieli-Crimi R, López-Palacios N, Bodas A, Medrano LM, Núñez C. Inflammatory bowel disease and celiac disease: overlaps and differences. World J Gastroenterol. 2014 May 7;20(17):4846-56. PMID: 24803796
[17] Zahm AM, Thayu M, Hand NJ, Horner A, Leonard MB, Friedman JR. Circulating microRNA is a biomarker of pediatric Crohn disease. Pediatr Gastroenterol Nutr. 2011 Jul;53(1):26-33. PMID: 21546856
[18] Simon Anders, Paul Theodor Pyl, and Wolfgang Huber. HTSeq-a Python framework to work with high-throughput sequencing data. Bioinformatics. 2015 Jan 15; 31(2): 166-169. PCDID: PCD4287950
[19] Love MI, Huber W, Anders S. Moderated estimation of fold change and dispersion for RNA-seq data with DESeq2. Genome Biol. 2014;15(12):550.
[20] Mestdagh P, Van Vlierberghe P, De Weer A, Muth D, Westermann F, Speleman F,Vandesompele J. A novel and universal method for microRNA RT-qPCR data normalization. Genome Biol 2009;10:R64.
[21] Swets JA. Measuring the accuracy of diagnostic systems. Science. 1988 Jun 3;240(4857):1285-93.
[22] Buoli Comani et al. miRNA-regulated gene expression differs in celiac disease patients according to the age of presentation. Genes & nutrition; Studying the relationship between genetics and nutrition in the improvement of human health, Springer, DE (2015); 10(5): 1-12.
[23] Felli et al. Intestinal and circulating microRNAs in coeliac disease. Int. J Mol. Sci. 2017, 18 (9):1907.

## Claims

1. Method for the *in vitro* **diagnosis of celiac disease** and/or for the *in vitro* **monitoring of adherence to a gluten-free diet,** wherein
said method comprises measuring the expression, in a biological sample, of both of the microRNAs hsa-miR-192-5p (MIMAT0000222) and hsa-miR-215-5p (MIMAT0000272), wherein said microRNAs are over-expressed in celiac disease compared to their expression in healthy controls or are over-expressed in subjects with celiac disease who do not follow a gluten-free diet compared to their expression in subjects with celiac disease who follow the gluten-free diet, wherein the biological sample is a liquid biological sample consisting of serum or plasma.

2. Method according to claim 1, wherein the expression of said microRNAs is measured by means of a sequencing technique such as, for example, RNA next generation sequencing, or by means of a Real-Time PCR technique.

3. Method according to any one of claims 1-2, which further comprises measuring the expression of hsa-miR-125b-5p (MIMAT0000423) by Real-Time PCR, wherein said hsa-miR-125b-5p is over-expressed in celiac disease compared to its expression in healthy controls or is over-expressed in subjects with celiac disease who do not follow a gluten-free diet compared to its expression in subjects with celiac disease who follow the gluten-free diet.

4. Method according to claim 3, wherein said method further comprises measuring the expression of one or more of the following microRNAs by Real-Time PCR: hsa-miR-150-5p (MIMAT0000451), hsa-miR-142-5p (MIMAT0000433), hsa-miR-223-3p (MIMAT0000280) and/or hsa-miR-338-3p (MIMAT0000763), wherein
hsa-miR-150-5p is over-expressed in celiac disease compared to its expression in healthy controls and is over-expressed in subjects with celiac disease who do not follow a gluten-free diet compared to its expression in subjects with celiac disease who follow the gluten-free diet; whereas
hsa-miR-142-5p, hsa-miR-223-3p and hsa-miR-338-3p are down-expressed in subjects with celiac disease who do not follow a gluten-free diet compared to their expression in subjects with celiac disease who follow the gluten-free diet.

5. Method according to claim 4, wherein said method comprises measuring the expression of all of the following microRNAs by Real-Time PCR: hsa-miR-192-5p, hsa-miR-215-5p, hsa-miR-125b-5p, hsa-miR-150-5p, hsa-miR-142-5p, hsa-miR-223-3p and hsa-miR-338-3p for the *in vitro* monitoring of adherence to a gluten-free diet.

6. Method according to claim 3, wherein said method further comprises measuring the expression of one or more of the following microRNAs by Real-Time PCR: hsa-miR-99a-5p (MIMAT0000097), hsa-miR-194-5p (MIMAT0000460), hsa-miR-142-3p (MIMAT0000434), hsa-miR-885-5p (MIMAT0004947) and/or hsa-miR-885-5p (MIMAT0004947), wherein
hsa-miR-99a-5p, hsa-miR-194-5p and hsa-miR-885-5p are over-expressed in celiac disease compared to their expression in healthy controls and are over-expressed in subjects with celiac disease who do not follow a gluten-free diet compared to their expression in subjects with celiac disease who follow the gluten-free diet; whereas
hsa-miR-142-3p is down-expressed in celiac disease compared to its expression in healthy controls.

7. Method according to claim 4, wherein said method comprises measuring the expression of all of the following microRNAs by Real-Time PCR: hsa-miR-192-5p, hsa-miR-215-5p, hsa-miR-125b-5p, hsa-miR-99a-5p, hsa-miR-194-5p, hsa-miR-885-5p and hsa-miR-142-3p.

8. Method according to any one of claims 1-2, which further comprises measuring the expression of one or more of the following microRNAs by means of a sequencing technique such as, for example, RNA next generation sequencing: hsa-miR-141-3p (MIMAT0000432), hsa-miR-24-2-5p (MIMAT0004497), hsa-miR-30a-3p (MIMAT0000088) and/or hsa-miR-375-3p (MIMAT0000728), wherein
hsa-miR-141-3p, hsa-miR-30a-3p and hsa-miR-375-3p are over-expressed in celiac disease compared to their expression in healthy controls and are over-expressed in subjects with celiac disease who do not follow a gluten-free diet compared to their expression in subjects with celiac disease who follow the gluten-free diet;
whereas hsa-miR-24-2-5p is down-expressed in celiac disease compared to its expression in healthy controls and is down-expressed in subjects with celiac disease who do not follow a gluten-free diet compared to its expression in subjects with celiac disease who follow the gluten-free diet.

9. Method according to claim 8, wherein said method comprises measuring the expression of all of the following microRNAs by means of a sequencing technique:
hsa-miR-192-5p, hsa-miR-215-5p, hsa-miR-141-3p, hsa-miR-30a-3p, hsa-miR-375-3p and hsa-miR-24-2-5p.

10. Method according to any one of claims 8-9, said method further comprising measuring the expression of hsa-let-7c-5p (MIMAT0000064), wherein said microRNA is over-expressed in celiac disease compared to its expression in healthy controls.

11. Method according to any one of claims 8-9, said method further comprising measuring the expression of one or more of the following microRNAs by means of a sequencing technique such as, for example, RNA next generation sequencing: hsa-miR-328-3p (MIMAT0000752), hsa-miR-6847-5p (MIMAT0027594) and/or hsa-miR-424-3p (MIMAT0004749), wherein
hsa-miR-6847-5p is over-expressed in subjects with celiac disease who do not follow a gluten-free diet compared to its expression in subjects with celiac disease who follow the gluten-free diet;
whereas hsa-miR-328-3p and hsa-miR-424-3p are down-expressed in subjects with celiac disease who do not follow a gluten-free diet compared to their expression in subjects with celiac disease who follow the gluten-free diet.

12. Method according to claim 11, wherein said method comprises measuring the expression of all of the following microRNAs by means of a sequencing technique:
hsa-miR-192-5p, hsa-miR-215-5p, hsa-miR-141-3p, hsa-miR-30a-3p, hsa-miR-375-3p, hsa-miR-24-2-5p, hsa-miR-6847-5p, hsa-miR-328-3p and hsa-miR-424-3p.

13. Method according to any one of the preceding claims, wherein
for the *in vitro* **diagnosis of celiac disease,** said method further comprises measuring the expression, in a biological sample, of one or more of the following microRNAs: hsa-let-7b-3p (MIMAT0004482), hsa-let-7f-1-3p (MIMAT0004486), hsa-let-7f-5p (MIMAT0000067), hsa-miR-100-5p (MIMAT0000098), hsa-miR-11401 (MIMAT0044658), hsa-miR-1246 (MIMAT0005898), hsa-miR-125b-2-3p (MIMAT0004603), hsa-miR-127-5p (MIMAT0004604), hsa-miR-144-5p (MIMAT0004600), hsa-miR-145-5p (MIMAT0000437), hsa-miR-148b-5p (MIMAT0004699), hsa-miR-152-3p (MIMAT0000438), hsa-miR-181a-5p (MIMAT0000256), hsa-miR-1976 (MIMAT0009451), hsa-miR-200a-3p (MIMAT0000682), hsa-miR-205-5p (MIMAT0000266), hsa-miR-224-5p (MIMAT0000281), hsa-miR-28-3p (MIMAT0004502), hsa-miR-323b-3p (MIMAT0015050), hsa-miR-326 (MIMAT0000756), hsa-miR-335-3p (MIMAT0004703), hsa-miR-335-5p (MIMAT0000765), hsa-miR-339-5p (MIMAT0000764), hsa-miR-363-3p (MIMAT0000707), hsa-miR-370-3p (MIMAT0000722), hsa-miR-374b-3p (MIMAT0004956), hsa-miR-376c-3p (MIMAT0000720), hsa-miR-423-5p (MIMAT0004748), hsa-miR-431-5p (MIMAT0001625), hsa-miR-4745-5p (MIMAT0019878), hsa-miR-4775 (MIMAT0019931), hsa-miR-486-5p (MIMAT0002177), hsa-miR-5009-5p (MIMAT0021041), hsa-miR-511-5p (MIMAT0002808), hsa-miR-532-5p (MIMAT0002888), hsa-miR-5683 (MIMAT0022472), hsa-miR-577 (MIMAT0003242), hsa-miR-584-5p (MIMAT0003249), hsa-miR-628-3p (MIMAT0003297), hsa-miR-6787-3p (MIMAT0027475), hsa-miR-6843-3p (MIMAT0027588), hsa-miR-6894-5p (MIMAT0027688), hsa-miR-766-3p (MIMAT0003888), hsa-miR-7976 (MIMAT0031179), hsa-miR-874-3p (MIMAT0004911), hsa-miR92b-3p (MIMAT0003218),
wherein the following microRNAs are over-expressed in celiac disease compared to their expression in healthy controls:
hsa-let-7b-3p (MIMAT0004482), hsa-let-7f-1-3p (MIMAT0004486), hsa-miR-100-5p (MIMAT0000098), hsa-miR-1246 (MIMAT0005898), hsa-miR-125b-2-3p (MIMAT0004603), hsa-miR-181a-5p (MIMAT0000256), hsa-miR-200a-3p (MIMAT0000682), hsa-miR-205-5p (MIMAT0000266), hsa-miR-224-5p (MIMAT0000281), hsa-miR-363-3p (MIMAT0000707), hsa-miR-374b-3p (MIMAT0004956), hsa-miR-423-5p (MIMAT0004748), hsa-miR-4745-5p (MIMAT0019878), hsa-miR-486-5p (MIMAT0002177), hsa-miR-5009-5p (MIMAT0021041), hsa-miR-511-5p (MIMAT0002808), hsa-miR-532-5p (MIMAT0002888), hsa-miR-5683 (MIMAT0022472), hsa-miR-577 (MIMAT0003242), hsa-miR-628-3p (MIMAT0003297), hsa-miR-6787-3p (MIMAT0027475), hsa-miR-6894-5p (MIMAT0027688), hsa-miR-7976 (MIMAT0031179), hsa-miR-874-3p (MIMAT0004911), hsa-miR-92b-3p (MIMAT0003218);
whereas the following microRNAs are down-expressed in celiac disease compared to their expression in healthy controls:
hsa-let-7f-5p (MIMAT0000067), hsa-miR-11401 (MIMAT0044658), hsa-miR-127-5p (MIMAT0004604), hsa-miR-144-5p (MIMAT0004600), hsa-miR-145-5p (MIMAT0000437), hsa-miR-148b-5p (MIMAT0004699), hsa-miR-152-3p (MIMAT0000438), hsa-miR-1976 (MIMAT0009451), hsa-miR-28-3p (MIMAT0004502), hsa-miR-323b-3p (MIMAT0015050), hsa-miR-326 (MIMAT0000756), hsa-miR-335-3p (MIMAT0004703), hsa-miR-335-5p (MIMAT0000765), hsa-miR-339-5p (MIMAT0000764), hsa-miR-370-3p (MIMAT0000722), hsa-miR-376c-3p (MIMAT0000720), hsa-miR-431-5p (MIMAT0001625), hsa-miR-4775 (MIMAT0019931), hsa-miR-584-5p (MIMAT0003249), hsa-miR-6843-3p (MIMAT0027588) and hsa-miR-766-3p (MIMAT0003888);
and wherein
for the *in vitro* **monitoring of adherence to a gluten-free diet,** said method further comprises measuring the expression, in a biological sample, of at least one or more of the following microRNAs:
hsa-let-7b-5p (MIMAT0000063), hsa-miR-122-5p (MIMAT0000421), hsa-miR-125a-3p (MIMAT0004602), hsa-miR-125b-2-3p (MIMAT0004603), hsa-miR-134-5p (MIMAT0000447), hsa-miR-139-3p (MIMAT0004552), hsa-miR-140-5p (MIMAT0000431), hsa-miR-1468-5p (MIMAT0006789), hsa-miR-148a-5p (MIMAT0004549), hsa-miR-150-3p (MIMAT0004610), hsa-miR-151a-5p (MIMAT0004697), hsa-miR-185-5p (MIMAT0000455), hsa-miR-190a-5p (MIMAT0000458), hsa-miR-190b-5p (MIMAT0004929), hsa-miR-1976 (MIMAT0009451), hsa-miR-199a-5p (MIMAT0000231), hsa-miR-200a-3p (MIMAT0000682), hsa-miR-20a-5p (MIMAT0000075), hsa-miR-224-5p ( MIMAT0000281), hsa-miR-301a-3p (MIMAT0000688), hsa-miR-30a-5p (MIMAT0000087), hsa-miR-30d-3p (MIMAT0004551), hsa-miR-3124-5p (MIMAT0014986), hsa-miR-3138 (MIMAT0015006), hsa-miR-326 (MIMAT0000756), hsa-miR-337-5p (MIMAT0004695), hsa-miR-339-5p (MIMAT0000764), hsa-miR-342-5p (MIMAT0004694), hsa-miR-362-5p (MIMAT0000705), hsa-miR-363-3p (MIMAT0000707), hsa-miR-3661 (MIMAT0018082), hsa-miR-370-3p (MIMAT0000722), hsa-miR-377-5p (MIMAT0004689), hsa-miR-423-3p (MIMAT0001340), hsa-miR-423-5p (MIMAT0004748), hsa-miR-432-5p (MIMAT0002814), hsa-miR-4745-5p (MIMAT0019878), hsa-miR-486-5p (MIMAT0002177), hsa-miR-487b-3p (MIMAT0003180), hsa-miR-5010-5p (MIMAT0021043), hsa-miR-539-3p (MIMAT0022705), hsa-miR-542-3p (MIMAT0003389), hsa-miR-5683 (MIMAT0022472), hsa-miR-6735-5p (MIMAT0027371), hsa-miR-7705 (MIMAT0030020), hsa-miR-873-5p (MIMAT0004953), hsa-miR-887-5p (MIMAT0026720);
wherein the following microRNAs are over-expressed in subjects with celiac disease who do not follow a gluten-free diet compared to their expression in subjects with celiac disease who follow the gluten-free diet:
hsa-let-7b-5p (MIMAT0000063), hsa-miR-122-5p (MIMAT0000421), hsa-miR-125b-2-3p (MIMAT0004603), hsa-miR-1468-5p (MIMAT0006789), hsa-miR-150-3p (MIMAT0004610), hsa-miR-200a-3p (MIMAT0000682), hsa-miR-224-5p (MIMAT0000281), , hsa-miR-30a-5p (MIMAT0000087), hsa-miR-3138 (MIMAT0015006), hsa-miR-342-5p (MIMAT0004694), hsa-miR-363-3p (MIMAT0000707), hsa-miR-3661 (MIMAT0018082), hsa-miR-423-5p (MIMAT0004748), hsa-miR-4745-5p (MIMAT0019878), hsa-miR-486-5p (MIMAT0002177), hsa-miR-5010-5p (MIMAT0021043), hsa-miR-5683 (MIMAT0022472), hsa-miR-6735-5p (MIMAT0027371);
whereas the following microRNAs are down-expressed in subjects with celiac disease who do not follow a gluten-free diet compared to their expression in subjects with celiac disease who follow the gluten-free diet:
hsa-miR-125a-3p (MIMAT0004602), hsa-miR-134-5p (MIMAT0000447), hsa-miR-139-3p (MIMAT0004552), hsa-miR-140-5p (MIMAT0000431), hsa-miR-148a-5p (MIMAT0004549), hsa-miR-151a-5p (MIMAT0004697), hsa-miR-185-5p (MIMAT0000455), hsa-miR-190a-5p (MIMAT0000458), hsa-miR-190b-5p (MIMAT0004929),
hsa-miR-1976 (MIMAT0009451), hsa-miR-199a-5p (MIMAT0000231), hsa-miR-20a-5p (MIMAT0000075), hsa-miR-301a-3p (MIMAT0000688), hsa-miR-30d-3p (MIMAT0004551), hsa-miR-3124-5p (MIMAT0014986), hsa-miR-326 (MIMAT0000756), hsa-miR-337-5p (MIMAT0004695), hsa-miR-339-5p (MIMAT0000764), hsa-miR-362-5p (MIMAT0000705), hsa-miR-370-3p (MIMAT0000722), hsa-miR-377-5p (MIMAT0004689), hsa-miR-423-3p (MIMAT0001340), hsa-miR-432-5p (MIMAT0002814), hsa-miR-487b-3p (MIMAT0003180), hsa-miR-539-3p (MIMAT0022705), hsa-miR-542-3p (MIMAT0003389), hsa-miR-7705 (MIMAT0030020), hsa-miR-873-5p (MIMAT0004953) and hsa-miR-887-5p (MIMAT0026720).

14. Method according to claim 13, wherein for the *in vitro* **diagnosis of celiac disease** said one or more microRNAs are selected from:
hsa-let-7b-3p (MIMAT0004482), hsa-let-7f-5p (MIMAT0000067), hsa-miR-200a-3p (MIMAT0000682), hsa-miR-28-3p (MIMAT0004502), hsa-miR-339-5p (MIMAT0000764), hsa-miR-4775 (MIMAT0019931), hsa-miR-577 (MIMAT0003242), hsa-miR-766-3p (MIMAT0003888), hsa-miR-874-3p (MIMAT0004911);
alternatively said one or more microRNAs are selected from:
hsa-miR-100-5p (MIMAT0000098), hsa-miR-125b-2-3p (MIMAT0004603), hsa-miR-323b-3p (MIMAT0015050), hsa-miR-335-5p (MIMAT0000765), hsa-miR-370-3p (MIMAT0000722), hsa-miR-423-5p (MIMAT0004748), hsa-miR-92b-3p (MIMAT0003218);
and wherein
for the *in vitro* **monitoring of adherence to a gluten-free diet,** said one or more microRNAs are selected from:
hsa-miR-150-3p (MIMAT0004610), hsa-miR-190a-5p (MIMAT0000458), hsa-miR-200a-3p (MIMAT0000682), hsa-miR-30a-3p (MIMAT0000088), hsa-miR-337-5p (MIMAT0004695), hsa-miR-377-5p (MIMAT0004689), hsa-miR-6735-5p (MIMAT0027371), and hsa-miR-887-5p (MIMAT0026720);
alternatively said one or more microRNAs are selected from:
hsa-miR-125b-2-3p (MIMAT0004603), hsa-miR-134-5p (MIMAT0000447), hsa-miR-185-5p (MIMAT0000455), hsa-miR-3661 (MIMAT0018082), hsa-miR-423-5p (MIMAT0004748).

## Patentansprüche

1. Verfahren zur *in vitro* diagnose von Zöliakie und/oder zur *in vitro* Überwachung der Einhaltung einer glutenfreien Diät, wobei
wobei das Verfahren die Messung der Expression der beiden microRNAs hsa-miR-192-5p (MIMAT0000222) und hsa-miR-215-5p (MIMAT0000272) in einer biologischen Probe umfasst, wobei die microRNAs bei Zöliakie im Vergleich zu ihrer Expression bei gesunden Kontrollen überexprimiert sind oder bei Personen mit Zöliakie, die keine glutenfreie Diät einhalten, im Vergleich zu ihrer Expression bei Personen mit Zöliakie, die die glutenfreie Diät einhalten, überexprimiert sind, wobei die biologische Probe eine flüssige biologische Probe ist, die aus Serum oder Plasma besteht.

2. Verfahren nach Anspruch 1, wobei die Expression der microRNAs mittels einer Sequenzierungstechnik, wie z. B. RNA Next Generation Sequencing, oder mittels einer Real-Time PCR-Technik gemessen wird.

3. Verfahren nach einem der Ansprüche 1-2, das ferner die Messung der Expression von hsa-miR-125b-5p (MIMAT0000423) durch Real-Time PCR umfasst, wobei das hsa-miR-125b-5p bei Zöliakie im Vergleich zu seiner Expression bei gesunden Kontrollen überexprimiert ist oder bei Personen mit Zöliakie, die keine glutenfreie Diät einhalten, im Vergleich zu seiner Expression bei Personen mit Zöliakie, die die glutenfreie Diät einhalten, überexprimiert ist.

4. Verfahren nach Anspruch 3, wobei das Verfahren ferner das Messen der Expression einer oder mehrerer der folgenden microRNAs durch Real-Time PCR umfasst:
hsa-miR-150-5p (MIMAT0000451), hsa-miR-142-5p (MIMAT0000433), hsa-miR-223-3p (MIMAT0000280) und/oder hsa-miR-338-3p (MIMAT0000763), wobei
hsa-miR-150-5p bei Zöliakie im Vergleich zu gesunden Kontrollpersonen überexprimiert ist und bei Personen mit Zöliakie, die sich nicht glutenfrei ernähren, im Vergleich zu Personen mit Zöliakie, die sich glutenfrei ernähren, überexprimiert ist; in Erwägung nachstehender Gründe
hsa-miR-142-5p, hsa-miR-223-3p und hsa-miR-338-3p werden bei Personen mit Zöliakie, die sich nicht glutenfrei ernähren, weniger stark exprimiert als bei Personen mit Zöliakie, die sich glutenfrei ernähren.

5. Verfahren nach Anspruch 4, wobei das Verfahren die Messung der Expression aller der folgenden microRNAs durch Real-Time PCR umfasst: hsa-miR-192-5p, hsa-miR-215-5p, hsa-miR-125b-5p, hsa-miR-150-5p, hsa-miR-142-5p, hsa-miR-223-3p und hsa-miR-338-3p für die *in vitro* Überwachung der Einhaltung einer glutenfreien Diät.

6. Verfahren nach Anspruch 3, wobei das Verfahren ferner das Messen der Expression einer oder mehrerer der folgenden microRNAs durch Real-Time PCR umfasst:
hsa-miR-99a-5p (MIMAT0000097), hsa-miR-194-5p (MIMAT0000460), hsa-miR-142-3p (MIMAT0000434), hsa-miR-885-5p (MIMAT0004947) und/oder hsa-miR-885-5p (MIMAT0004947), wobei
hsa-miR-99a-5p, hsa-miR-194-5p und hsa-miR-885-5p werden bei Zöliakie im Vergleich zu gesunden Kontrollpersonen übermäßig stark exprimiert und sind bei Zöliakiepatienten, die sich nicht glutenfrei ernähren, im Vergleich zu Zöliakiepatienten, die sich glutenfrei ernähren, übermäßig stark exprimiert, während
hsa-miR-142-3p wird bei Zöliakie weniger stark exprimiert als bei gesunden Kontrollpersonen.

7. Verfahren nach Anspruch 4, wobei das Verfahren die Messung der Expression aller der folgenden microRNAs durch Real-Time PCR umfasst: hsa-miR-192-5p, hsa-miR-215-5p, hsa-miR-125b-5p, hsa-miR-99a-5p, hsa-miR-194-5p, hsa-miR-885-5p und hsa-miR-142-3p.

8. Verfahren nach einem der Ansprüche 1-2, das ferner das Messen der Expression einer oder mehrerer der folgenden microRNAs mittels einer Sequenzierungstechnik, wie beispielsweise RNA Next Generation Sequencing, umfasst: hsa-miR-141-3p (MIMAT0000432), hsa-miR-24-2-5p (MIMAT0004497), hsa-miR-30a-3p (MIMAT0000088) und/oder hsa-miR-375-3p (MIMAT0000728), wobei
hsa-miR-141-3p, hsa-miR-30a-3p und hsa-miR-375-3p werden bei Zöliakie im Vergleich zu gesunden Kontrollpersonen übermäßig stark exprimiert und sind bei Zöliakiepatienten, die sich nicht glutenfrei ernähren, im Vergleich zu Zöliakiepatienten, die sich glutenfrei ernähren, übermäßig stark ausgeprägt,
in der Erwägung, dass die Expression von hsa-miR-24-2-5p bei Zöliakie im Vergleich zur Expression bei gesunden Kontrollpersonen und bei Zöliakiepatienten, die sich nicht glutenfrei ernähren, im Vergleich zur Expression bei Zöliakiepatienten, die sich glutenfrei ernähren, verringert ist.

9. Verfahren nach Anspruch 8, wobei das Verfahren die Messung der Expression aller der folgenden microRNAs mittels einer Sequenzierungstechnik umfasst:
hsa-miR-192-5p, hsa-miR-215-5p, hsa-miR-141-3p, hsa-miR-30a-3p, hsa-miR-375-3p und hsa-miR-24-2-5p.

10. Verfahren nach einem der Ansprüche 8-9, wobei das Verfahren ferner die Messung der Expression von hsa-let-7c-5p (MIMAT0000064) umfasst, wobei die microRNA bei Zöliakie im Vergleich zu ihrer Expression in gesunden Kontrollen überexprimiert ist.

11. Verfahren nach einem der Ansprüche 8-9, wobei das Verfahren ferner das Messen der Expression einer oder mehrerer der folgenden microRNAs mittels einer Sequenzierungstechnik, wie beispielsweise RNA Next Generation Sequencing, umfasst: hsa-miR-328-3p (MIMAT0000752), hsa-miR-6847-5p (MIMAT0027594) und/oder hsa-miR-424-3p (MIMAT0004749), wobei
hsa-miR-6847-5p ist bei Personen mit Zöliakie, die sich nicht glutenfrei ernähren, überexprimiert, verglichen mit seiner Expression bei Personen mit Zöliakie, die sich glutenfrei ernähren;
wohingegen hsa-miR-328-3p und hsa-miR-424-3p bei Personen mit Zöliakie, die sich nicht glutenfrei ernähren, im Vergleich zu ihrer Expression bei Personen mit Zöliakie, die sich glutenfrei ernähren, weniger stark ausgeprägt sind.

12. Verfahren nach Anspruch 11, wobei das Verfahren die Messung der Expression aller der folgenden microRNAs mittels einer Sequenzierungstechnik umfasst:
hsa-miR-192-5p, hsa-miR-215-5p, hsa-miR-141-3p, hsa-miR-30a-3p, hsa-miR-375-3p, hsa-miR-24-2-5p, hsa-miR-6847-5p, hsa-miR-328-3p und hsa-miR-424-3p.

13. Verfahren nach einem der vorhergehenden Ansprüche, wobei das Verfahren für die *in vitro* Diagnose von Zöliakie ferner die Messung der Expression einer oder mehrerer der folgenden microRNAs in einer biologischen Probe umfasst: hsa-let-7b-3p (MIMAT0004482), hsa-let-7f-1-3p (MIMAT0004486), hsa-let-7f-5p (MIMAT0000067), hsa-miR-100-5p (MIMAT0000098), hsa-miR-11401 (MIMAT0044658), hsa-miR-1246 (MIMAT0005898), hsa-miR-125b-2-3p (MIMAT0004603), hsa-miR-127-5p (MIMAT0004604), hsa-miR-144-5p (MIMAT0004600), hsa-miR-145-5p (MIMAT0000437), hsa-miR-1486-5p (MIMAT0004699), hsa-miR-152-3p (MIMAT0000438), hsa-miR-181a-5p (MIMAT0000256), hsa-miR-1976 (MIMAT0009451), hsa-miR-200a-3p (MIMAT0000682), hsa-miR-205-5p (MIMAT0000266), hsa-miR-224-5p (MIMAT0000281), hsa-miR-28-3p (MIMAT0004502), hsa-miR-323b-3p (MIMAT0015050), hsa-miR-326 (MIMAT0000756), hsa-miR-335-3p (MIMAT0004703), hsa-miR-335-5p (MIMAT0000765), hsa-miR-339-5p (MIMAT0000764), hsa-miR-363-3p (MIMAT0000707), hsa-miR-370-3p (MIMAT0000722), hsa-miR-374b-3p (MIMAT0004956), hsa-miR-376c-3p (MIMAT0000720), hsa-miR-423-5p (MIMAT0004748), hsa-miR-431-5p (MIMAT0001625), hsa-miR-4745-5p (MIMAT0019878), hsa-miR-4775 (MIMAT0019931), hsa-miR-486-5p (MIMAT0002177), hsa-miR-5009-5p (MIMAT0021041), hsa-miR-511-5p (MIMAT0002808), hsa-miR-532-5p (MIMAT0002888), hsa-miR-5683 (MIMAT0022472), hsa-miR-577 (MIMAT0003242), hsa-miR-584-5p (MIMAT0003249), hsa-miR-628-3p (MIMAT0003297), hsa-miR-6787-3p (MIMAT0027475), hsa-miR-6843-3p (MIMAT0027588), hsa-miR-6894-5p (MIMAT0027688), hsa-miR-766-3p (MIMAT0003888), hsa-miR-7976 (MIMAT0031179), hsa-miR-874-3p (MIMAT0004911), hsa-miR92b-3p (MIMAT0003218), wobei die folgenden microRNAs bei Zöliakie im Vergleich zu ihrer Expression in gesunden Kontrollen überexprimiert sind:
hsa-let-7b-3p (MIMAT0004482), hsa-let-7f-1-3p (MIMAT0004486), hsa-miR-100-5p (MIMAT0000098), hsa-miR-1246 (MIMAT0005898), hsa-miR-125b-2-3p (MIMAT0004603), hsa-miR-181a-5p (MIMAT0000256), hsa-miR-200a-3p (MIMAT0000682), hsa-miR-205-5p (MIMAT0000266), hsa-miR-224-5p (MIMAT0000281), hsa-miR-363-3p (MIMAT0000707), hsa-miR-374b-3p (MIMAT0004956), hsa-miR-423-5p (MIMAT0004748), hsa-miR-4745-5p (MIMAT0019878), hsa-miR-486-5p (MIMAT0002177), hsa-miR-5009-5p (MIMAT0021041), hsa-miR-511-5p (MIMAT0002808), hsa-miR-532-5p (MIMAT0002888), hsa-miR-5683 (MIMAT0022472), hsa-miR-577 (MIMAT0003242), hsa-miR-628-3p (MIMAT0003297), hsa-miR-6787-3p (MIMAT0027475), hsa-miR-6894-5p (MIMAT0027688), hsa-miR-7976 (MIMAT0031179), hsa-miR-874-3p (MIMAT0004911), hsa-miR-92b-3p (MIMAT0003218);
wohingegen die folgenden microRNAs bei Zöliakie im Vergleich zu ihrer Expression in gesunden Kontrollen weniger stark exprimiert werden: hsa-let-7f-5p (MIMAT0000067), hsa-miR-11401 (MIMAT0044658), hsa-miR-127-5p (MIMAT0004604), hsa-miR-144-5p (MIMAT0004600), hsa-miR-145-5p (MIMAT0000437), hsa-miR-148b-5p (MIMAT0004699), hsa-miR-152-3p (MIMAT0000438), hsa-miR-1976 (MIMAT0009451), hsa-miR-28-3p (MIMAT0004502), hsa-miR-323b-3p (MIMAT0015050), hsa-miR-326 (MIMAT0000756), hsa-miR-335-3p (MIMAT0004703), hsa-miR-335-5p (MIMAT0000765), hsa-miR-339-5p (MIMAT0000764), hsa-miR-370-3p (MIMAT0000722), hsa-miR-376c-3p (MIMAT0000720), hsa-miR-431-5p (MIMAT0001625), hsa-miR-4775 (MIMAT0019931), hsa-miR-584-5p (MIMAT0003249), hsa-miR-6843-3p (MIMAT0027588) und hsa-miR-766-3p (MIMAT0003888);
und wobei
für die *in-vitro* Überwachung der Einhaltung einer glutenfreien Diät, wobei das Verfahren ferner die Messung der Expression von mindestens einer oder
mehreren der folgenden microRNAs in einer biologischen Probe umfasst:
hsa-let-7b-5p (MIMAT0000063), hsa-miR-122-5p (MIMAT0000421), hsa-miR-125a-3p (MIMAT0004602), hsa-miR-125b-2-3p (MIMAT0004603), hsa-miR-134-5p (MIMAT0000447), hsa-miR-139-3p (MIMAT0004552), hsa-miR-140-5p (MIMAT0000431), hsa-miR-1468-5p (MIMAT0006789), hsa-miR-148a-5p (MIMAT0004549), hsa-miR-150-3p (MIMAT0004610), hsa-miR-151a-5p (MIMAT0004697), hsa-miR-185-5p (MIMAT0000455), hsa-miR-190a-5p (MIMAT0000458), hsa-miR-190b-5p (MIMAT0004929), hsa-miR-1976 (MIMAT0009451), hsa-miR-199a-5p (MIMAT0000231), hsa-miR-200a-3p (MIMAT0000682), hsa-miR-20a-5p (MIMAT0000075), hsa-miR-224-5p (MIMAT0000281), hsa-miR-301a-3p (MIMAT0000688), hsa-miR-30a-5p (MIMAT0000087), hsa-miR-30d-3p (MIMAT0004551), hsa-miR-3124-5p (MIMAT0014986), hsa-miR-3138 (MIMAT0015006), hsa-miR-326 (MIMAT0000756), hsa-miR-337-5p (MIMAT0004695), hsa-miR-339-5p (MIMAT0000764), hsa-miR-342-5p (MIMAT0004694), hsa-miR-362-5p (MIMAT0000705), hsa-miR-363-3p (MIMAT0000707), hsa-miR-3661 (MIMAT0018082), hsa-miR-370-3p (MIMAT0000722), hsa-miR-377-5p (MIMAT0004689), hsa-miR-423-3p (MIMAT0001340), hsa-miR-423-5p (MIMAT0004748), hsa-miR-432-5p (MIMAT0002814), hsa-miR-4745-5p (MIMAT0019878), hsa-miR-486-5p (MIMAT0002177), hsa-miR-487b-3p (MIMAT0003180), hsa-miR-5010-5p (MIMAT0021043), hsa-miR-539-3p (MIMAT0022705), hsa-miR-542-3p (MIMAT0003389), hsa-miR-5683 (MIMAT0022472), hsa-miR-6735-5p (MIMAT0027371), hsa-miR-7705 (MIMAT0030020), hsa-miR-873-5p (MIMAT0004953), hsa-miR-887-5p (MIMAT0026720);
wobei die folgenden microRNAs bei Personen mit Zöliakie, die keine glutenfreie Diät einhalten, im Vergleich zu ihrer Expression bei Personen mit Zöliakie, die die glutenfreie Diät einhalten, überexprimiert sind:
hsa-let-7b-5p (MIMAT0000063), hsa-miR-122-5p (MIMAT0000421), hsa-miR-125b-2-3p (MIMAT0004603), hsa-miR-1468-5p (MIMAT0006789), hsa-miR-150-3p (MIMAT0004610), hsa-miR-200a-3p (MIMAT0000682), hsa-miR-224-5p (MIMAT0000281), hsa-miR-30a-5p (MIMAT0000087), hsa-miR-3138 (MIMAT0015006), hsa-miR-342-5p (MIMAT0004694), hsa-miR-363-3p (MIMAT0000707), hsa-miR-3661 (MIMAT0018082), hsa-miR-423-5p (MIMAT0004748), hsa-miR-4745-5p (MIMAT0019878), hsa-miR-486-5p (MIMAT0002177), hsa-miR-5010-5p (MIMAT0021043), hsa-miR-5683 (MIMAT0022472), hsa-miR-6735-5p (MIMAT0027371);
während die folgenden microRNAs bei Probanden mit Zöliakie, die sich nicht glutenfrei ernähren, weniger stark exprimiert werden. mit Zöliakie, die sich nicht glutenfrei ernähren, im Vergleich zu ihrer Expression bei Personen mit Zöliakie, die sich glutenfrei ernähren: hsa-miR-125a-3p (MIMAT0004602), hsa-miR-134-5p (MIMAT0000447), hsa-miR-139-3p (MIMAT0004552), hsa-miR-140-5p (MIMAT0000431), hsa-miR-148a-5p (MIMAT0004549), hsa-miR-151a-5p (MIMAT0004697), hsa-miR-185-5p (MIMAT0000455), hsa-miR-190a-5p (MIMAT0000458), hsa-miR-190b-5p (MIMAT0004929), hsa-miR-1976 (MIMAT0009451), hsa-miR-199a-5p (MIMAT0000231), hsa-miR-20a-5p (MIMAT0000075), hsa-miR-301a-3p (MIMAT0000688), hsa-miR-30d-3p (MIMAT0004551), hsa-miR-3124-5p (MIMAT0014986), hsa-miR-326 (MIMAT0000756), hsa-miR-337-5p (MIMAT0004695), hsa-miR-339-5p (MIMAT0000764), hsa-miR-362-5p (MIMAT0000705), hsa-miR-370-3p (MIMAT0000722), hsa-miR-377-5p (MIMAT0004689), hsa-miR-423-3p (MIMAT0001340), hsa-miR-432-5p (MIMAT0002814), hsa-miR-487b-3p (MIMAT0003180), hsa-miR-539-3p (MIMAT0022705), hsa-miR-542-3p (MIMAT0003389), hsa-miR-7705 (MIMAT0030020), hsa-miR-873-5p (MIMAT0004953) und hsa-miR-887-5p (MIMAT0026720).

14. Verfahren nach Anspruch 13, wobei für die In-vitro-Diagnose der Zöliakie die eine oder mehreren microRNAs ausgewählt werden aus:
hsa-let-7b-3p (MIMAT0004482), hsa-let-7f-5p (MIMAT0000067), hsa-miR-200a-3p (MIMAT0000682), hsa-miR-28-3p (MIMAT0004502), hsa-miR-339-5p (MIMAT0000764), hsa-miR-4775 (MIMAT0019931), hsa-miR-577 (MIMAT0003242), hsa-miR-766-3p (MIMAT0003888), hsa-miR-874-3p (MIMAT0004911);
alternativ die eine oder mehrere microRNAs ausgewählt sind aus:
hsa-miR-100-5p (MIMAT0000098), hsa-miR-125b-2-3p (MIMAT0004603), hsa-miR-323b-3p (MIMAT0015050), hsa-miR-335-5p (MIMAT0000765), hsa-miR-370-3p (MIMAT0000722), hsa-miR-423-5p (MIMAT0004748), hsa-miR-92b-3p (MIMAT0003218);
und wobei
für die In-vitro-Überwachung der Einhaltung einer glutenfreien Diät, wobei die eine oder mehrere microRNAs ausgewählt sind aus:
hsa-miR-150-3p (MIMAT0004610), hsa-miR-190a-5p (MIMAT0000458), hsa-miR-200a-3p (MIMAT0000682), hsa-miR-30a-3p (MIMAT0000088), hsa-miR-337-5p (MIMAT0004695), hsa-miR-377-5p (MIMAT0004689), hsa-miR-6735-5p (MIMAT0027371) und hsa-miR-887-5p (MIMAT0026720);
alternativ die eine oder mehrere microRNAs ausgewählt sind aus:
hsa-miR-125b-2-3p (MIMAT0004603), hsa-miR-134-5p (MIMAT0000447), hsa-miR-185-5p (MIMAT0000455), hsa-miR-3661 (MIMAT0018082), hsa-miR-423-5p (MIMAT0004748).

## Revendications

1. Méthode de diagnostic *in vitro* de la maladie cœliaque et/ou de contrôle in vitro de l'adhésion à un régime sans gluten, dans laquelle
ladite méthode consiste à mesurer l'expression, dans un échantillon biologique, des deux microRNA hsa-miR-192-5p (MIMAT0000222) et hsa-miR-215-5p (MIMAT0000272), dans lequel lesdits microRNA sont surexprimés dans la maladie cœliaque par rapport à leur expression chez les témoins sains ou sont surexprimés chez les sujets atteints de la maladie cœliaque qui ne suivent pas un régime sans gluten par rapport à leur expression chez les sujets atteints de la maladie cœliaque qui suivent le régime sans gluten, dans lequel l'échantillon biologique est un échantillon biologique liquide constitué de sérum ou de plasma.

2. Méthode selon la revendication 1, dans laquelle l'expression desdits microRNA est mesurée au moyen d'une technique de séquençage telle que, par exemple, le séquençage de l'RNA de nouvelle génération, ou au moyen d'une technique de PCR en temps réel.

3. Méthode selon l'une des revendications 1-2, comprenant en outre la mesure de l'expression de hsa-miR-125b-5p (MIMAT0000423) par PCR en temps réel, dans laquelle hsa-miR-125b-5p est surexprimée dans la maladie cœliaque par rapport à son expression chez les témoins sains ou est surexprimée chez les sujets atteints de la maladie cœliaque qui ne suivent pas un régime sans gluten par rapport à son expression chez les sujets atteints de la maladie cœliaque qui suivent le régime sans gluten.

4. Méthode selon la revendication 3, dans laquelle ladite méthode comprend en outre la mesure de l'expression d'un ou de plusieurs des microRNA suivants par PCR en temps réel: hsa-miR-150-5p (MIMAT0000451), hsa-miR-142-5p (MIMAT0000433), hsa-miR-223-3p (MIMAT0000280) et/ou hsa-miR-338-3p (MIMAT0000763), dans laquelle
hsa-miR-150-5p est surexprimée dans la maladie cœliaque par rapport à son expression chez les témoins sains et est surexprimée chez les sujets atteints de la maladie cœliaque qui ne suivent pas un régime sans gluten par rapport à son expression chez les sujets atteints de la maladie cœliaque qui suivent un régime sans gluten; considérant ce qui suit
hsa-miR-142-5p, hsa-miR-223-3p et hsa-miR-338-3p sont moins exprimés chez les sujets atteints de la maladie cœliaque qui ne suivent pas un régime sans gluten que chez les sujets atteints de la maladie cœliaque qui suivent un régime sans gluten.

5. Méthode selon la revendication 4, dans laquelle ladite méthode comprend la mesure de l'expression de tous les microRNA suivants par PCR en temps réel: hsa-miR-192-5p, hsa-miR-215-5p, hsa-miR-125b-5p, hsa-miR-150-5p, hsa-miR-142-5p, hsa-miR-223-3p et hsa-miR-338-3p pour le suivi *in vitro* de l'adhésion à un régime sans gluten.

6. Méthode selon la revendication 3, dans laquelle ladite méthode comprend en outre la mesure de l'expression d'un ou de plusieurs des microRNA suivants par PCR en temps réel: hsa-miR-99a-5p (MIMAT0000097), hsa-miR-194-5p (MIMAT0000460), hsa-miR-142-3p (MIMAT0000434), hsa-miR-885-5p (MIMAT0004947) et/ou hsa-miR-885-5p (MIMAT0004947), dans laquelle
hsa-miR-99a-5p, hsa-miR-194-5p et hsa-miR-885-5p sont surexprimés dans la maladie cœliaque par rapport à leur expression chez les témoins sains et sont surexprimés chez les sujets atteints de la maladie cœliaque qui ne suivent pas un régime sans gluten par rapport à leur expression chez les sujets atteints de la maladie cœliaque qui suivent un régime sans gluten, alors que
hsa-miR-142-3p est moins exprimé dans la maladie cœliaque que chez les témoins sains.

7. Méthode selon la revendication 4, dans laquelle ladite méthode comprend la mesure de l'expression de tous les microRNA suivants par PCR en temps réel: hsa-miR-192-5p, hsa-miR-215-5p, hsa-miR-125b-5p, hsa-miR-99a-5p, hsa-miR-194-5p, hsa-miR-885-5p et hsa-miR-142-3p.

8. Méthode selon l'une quelconque des revendications 1-2, qui comprend en outre la mesure de l'expression d'un ou de plusieurs des microRNA suivants au moyen d'une technique de séquençage telle que, par exemple, le séquençage de l'RNA de nouvelle génération: hsa-miR-141-3p (MIMAT0000432), hsa-miR-24-2-5p (MIMAT0004497), hsa-miR-30a-3p (MIMAT0000088) et/ou hsa-miR-375-3p (MIMAT0000728), dans laquelle
hsa-miR-141-3p, hsa-miR-30a-3p et hsa-miR-375-3p sont surexprimés dans la maladie cœliaque par rapport à leur expression chez les témoins sains et sont surexprimés chez les sujets atteints de la maladie cœliaque qui ne suivent pas un régime sans gluten par rapport à leur expression chez les sujets atteints de la maladie cœliaque qui suivent un régime sans gluten, tandis que hsa-miR-24-2-5p est moins exprimé dans la maladie cœliaque que chez les témoins sains et moins exprimé chez les sujets atteints de la maladie cœliaque qui ne suivent pas un régime sans gluten que chez les sujets atteints de la maladie cœliaque qui suivent un régime sans gluten.

9. Méthode selon la revendication 8, dans laquelle ladite méthode comprend la mesure de l'expression de tous les microRNA suivants au moyen d'une technique de séquençage:
hsa-miR-192-5p, hsa-miR-215-5p, hsa-miR-141-3p, hsa-miR-30a-3p, hsa-miR-375-3p et hsa-miR-24-2-5p.

10. Méthode selon l'une des revendications 8-9, ladite méthode comprenant en outre la mesure de l'expression de hsa-let-7c-5p (MIMAT0000064), dans laquelle ledit microRNA est surexprimé dans la maladie cœliaque par rapport à son expression chez les témoins sains.

11. Méthode selon l'une quelconque des revendications 8-9, ladite méthode comprenant en outre la mesure de l'expression d'un ou de plusieurs des microRNA suivants au moyen d'une technique de séquençage telle que, par exemple, le séquençage de l'RNA de nouvelle génération: hsa-miR-328-3p (MIMAT0000752), hsa-miR-6847-5p (MIMAT0027594) et/ou hsa-miR-424-3p (MIMAT0004749), dans laquelle
hsa-miR-6847-5p est surexprimé chez les sujets atteints de la maladie cœliaque qui ne suivent pas un régime sans gluten, par rapport à son expression chez les sujets atteints de la maladie cœliaque qui suivent un régime sans gluten;
tandis que hsa-miR-328-3p et hsa-miR-424-3p sont moins exprimés chez les sujets atteints de la maladie cœliaque qui ne suivent pas un régime sans gluten que chez les sujets atteints de la maladie cœliaque qui suivent un régime sans gluten.

12. Méthode selon la revendication 11, dans laquelle ladite méthode comprend la mesure de l'expression de tous les microRNA suivants au moyen d'une technique de séquençage:
hsa-miR-192-5p, hsa-miR-215-5p, hsa-miR-141-3p, hsa-miR-30a-3p, hsa-miR-375-3p, hsa-miR-24-2-5p, hsa-miR-6847-5p, hsa-miR-328-3p et hsa-miR-424-3p.

13. Méthode selon l'une quelconque des revendications précédentes, dans laquelle, pour le diagnostic *in vitro* de la maladie cœliaque, ladite méthode comprend en outre la mesure de l'expression, dans un échantillon biologique, d'un ou de plusieurs des microRNA suivants: hsa-let-7b-3p (MIMAT0004482), hsa-let-71-1-3p (MIMAT0004486), hsa-let-7f-5p (MIMAT0000067), hsamiR-100-5p (MIMAT0000098), hsa-miR-11401 (MIMAT0044658), hsa-miR-1246 (MIMAT0005898), hsa-miR-125b-2-3p (MIMAT0004603), hsa-miR-127-5p (MIMAT0004604), hsa-miR-144-5p (MIMAT0004600), hsa-miR-145-5p (MIMAT0000437), hsa-miR-1486-5p (MIMAT0004699), hsa-miR-152-3p (MIMAT0000438), hsa-miR-181a-5p (MIMAT0000256), hsa-miR-1976 (MIMAT0009451), hsa-miR-200a-3p (MIMAT0000682), hsa-miR-205-5p (MIMAT0000266), hsa-miR-224-5p (MIMAT0000281), hsa-miR-28-3p (MIMAT0004502), hsa-miR-323b-3p (MIMAT0015050), hsa-miR-326 (MIMATO000756), hsa-miR-335-3p (MIMAT0004703), hsa-miR-335-5p (MIMAT0000765), hsa-miR-339-5p (MIMAT0000764), hsa-miR-363-3p (MIMAT0000707), hsa-miR-370-3p (MIMAT0000722), hsa-miR-374b-3p (MIMAT0004956), hsa-miR-376c-3p (MIMAT0000720), hsa-miR-423-5p (MIMAT0004748), hsa-miR-431-5p (MIMAT0001625), hsa-miR-4745-5p (MIMAT0019878), hsa-miR-4775 (MIMAT0019931), hsa-miR-486-5p (MIMAT0002177), hsa-miR-5009-5p (MIMAT0021041), hsa-miR-511-5p (MIMAT0002808), hsa-miR-532-5p (MIMAT0002888), hsa-miR-5683 (MIMAT0022472), hsa-miR-577 (MIMAT0003242), hsa-miR-584-5p (MIMAT0003249), hsa-miR-628-3p (MIMAT0003297), hsa-miR-6787-3p (MIMAT0027475), hsa-miR-6843-3p (MIMAT0027588), hsa-miR-6894-5p (MIMAT0027688), hsa-miR-766-3p (MIMAT0003888), hsa-miR-7976 (MIMAT0031179), hsa-miR-874-3p (MIMAT0004911), hsa-miR92b-3p (MIMAT0003218),
dans lequel les microRNA suivants sont surexprimés dans la maladie cœliaque par rapport à leur expression chez les témoins sains:
hsa-let-7b-3p (MIMAT0004482), hsa-let-7f-1-3p (MIMAT0004486), hsa-miR-100-5p (MIMAT0000098), hsa-miR-1246 (MIMAT0005898), hsa-miR-125b-2-3p (MIMAT0004603), hsa-miR-181a-5p (MIMAT0000256), hsa-miR-200a-3p (MIMAT0000682), hsa-miR-205-5p (MIMAT0000266), hsa-miR-224-5p (MIMAT0000281), hsa-miR-363-3p (MIMAT0000707), hsa-miR-374b-3p (MIMAT0004956), hsa-miR-423-5p (MIMAT0004748), hsa-miR-4745-5p (MIMAT0019878), hsa-miR-486-5p (MIMAT0002177), hsa-miR-5009-5p (MIMAT0021041), hsa-miR-511-5p (MIMAT0002808), hsa-miR-532-5p (MIMAT0002888), hsa-miR-5683 (MIMAT0022472), hsa-miR-577 (MIMAT0003242), hsa-miR-628-3p (MIMAT0003297), hsa-miR-6787-3p (MIMAT0027475), hsa-miR-6894-5p (MIMAT0027688), hsa-miR-7976 (MIMAT0031179), hsa-miR-874-3p (MIMAT0004911), hsa-miR-92b-3p (MIMAT0003218) ;
tandis que les microRNA suivants sont moins exprimés dans la maladie cœliaque que chez les témoins sains:
hsa-let-7f-5p (MIMAT0000067), hsa-miR-11401 (MIMAT0044658), hsa-miR-127-5p (MIMAT0004604), hsa-miR-144-5p (MIMAT0004600), hsa-miR-145-5p (MIMAT0000437), hsa-miR-148b-5p (MIMAT0004699), hsa-miR-152-3p (MIMAT0000438), hsa-miR-1976 (MIMAT0009451), hsa-miR-28-3p (MIMAT0004502), hsa-miR-323b-3p (MIMAT0015050), hsa-miR-326 (MIMAT0000756), hsa-miR-335-3p (MIMAT0004703), hsa-miR-335-5p (MIMAT0000765), hsa-miR-339-5p (MIMAT0000764), hsa-miR-370-3p (MIMAT0000722), hsa-miR-376c-3p (MIMAT0000720), hsa-miR-431-5p (MIMAT0001625), hsa-miR-4775 (MIMAT0019931), hsa-miR-584-5p (MIMAT0003249), hsa-miR-6843-3p (MIMAT0027588) et hsa-miR-766-3p (MIMAT0003888);
et dans lequel
pour le contrôle in vitro de l'adhésion à un régime sans gluten, ladite méthode comprend en outre la mesure de l'expression, dans un échantillon biologique, d'au moins un ou plusieurs des microRNA suivants:
hsa-let-7b-5p (MIMAT0000063), hsa-miR-122-5p (MIMAT0000421), hsa-miR-125a-3p (MIMAT0004602), hsa-miR-125b-2-3p (MIMAT0004603), hsa-miR-134-5p (MIMAT0000447), hsa-miR-139-3p (MIMAT0004552), hsa-miR-140-5p (MIMAT0000431), hsa-miR-1468-5p (MIMAT0006789), hsa-miR-148a-5p (MIMAT0004549), hsa-miR-150-3p (MIMAT0004610), hsa-miR-151a-5p (MIMAT0004697), hsa-miR-185-5p (MIMAT0000455), hsa-miR-190a-5p (MIMAT0000458), hsa-miR-190b-5p (MIMAT0004929), hsa-miR-1976 (MIMAT0009451), hsa-miR-199a-5p (MIMAT0000231), hsa-miR-200a-3p (MIMAT0000682), hsa-miR-20a-5p (MIMAT0000075), hsa-miR-224-5p (MIMAT0000281), hsa-miR-301a-3p (MIMAT0000688), hsa-miR-30a-5p (MIMAT0000087), hsa-miR-30d-3p (MIMAT0004551), hsa-miR-3124-5p (MIMAT0014986), hsa-miR-3138 (MIMAT0015006), hsa-miR-326 (MIMAT0000756), hsa-miR-337-5p (MIMAT0004695), hsa-miR-339-5p (MIMAT0000764), hsa-miR-342-5p (MIMAT0004694), hsa-miR-362-5p (MIMAT0000705), hsa-miR-363-3p (MIMAT0000707), hsa-miR-3661 (MIMAT0018082), hsa-miR-370-3p (MIMAT0000722), hsa-miR-377-5p (MIMAT0004689), hsa-miR-423-3p (MIMATO001340), hsa-miR-423-5p (MIMAT0004748), hsa-miR-432-5p (MIMAT0002814), hsa-miR-4745-5p (MIMAT0019878), hsa-miR-486-5p (MIMAT0002177), hsa-miR-487b-3p (MIMAT0003180), hsa-miR-5010-5p (MIMAT0021043), hsa-miR-539-3p (MIMAT0022705), hsa-miR-542-3p (MIMAT0003389), hsa-miR-5683 (MIMAT0022472), hsa-miR-6735-5p (MIMAT0027371), hsa-miR-7705 (MIMAT0030020), hsa-miR-873-5p (MIMAT0004953), hsa-miR-887-5p (MIMAT0026720);
dans lequel les microRNA suivants sont surexprimés chez les sujets atteints de la maladie cœliaque qui ne suivent pas un régime sans gluten par rapport à leur expression chez les sujets atteints de la maladie cœliaque qui suivent le régime sans gluten:
hsa-let-7b-5p (MIMAT0000063), hsa-miR-122-5p (MIMAT0000421), hsa-miR-125b-2-3p (MIMAT0004603), hsa-miR-1468-5p (MIMAT0006789), hsa-miR-150-3p (MIMAT0004610), hsa-miR-200a-3p (MIMAT0000682), hsa-miR-224-5p (MIMAT0000281), hsa-miR-30a-5p (MIMAT0000087), hsa-miR-3138 (MIMAT0015006), hsa-miR-342-5p (MIMAT0004694), hsa-miR-363-3p (MIMAT0000707), hsa-miR-3661 (MIMAT0018082), hsa-miR-423-5p (MIMAT0004748), hsa-miR-4745-5p (MIMAT0019878), hsa-miR-486-5p (MIMAT0002177), hsa-miR-5010-5p (MIMAT0021043), hsa-miR-5683 (MIMAT0022472), hsa-miR-6735-5p (MIMAT0027371);
tandis que les microRNA suivants sont moins exprimés chez les sujets. Les microRNA suivants sont exprimés à la baisse chez les sujets atteints de maladie cœliaque qui ne suivent pas un régime sans gluten, par rapport à leur expression chez les sujets atteints de maladie cœliaque qui suivent un régime sans gluten:
hsa-miR-125a-3p (MIMAT0004602), hsa-miR-134-5p (MIMAT0000447), hsa-miR-139-3p (MIMAT0004552), hsa-miR-140-5p (MIMAT0000431), hsa-miR-148a-5p (MIMAT0004549), hsa-miR-151a-5p (MIMAT0004697), hsa-miR-185-5p (MIMAT0000455), hsa-miR-190a-5p (MIMAT0000458), hsa-miR-190b-5p (MIMAT0004929), hsa-miR-1976 (MIMAT0009451), hsa-miR-199a-5p (MIMAT0000231), hsa-miR-20a-5p (MIMAT0000075), hsa-miR-301a-3p (MIMAT0000688), hsa-miR-30d-3p (MIMAT0004551), hsa-miR-3124-5p (MIMAT0014986), hsa-miR-326 (MIMAT0000756), hsa-miR-337-5p (MIMAT0004695), hsa-miR-339-5p (MIMAT0000764), hsa-miR-362-5p (MIMAT0000705), hsa-miR-370-3p (MIMAT0000722), hsa-miR-377-5p (MIMAT0004689), hsa-miR-423-3p (MIMAT0001340), hsa-miR-432-5p (MIMAT0002814), hsa-miR-487b-3p (MIMAT0003180), hsa-miR-539-3p (MIMAT0022705), hsa-miR-542-3p (MIMAT0003389), hsa-miR-7705 (MIMAT0030020), hsa-miR-873-5p (MIMAT0004953) et hsa-miR-887-5p (MIMAT0026720).

14. Méthode selon la revendication 13, dans laquelle, pour le diagnostic in vitro de la maladie cœliaque, ledit ou lesdits microRNA sont choisis parmi:
hsa-let-7b-3p (MIMAT0004482), hsa-let-7f-5p (MIMAT0000067), hsa-miR-200a-3p (MIMAT0000682), hsa-miR-28-3p (MIMAT0004502), hsa-miR-339-5p (MIMAT0000764), hsa-miR-4775 (MIMAT0019931), hsa-miR-577 (MIMAT0003242), hsa-miR-766-3p (MIMAT0003888), hsa-miR-874-3p (MIMAT0004911) ;
alternativement, ledit ou lesdits microRNA sont choisis parmi:
hsa-miR-100-5p (MIMAT0000098), hsa-miR-125b-2-3p (MIMAT0004603), hsa-miR-323b-3p (MIMAT0015050), hsa-miR-335-5p (MIMAT0000765), hsa-miR-370-3p (MIMAT0000722), hsa-miR-423-5p (MIMAT0004748), hsa-miR-92b-3p (MIMAT0003218) ;
et dans lequel
pour la surveillance in vitro de l'adhésion à un régime sans gluten, ledit ou lesdits microRNA sont choisis parmi:
hsa-miR-150-3p (MIMAT0004610), hsa-miR-190a-5p (MIMAT0000458), hsa-miR-200a-3p (MIMAT0000682), hsa-miR-30a-3p (MIMAT0000088), hsa-miR-337-5p (MIMATO004695), hsa-miR-377-5p (MIMAT0004689), hsa-miR-6735-5p (MIMAT0027371) et hsa-miR-887-5p (MIMAT0026720) ;
alternativement, ledit ou lesdits microRNA sont choisis parmi:
hsa-miR-125b-2-3p (MIMAT0004603), hsa-miR-134-5p (MIMAT0000447), hsa-miR-185-5p (MIMAT0000455), hsa-miR-3661 (MIMAT0018082), hsa-miR-423-5p (MIMAT0004748) .
